# EUROPEAN PATENT APPLICATION

(11) **EP 4 455 290 A1**
(43) Date of publication of application: **30.10.2024**
(21) Application number: 22916101.3
(22) Date of filing: 27.12.2022
(51) Int. Cl.: C12N 15/62, A01K 67/027, C12N 5/0735, C12N 5/074, C12N 5/0775, C12N 5/0789, C12N 5/0797, C12N 5/10, C12N 7/01, C12N 15/12, C12N 15/13, C12N 15/63

(54) **FUSION PROTEIN OF ANTI-TRANSFERRIN RECEPTOR ANTIBODY AND BIOACTIVE PROTEIN FOR SAFE GENE THERAPY**

(30) Priority: 28.12.2021 JP 2021214539
(71) Applicant: JCR Pharmaceuticals Co., Ltd., Ashiya-shi Hyogo 659-0021 (JP)
(72) Inventor: KINOSHITA, Masafumi, Kobe-shi, Hyogo 651-2241 (JP); TAKAGI, Haruna, Kobe-shi, Hyogo 651-2241 (JP); IIZUKA, Shunsuke, Kobe-shi, Hyogo 651-2241 (JP); OHTSUKI, Kota, Kobe-shi, Hyogo 651-2241 (JP); SONODA, Hiroyuki, Kobe-shi, Hyogo 651-2241 (JP)
(74) Representative: Boult Wade Tennant LLP
(86) International application number: PCT/JP2022/048203
(87) International publication number: WO 2023/127879

(57) **Abstract**

An object of the present invention is to provide a nucleic acid molecule or a viral virion containing the nucleic acid molecule, the nucleic acid molecule having integrated therein a gene encoding a fusion protein of an anti-transferrin receptor antibody and a physiologically active protein, the fusion protein having an adjusted binding activity to TfR, and being capable of reducing a side reaction such as an anemia symptom caused by binding of the anti-TfR antibody to the transferrin receptor, for example, when the gene encoding the fusion protein of the anti-TfR antibody and the physiologically active protein is used in gene therapy.

## Description

### Technical Field

The present invention relates to a gene encoding an anti-transferrin receptor antibody, and more particularly to a gene encoding an anti-transferrin receptor antibody, which, for example, in an AAV vector system, is used at the time of preparation of a vector having integrated therein a nucleic acid molecule encoding a fusion protein of an anti-transferrin receptor antibody and a physiologically active protein.

### Background Art

Many recombinant proteins used as medicaments are administered to patients by parenteral means such as subcutaneous injection, intramuscular injection, and intravenous injection. If a patient suffers from a chronic disease, administration of a medicament needs to be repeated over a long period of time. Thus, a heavy burden is imposed on the patient when the administration is performed by the parenteral means.

Some recombinant proteins are fusion proteins of an antibody and a physiologically active protein. Examples of such fusion proteins include fusion proteins of an antibody and a lysosomal enzyme (Patent Documents 1 to 5 and Non-Patent Document 1).

A lysosomal disease is a genetic disease caused by reduction or absence of activity of a lysosomal enzyme due to an abnormality in gene encoding the lysosomal enzyme which has to be present in the lysosome. To patients with a lysosomal disease, so-called enzyme replacement therapy is carried out in which a recombinant lysosomal enzyme is administered by intravenous injection so as to replace the reduced or absent lysosomal enzyme. Patients with a lysosomal disease, which is a genetic disease, need to receive this enzyme replacement therapy throughout their life.

Some lysosomal diseases cause brain disorders. In order to treat the brain disorders, it is necessary to supplement a lysosomal enzyme in the brain, but the lysosomal enzyme administered by intravenous injection hardly passes through the blood-brain barrier (BBB), and thus cannot be supplemented in the brain.

A method for supplementing a lysosomal enzyme into the brain has been reported in which a lysosomal enzyme is bound to an antibody that recognizes, as an antigen, a molecule present on surfaces of vascular endothelial cells to form a fusion protein, and the fusion protein is administered by intravenous injection (Non-Patent Document 2). Such a fusion protein can bind to the surfaces of the vascular endothelial cells via the antibody portion, further pass through the BBB, and reach the brain. Thus, the lysosomal enzyme can be supplemented into the brain by such fusion proteins. Even when the lysosomal enzyme is made into such a fusion protein, patients with a lysosomal disease, which is a genetic disease, need to receive an enzyme replacement therapy with the fusion protein throughout their lifetime.

A molecule present on the surfaces of the vascular endothelial cells is a human transferrin receptor (hTfR). In recent years, Pabinafusp alfa, which is a fusion protein of the hTfR and human iduronate-2-sulfatase, a kind of lysosomal enzyme, has started to be marketed as a therapeutic agent effective for central nervous system disorders associated with Hunter syndrome. Patients have to continue to receive this Pabinafusp alfa by intravenous drip infusion once a week (Non-Patent Document 3). It has been reported that, when an anti-TfR antibody is administered to an animal, the anti-TfR antibody acts on reticulocytes, which express a large amount of TfR, thereby reducing the number of reticulocytes (Non-Patent Document 4).

### Citation List

### Patent Documents

Patent Document 1: WO2016/208695
Patent Document 2: WO 2018/124121
Patent Document 3: US 2007/0082380
Patent Document 4: US 2009/0053219
Patent Document 5: US 2011/0110935

### Non-Patent Documents

Non-Patent Document 1: Sonoda H. et al., Mol Ther. 26. 1366-74 (2018)
Non-Patent Document 2: Okuyama T. et al., Mol Ther. 26. 27. 456-64 (2019)
Non-Patent Document 3: Package Insert of IZCARGO (trade name) for I. V. infusion 10 mg, First Edition, created in March 2021
Non-Patent Document 4: Couch JA. Sci Transl Med. 5. 183ra57 (2013)

### Summary of Invention

### Technical Problem

An object of the present invention is to provide a nucleic acid molecule having integrated therein a gene encoding a fusion protein of an anti-transferrin receptor antibody (anti-TfR antibody) and a physiologically active protein, the fusion protein having a certain defined binding activity to a transferrin receptor (TfR), and being capable of reducing a side reaction such as an anemia symptom caused by binding of the anti-TfR antibody to the TfR, for example, when the gene encoding the fusion protein of the anti-TfR antibody and the physiologically active protein is used in gene therapy.

### Solution to Problem

In the research aimed at the above object, the present inventors have found, as a result of intensive studies, that a highly safe viral virion that can be used for gene therapy can be prepared by using a nucleic acid molecule containing a gene encoding a fusion protein of an anti-TfR antibody and a physiologically active protein, the fusion protein having a certain level of binding activity (affinity) to TfR, and have completed the present invention. That is, the present invention includes the following.
1. A nucleic acid molecule encoding a fusion protein of an anti-transferrin receptor antibody and a physiologically active protein, wherein the fusion protein has a binding activity (EC₅₀) to a transferrin receptor of from 0.5 nM to 1 µM, and wherein the nucleic acid molecule containing any nucleotide sequence selected from the group consisting of (1) to (6) below:
   (1) a nucleotide sequence containing a first inverted terminal repeat (ITR) or a functional equivalent thereof, downstream thereof a nucleotide sequence encoding the fusion protein of the anti-transferrin receptor antibody and the physiologically active protein, and further downstream thereof a nucleotide sequence containing a second inverted terminal repeat (ITR) or a functional equivalent thereof;
   (2) a nucleotide sequence containing a first inverted terminal repeat (ITR) or a functional equivalent thereof, downstream thereof a nucleotide sequence containing a gene expression regulatory site, further downstream thereof a nucleotide sequence encoding the fusion protein of the anti-transferrin receptor antibody and the physiologically active protein, and further downstream thereof a nucleotide sequence containing a second inverted terminal repeat (ITR) or a functional equivalent thereof;
   (3) a nucleotide sequence containing a first long terminal repeat (LTR) or a functional equivalent thereof, downstream thereof a nucleotide sequence encoding the fusion protein of the anti-transferrin receptor antibody and the physiologically active protein, and further downstream thereof a nucleotide sequence containing a second long terminal repeat (LTR) or a functional equivalent thereof;
   (4) a nucleotide sequence containing a first long terminal repeat (LTR) or a functional equivalent thereof, downstream thereof a nucleotide sequence containing a gene expression regulatory site, further downstream thereof a nucleotide sequence encoding the fusion protein of the anti-transferrin receptor antibody and the physiologically active protein, and further downstream thereof a nucleotide sequence containing a second long terminal repeat (LTR) or a functional equivalent thereof;
   (5) a nucleotide sequence containing a leader or a functional equivalent thereof, downstream thereof a nucleotide sequence encoding the fusion protein of the anti-transferrin receptor antibody and the physiologically active protein, and further downstream thereof a nucleotide sequence containing a trailer or a functional equivalent thereof; and
   (6) a nucleotide sequence containing a leader or a functional equivalent thereof, downstream thereof a nucleotide sequence containing a gene expression regulatory site, further downstream thereof a nucleotide sequence encoding the fusion protein of the anti-transferrin receptor antibody and the physiologically active protein, and further downstream thereof a nucleotide sequence containing a trailer or a functional equivalent thereof.
2. The nucleic acid molecule according to 1 above, wherein the nucleic acid molecule is selected from the group consisting of (1) to (4) below:
   (1) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a heavy chain (which may be a variable region thereof) of the anti-transferrin receptor antibody, and a nucleotide sequence encoding a light chain (which may be a variable region thereof) of the anti-transferrin receptor antibody;
   (2) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a heavy chain (which may be a variable region thereof) of the anti-transferrin receptor antibody, and a nucleotide sequence encoding a light chain (which may be a variable region thereof) of the anti-transferrin receptor antibody;
   (3) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a light chain (which may be a variable region thereof) of the anti-transferrin receptor antibody, and a nucleotide sequence encoding a heavy chain (which may be a variable region thereof) of the anti-transferrin receptor antibody; and
   (4) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a light chain (which may be a variable region thereof) of the anti-transferrin receptor antibody, and a nucleotide sequence encoding a heavy chain (which may be a variable region thereof) of the anti-transferrin receptor antibody.
3. The nucleic acid molecule according to 1 above, wherein the nucleic acid molecule is selected from the group consisting of (1) to (4) below:
   (1) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a heavy chain (which may be a variable region thereof) of the anti-transferrin receptor antibody via a linker, and a nucleotide sequence encoding a light chain (which may be a variable region thereof) of the anti-transferrin receptor antibody;
   (2) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a heavy chain (which may be a variable region thereof) of the anti-transferrin receptor antibody via a linker, and a nucleotide sequence encoding a light chain (which may be a variable region thereof) of the anti-transferrin receptor antibody;
   (3) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a light chain (which may be a variable region thereof) of the anti-transferrin receptor antibody via a linker, and a nucleotide sequence encoding a heavy chain (which may be a variable region thereof) of the anti-transferrin receptor antibody; and
   (4) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a light chain (which may be a variable region thereof) of the anti-transferrin receptor antibody via a linker, and a nucleotide sequence encoding a heavy chain (which may be a variable region thereof) of the anti-transferrin receptor antibody.
4. The nucleic acid molecule according to 3 above, wherein the linker is a peptide containing from 1 to 50 amino acid residues.
5. The nucleic acid molecule according to 4 above, wherein the linker is a peptide containing an amino acid sequence selected from the group consisting of one glycine, one serine, an amino acid sequence Gly-Ser, an amino acid sequence Gly-Gly-Ser, an amino acid sequence of SEQ ID NO: 1, an amino acid sequence of SEQ ID NO: 2, an amino acid sequence of SEQ ID NO: 3, and an amino acid sequence containing successively from 2 to 10 of these amino acid sequences.
6. The nucleic acid molecule according to 1 above, wherein the nucleic acid molecule is selected from the group consisting of (1) to (4) below:
   (1) a nucleic acid molecule wherein the nucleotide sequence encoding the fusion protein of the anti-transferrin receptor antibody and the physiologically active protein contains a nucleotide sequence encoding a conjugate in which a heavy chain (which may be a variable region thereof) of the anti-transferrin receptor antibody binds to a C-terminus of a light chain (which may be a variable region thereof) of the anti-transferrin receptor antibody via a second linker, and further the physiologically active protein binds to a C-terminus thereof directly or via a linker;
   (2) a nucleic acid molecule wherein the nucleotide sequence encoding the fusion protein of the anti-transferrin receptor antibody and the physiologically active protein contains a nucleotide sequence encoding a conjugate in which a light chain (which may be a variable region thereof) of the anti-transferrin receptor antibody binds to a C-terminus of a heavy chain (which may be a variable region thereof) of the anti-transferrin receptor antibody via a second linker, and further the physiologically active protein binds to a C-terminus thereof directly or via a linker;
   (3) a nucleic acid molecule wherein the nucleotide sequence encoding the fusion protein of the anti-transferrin receptor antibody and the physiologically active protein contains a nucleotide sequence encoding a conjugate in which a heavy chain (which may be a variable region thereof) of the anti-transferrin receptor antibody binds to a C-terminus of the physiologically active protein directly or via a linker, and further a light chain (which may be a variable region thereof) of the anti-transferrin receptor antibody binds to a C-terminus thereof via a second linker; or
   (4) a nucleic acid molecule wherein the nucleotide sequence encoding the fusion protein of the anti-transferrin receptor antibody and the physiologically active protein contains a nucleotide sequence encoding a conjugate in which a light chain (which may be a variable region thereof) of the anti-transferrin receptor antibody binds to a C-terminus of the physiologically active protein directly or via a linker, and further a heavy chain (which may be a variable region thereof) of the anti-transferrin receptor antibody binds to a C-terminus thereof via a second linker.
7. The nucleic acid molecule according to 6 above, wherein the linker is a peptide containing from 1 to 50 amino acid residues.
8. The nucleic acid molecule according to 7 above, wherein the linker is a peptide containing an amino acid sequence selected from the group consisting of one glycine, one serine, an amino acid sequence Gly-Ser, an amino acid sequence Gly-Gly-Ser, an amino acid sequence of SEQ ID NO: 1, an amino acid sequence of SEQ ID NO: 2, an amino acid sequence of SEQ ID NO: 3, and an amino acid sequence containing successively from 2 to 10 of these amino acid sequences.
9. The nucleic acid molecule according to 8 above, wherein the second linker contains from 8 to 50 amino acid residues.
10. The nucleic acid molecule according to 9 above, wherein the second linker is selected from the group consisting of an amino acid sequence Gly-Ser, an amino acid sequence Gly-Gly-Ser, an amino acid sequence Gly-Gly-Gly, amino acid sequences of SEQ ID NO: 1, SEQ ID NO: 2, and SEQ ID NO: 3, an amino acid sequence containing successively 3 of the amino acid sequences of SEQ ID NO: 1, and an amino acid sequence containing successively from 2 to 10 of these amino acid sequences.
11. The nucleic acid molecule according to 2 above, wherein the nucleic acid molecule is selected from the group consisting of (1) to (4) below:
   (1) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a heavy chain (which may be a variable region thereof) of the anti-transferrin receptor antibody, downstream thereof a nucleotide sequence encoding an internal ribosome binding site, and further downstream thereof a nucleotide sequence encoding a light chain (which may be a variable region thereof) of the anti-transferrin receptor antibody;
   (2) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a heavy chain (which may be a variable region thereof) of the anti-transferrin receptor antibody, downstream thereof a nucleotide sequence encoding an internal ribosome binding site, and further downstream thereof a nucleotide sequence encoding a light chain (which may be a variable region thereof) of the anti-transferrin receptor antibody;
   (3) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a light chain (which may be a variable region thereof) of the anti-transferrin receptor antibody, downstream thereof a nucleotide sequence encoding an internal ribosome binding site, and further downstream thereof a nucleotide sequence encoding a heavy chain (which may be a variable region thereof) of the anti-transferrin receptor antibody; and
   (4) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a light chain (which may be a variable region thereof) of the anti-transferrin receptor antibody, downstream thereof a nucleotide sequence encoding an internal ribosome binding site, and further downstream thereof a nucleotide sequence encoding a heavy chain (which may be a variable region thereof) of the anti-transferrin receptor antibody.
12. The nucleic acid molecule according to any one of 3 to 5 above, wherein the nucleic acid molecule is selected from the group consisting of (1) to (4) below:
   (1) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a heavy chain (which may be a variable region thereof) of the anti-transferrin receptor antibody via a linker, downstream thereof a nucleotide sequence encoding an internal ribosome binding site, and further downstream thereof a nucleotide sequence encoding a light chain (which may be a variable region thereof) of the anti-transferrin receptor antibody;
   (2) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a heavy chain (which may be a variable region thereof) of the anti-transferrin receptor antibody via a linker, downstream thereof a nucleotide sequence encoding an internal ribosome binding site, and further downstream thereof a nucleotide sequence encoding a light chain (which may be a variable region thereof) of the anti-transferrin receptor antibody;
   (3) a nucleic acid molecule wherein the nucleotide sequence encoding the fusion protein of the anti-transferrin receptor antibody and the physiologically active protein contains a nucleotide sequence encoding a conjugate in which the physiologically active protein binds to a C-terminus of a light chain (which may be a variable region thereof) of the anti-transferrin receptor antibody via a linker, downstream thereof a nucleotide sequence encoding an internal ribosome binding site, and further downstream thereof a nucleotide sequence encoding a heavy chain (which may be a variable region thereof) of the anti-transferrin receptor antibody; or
   (4) a nucleic acid molecule wherein the nucleotide sequence encoding the fusion protein of the anti-transferrin receptor antibody and the physiologically active protein contains a nucleotide sequence encoding a conjugate in which the physiologically active protein binds to an N-terminus of a light chain (which may be a variable region thereof) of the anti-transferrin receptor antibody via a linker, downstream thereof a nucleotide sequence encoding an internal ribosome binding site, and further downstream thereof a nucleotide sequence encoding a heavy chain (which may be a variable region thereof) of the anti-transferrin receptor antibody.
13. The nucleic acid molecule according to 11 or 12 above, wherein the internal ribosome binding site is derived from a 5' untranslated region of a virus or gene selected from the group consisting of virus belonging to the family *Picornaviridae,* foot-and-mouth disease virus, hepatitis A virus, hepatitis C virus, coronavirus, bovine enterovirus, Theiler's murine encephalomyelitis virus, Coxsackie type B virus, human immunoglobulin heavy chain binding protein gene, Drosophila antennapedia gene, and Drosophila ultrabithorax gene.
14. The nucleic acid molecule according to 11 or 12 above, wherein the internal ribosome binding site is derived from the 5' untranslated region of the virus belonging to the family *Picornaviridae.*
15. The nucleic acid molecule according to any one of 1 to 14 above, wherein the gene expression regulatory site is selected from the group consisting of a cytomegalovirus-derived promoter, an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, a mouse albumin promoter, a human albumin promoter, a human α-1 antitrypsin promoter, and a mouse α-fetoprotein enhancer/mouse albumin promoter.
16. The nucleic acid molecule according to any one of 1 to 15 above, wherein the anti-transferrin receptor antibody is an antigen binding fragment.
17. The nucleic acid molecule according to any one of 1 to 15 above, wherein the anti-transferrin receptor antibody is a Fab.
18. The nucleic acid molecule according to any one of 1 to 17 above, wherein the physiologically active protein is selected from the group consisting of a growth hormone, a lysosomal enzyme, a somatomedin, an insulin, a glucagon, a cytokine, a lymphokine, a blood coagulation factor, an anti-transferrin receptor antibody, a fusion protein of an anti-transferrin receptor antibody with another protein, granulocyte-macrophage colony-stimulating factor (GM-CSF), granulocyte-colony stimulating factor (G-CSF), macrophage-colony stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, nerve growth factor (NGF), ciliary neurotrophic factor (CNTF), glial-cell derived neurotrophic factor (GDNF), neurotrophin-3, neurotrophin-4/5, neurotrophin-6, neuregulin-1, activin, basic fibroblast growth factor (bFGF), fibroblast growth factor 2 (FGF2), epidermal growth factor (EGF), vascular endothelial growth factor (VEGF), interferon α, interferon β, interferon γ, interleukin-6, PD-1, a PD-1 ligand, tumor necrosis factor-α receptor (TNF-α receptor), an enzyme having beta-amyloid-degrading activity, etanercept, pegvisomant, metreleptin, abatacept, asfotase, a GLP-1 receptor agonist, and an antibody medicament.
19. The nucleic acid molecule according to any one of 1 to 17 above, wherein the physiologically active protein is selected from the group consisting of α-L-iduronidase, iduronate-2-sulfatase, acid α-glucosidase, glucocerebrosidase, β-galactosidase, a GM2 activating protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoA α-glucosaminide N-acetyltransferase, N-acetylglucosamine-6-sulfatase, acid ceramidase, amylo-1,6-glucosidase, sialidase, palmitoylprotein thioesterase-1, tripeptidylpeptidase-1, hyaluronidase-1, CLN1 and CLN2.
20. The nucleic acid molecule according to any one of 1 to 19 above, wherein the first inverted terminal repeat and the second inverted terminal repeat are derived from an adeno-associated virus, derived from an adenovirus, or are mutants thereof.
21. The nucleic acid molecule according to any one of 1 to 19 above, wherein the first inverted terminal repeat contains a nucleotide sequence represented by SEQ ID NO: 5 and the second inverted terminal repeat contains a nucleotide sequence represented by SEQ ID NO: 6.
22. The nucleic acid molecule according to any one of 1 to 19 above, wherein the first inverted terminal repeat is selected from the group consisting of (1) to (3) below:
   (1) an inverted terminal repeat containing a nucleotide sequence exhibiting 80% or more identity with the nucleotide sequence represented by SEQ ID NO: 5,
   (2) an inverted terminal repeat containing a nucleotide sequence showing 90% or more identity with the nucleotide sequence represented by SEQ ID NO: 5, and
   (3) an inverted terminal repeat containing the nucleotide sequence represented by SEQ ID NO: 5 modified by substitution, deletion, or addition of from 1 to 20 nucleotides; and
   the second inverted terminal repeat is selected from the group consisting of (4) to (6) below:
   (4) an inverted terminal repeat containing a nucleotide sequence exhibiting 80% or more identity with the nucleotide sequence represented by SEQ ID NO: 6,
   (5) an inverted terminal repeat containing a nucleotide sequence exhibiting 90% or more identity with the nucleotide sequence represented by SEQ ID NO: 6, and
   (6) an inverted terminal repeat containing the nucleotide sequence represented by SEQ ID NO: 6 modified by substitution, deletion, or addition of from 1 to 20 nucleotides.
23. The nucleic acid molecule according to any one of 1 to 19 above, wherein the functional equivalent of the first inverted terminal repeat contains a nucleotide sequence represented by SEQ ID NO: 7 and the functional equivalent of the second inverted terminal repeat contains a nucleotide sequence represented by SEQ ID NO: 8.
24. The nucleic acid molecule according to any one of 1 to 19 above, wherein the functional equivalent of the first inverted terminal repeat is selected from the group consisting of (1) to (3) below:
   (1) a functional equivalent containing a nucleotide sequence exhibiting 80% or more identity with the nucleotide sequence represented by SEQ ID NO: 7,
   (2) a functional equivalent containing a nucleotide sequence showing 90% or more identity with the nucleotide sequence represented by SEQ ID NO: 7, and
   (3) a functional equivalent containing the nucleotide sequence represented by SEQ ID NO: 7 modified by substitution, deletion, or addition of from 1 to 20 nucleotides; and
   the functional equivalent of the second inverted terminal repeat is selected from the group consisting of (4) to (6) below:
   (4) a functional equivalent containing a nucleotide sequence exhibiting 80% or more identity with the nucleotide sequence represented by SEQ ID NO: 8,
   (5) a functional equivalent containing a nucleotide sequence exhibiting 90% or more identity with the nucleotide sequence represented by SEQ ID NO: 8, and
   (6) a functional equivalent containing the nucleotide sequence represented by SEQ ID NO: 8 modified by substitution, deletion, or addition of from 1 to 20 nucleotides.
25. The nucleic acid molecule according to any one of 1 to 19 above, wherein the first long terminal repeat and the second long terminal repeat are derived from a lentivirus or a retrovirus, or are mutants thereof.
26. The nucleic acid molecule according to any one of 1 to 19 above, wherein the first long terminal repeat contains a nucleotide sequence represented by SEQ ID NO: 52 and the second long terminal repeat contains a nucleotide sequence represented by SEQ ID NO: 53.
27. The nucleic acid molecule according to any one of 1 to 19 above, wherein the first long terminal repeat is selected from the group consisting of (1) to (3) below:
   (1) a long terminal repeat containing a nucleotide sequence exhibiting 80% or more identity with the nucleotide sequence represented by SEQ ID NO: 52,
   (2) a long terminal repeat containing a nucleotide sequence showing 90% or more identity with the nucleotide sequence represented by SEQ ID NO: 52, and
   (3) a long terminal repeat containing the nucleotide sequence represented by SEQ ID NO: 52 modified by substitution, deletion, or addition of from 1 to 20 nucleotides; and
   the second long terminal repeat is selected from the group consisting of (4) to (6) below:
   (4) a long terminal repeat containing a nucleotide sequence exhibiting 80% or more identity with the nucleotide sequence represented by SEQ ID NO: 53,
   (5) a long terminal repeat containing a nucleotide sequence exhibiting 90% or more identity with the nucleotide sequence represented by SEQ ID NO: 53, and
   (6) a long terminal repeat containing the nucleotide sequence represented by SEQ ID NO: 53 modified by substitution, deletion, or addition of from 1 to 20 nucleotides.
28. The nucleic acid molecule according to any one of 1 to 19 above, wherein the leader and the trailer are derived from a Sendai virus, or are mutants thereof.
29. The nucleic acid molecule according to any one of 1 to 19 above, wherein the leader contains a nucleotide sequence represented by SEQ ID NO: 54 and the trailer contains a nucleotide sequence represented by SEQ ID NO: 55.
30. The nucleic acid molecule according to any one of 1 to 19 above, wherein the leader is selected from the group consisting of (1) to (3) below:
   (1) a leader containing a nucleotide sequence exhibiting 80% or more identity with the nucleotide sequence represented by SEQ ID NO: 54,
   (2) a leader containing a nucleotide sequence showing 90% or more identity with the nucleotide sequence represented by SEQ ID NO: 54, and
   (3) a leader containing a nucleotide sequence represented by SEQ ID NO: 54 modified by substitution, deletion, or addition of from 1 to 20 nucleotides; and
   the trailer is selected from the group consisting of (4) to (6) below
   (4) a trailer containing a nucleotide sequence exhibiting 80% or more identity with the nucleotide sequence represented by SEQ ID NO: 55,
   (5) a trailer containing a nucleotide sequence exhibiting 90% or more identity with the nucleotide sequence represented by SEQ ID NO: 55, and
   (6) a trailer containing a nucleotide sequence represented by SEQ ID NO: 55 modified by substitution, deletion, or addition of from 1 to 20 nucleotides.
31. The nucleic acid molecule according to any one of 1 to 30 above, wherein the binding activity (EC₅₀) is from 0.5 to 3 nM, from 0.8 to 3 nM, or from 1 to 3 nM.
32. The nucleic acid molecule according to any one of 1 to 30 above, wherein the binding activity (EC₅₀) is from 3 to 500 nM, from 3 to 100 nM, from 4 nM to 1 µM, from 4 to 500 nM, from 4 to 100 nM, from 5 nM to 1 µM, from 5 to 500 nM, or from 5 to 100 nM.
33. A cell, a tissue or an animal having introduced therein the nucleic acid molecule according to any one of 1 to 32 above.
34. A stem cell into which the nucleic acid molecule according to any one of 1 to 32 above is introduced.
35. The stem cell according to 34 above, wherein the stem cell is selected from the group consisting of a mesenchymal stem cell, a dental pulp-derived stem cell, a hematopoietic stem cell, an embryonic stem cell, an endothelial stem cell, a mammary stem cell, an intestinal stem cell, a hepatic stem cell, a pancreatic stem cell, a neural stem cell, and an iPS cell.
36. A plasmid containing the nucleic acid molecule according to any one of 1 to 32 above.
37. A viral virion containing the nucleic acid molecule according to any one of 1 to 32 above.
38. A pharmaceutical composition containing the viral virion according to 37 above and a carrier.
39. A viral virion containing the nucleic acid molecule according to 31 above.
40. A pharmaceutical composition containing the viral virion according to 39 above.
41. The pharmaceutical composition according to 40 above, wherein a dosage per administration is from 1.0 × 10¹⁰ to 7.5 × 10¹² vg/kg.

### Advantageous Effects of Invention

The present invention can provide a fusion protein of an anti-TfR antibody and a physiologically active protein, which may reduce the possibility to induce an anemia symptom, or a safe nucleic acid molecule containing a gene encoding such a fusion protein, which can be used for expressing the fusion protein in a cell, a tissue, or a living organism.

### Brief Description of Drawings

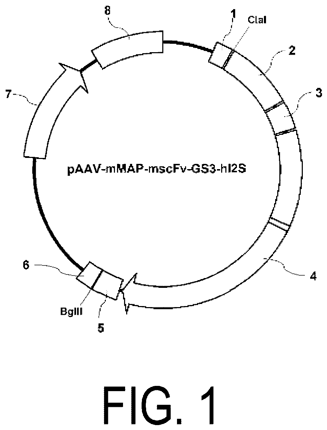

FIG. 1 is a schematic diagram illustrating a structure of a pAAV-mMAP-mscFv-GS3-hI2S vector (plasmid).

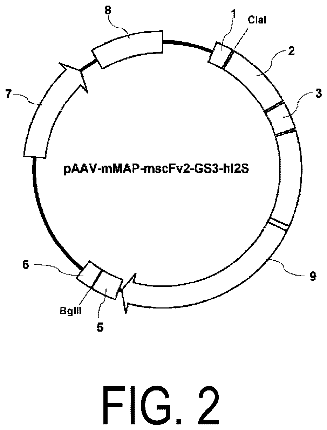

FIG. 2 is a schematic diagram illustrating a structure of a pAAV-mMAP-mscFv2-GS3-hI2S vector (plasmid).

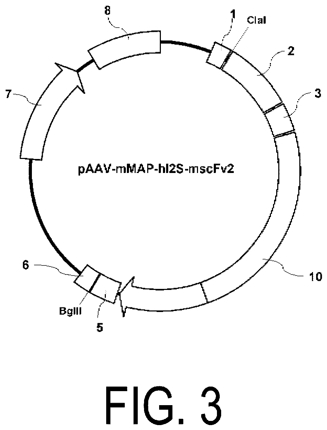

FIG. 3 is a schematic diagram illustrating a structure of a pAAV-mMAP-hI2S-mscFv2 vector (plasmid).

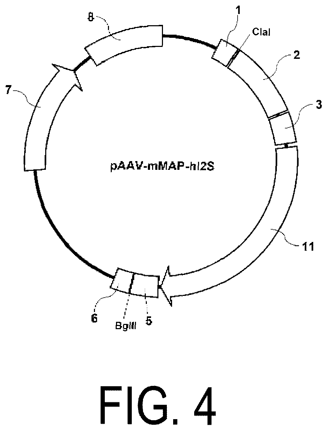

FIG. 4 is a schematic diagram illustrating a structure of a pAAV-mMAP-hI2S vector (plasmid).

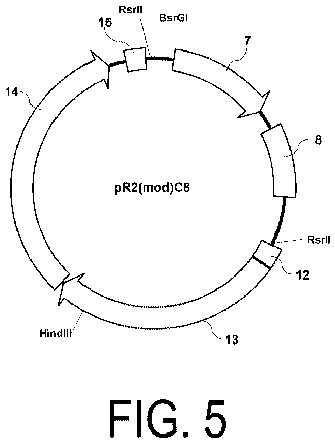

FIG. 5 is a schematic diagram illustrating a structure of a pR2 (mod) C8 vector.

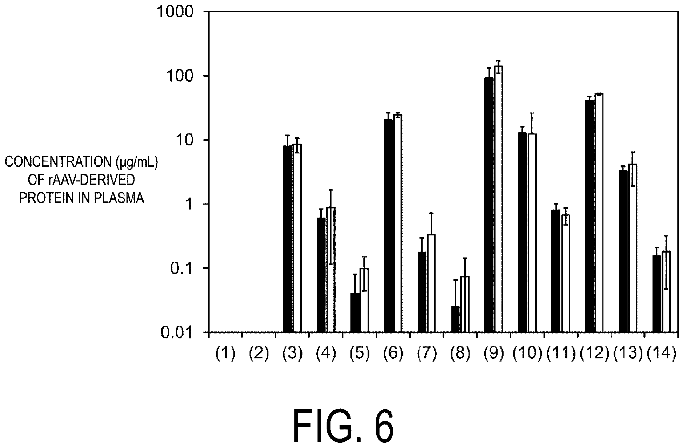

FIG. 6 is a graph illustrating results of measuring a concentration of rAAV-derived protein in plasma (Example 17). The vertical axis of a bar graph represents a logarithmic representation of the concentration (µg/mL) of rAAV-derived protein in plasma. A black bar represents a measured value of the concentration of rAAV-derived protein in plasma one week after administration of each rAAV virion, and a white bar represents a measured value of the concentration of rAAV-derived protein in plasma 6 weeks after administration of each rAAV virion. (1) indicates results of a normal control group; (2) indicates results of a pathological control group; (3) indicates results of an rAAV-mscFv-GS3-hI2S (1.0 × 10¹³ vg/kg) administration group; (4) indicates results of an rAAV-mscFv-GS3-hI2S (1.0 × 10¹² vg/kg) administration group; (5) indicates results of an rAAV-mscFv-GS3-hI2S (1.0 × 10¹¹ vg/kg) administration group; (6) indicates results of an rAAV-mscFv2-GS3-hI2S (1.0 × 10¹³ vg/kg) administration group; (7) indicates results of an rAAV-mscFv2-GS3-hI2S (1.0 × 10¹² vg/kg) administration group; (8) indicates results of an rAAV-mscFv2-GS3-hI2S (1.0 × 10¹¹ vg/kg) administration group; (9) indicates results of an rAAV-hI2S-mscFv2 (1.0 × 10¹³ vg/kg) administration group; (10) indicates results of an rAAV-hI2S-mscFv2 (1.0 × 10¹² vg/kg) administration group; (11) indicates results of an rAAV-hI2S-mscFv2 (1.0 × 10¹¹ vg/kg) administration group; (12) indicates results of an rAAV-hI2S (1.0 × 10¹³ vg/kg) administration group; (13) indicates results of an rAAV-hI2S (1.0 × 10¹² vg/kg) administration group; and (14) indicates results of an rAAV-hI2S (1.0 × 10¹¹ vg/kg) administration group. Values are mean values and error bars indicate standard deviations (n = 3). Note that, for the normal control group (1) or the pathological control group (2), measurement was not performed because of no protein expression, and a zero value is indicated for reference.

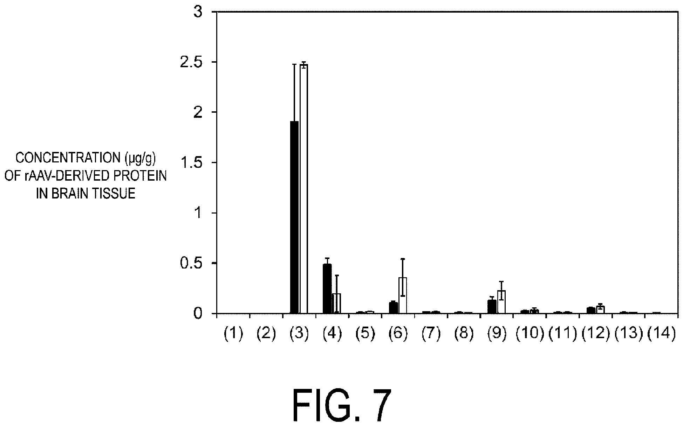

FIG. 7 is a graph illustrating results of measuring a concentration of rAAV-derived protein in the brain tissue (Example 17). The vertical axis of a bar graph represents the concentration (µg/g) of rAAV-derived protein in the brain tissue. A black bar represents a measured value of the concentration of rAAV-derived protein in the brain tissue one week after administration of each rAAV virion, and a white bar represents a measured value of the concentration of rAAV-derived protein in the brain tissue 6 weeks after administration of each rAAV virion. (1) indicates results of a normal control group; (2) indicates results of a pathological control group; (3) indicates results of an rAAV-mscFv-GS3-hI2S (1.0 × 10¹³ vg/kg) administration group; (4) indicates results of an rAAV-mscFv-GS3-hI2S (1.0 × 10¹² vg/kg) administration group; (5) indicates results of an rAAV-mscFv-GS3-hI2S (1.0 × 10¹¹ vg/kg) administration group; (6) indicates results of an rAAV-mscFv2-GS3-hI2S (1.0 × 10¹³ vg/kg) administration group; (7) indicates results of an rAAV-mscFv2-GS3-hI2S (1.0 × 10¹² vg/kg) administration group; (8) indicates results of an rAAV-mscFv2-GS3-hI2S (1.0 × 10¹¹ vg/kg) administration group; (9) indicates results of an rAAV-hI2S-mscFv2 (1.0 × 10¹³ vg/kg) administration group; (10) indicates results of an rAAV-hI2S-mscFv2 (1.0 × 10¹² vg/kg) administration group; (11) indicates results of an rAAV-hI2S-mscFv2 (1.0 × 10¹¹ vg/kg) administration group; (12) indicates results of an rAAV-hI2S (1.0 × 10¹³ vg/kg) administration group; (13) indicates results of an rAAV-hI2S (1.0 × 10¹² vg/kg) administration group; and (14) indicates results of an rAAV-hI2S (1.0 × 10¹¹ vg/kg) administration group. Values are mean values and error bars indicate standard deviations (n = 3). Note that, for the normal control group (1) or the pathological control group (2), measurement was not performed because of no protein expression, and a zero value is indicated for reference.

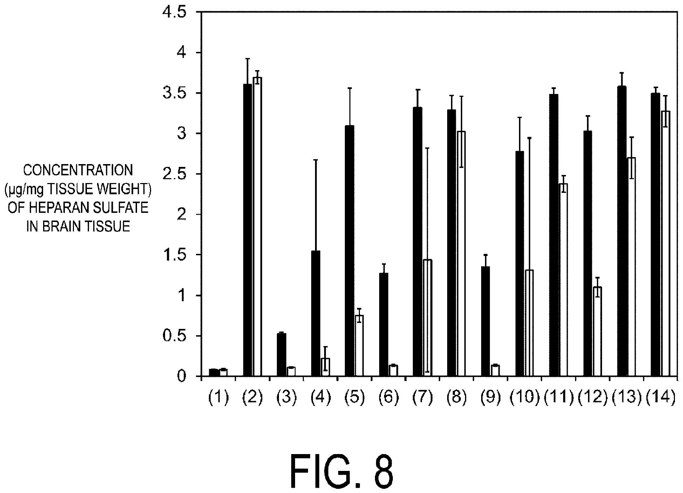

FIG. 8 is a graph illustrating results of measuring a concentration of heparan sulfate in the brain tissue (Example 18). The vertical axis of a bar graph represents the concentration (µg/mg tissue weight) of heparan sulfate in the brain tissue. A black bar represents a measured value of the concentration of heparan sulfate in the brain tissue one week after administration of each rAAV virion, and a white bar represents a measured value of the concentration of heparan sulfate in the brain tissue 6 weeks after administration of each rAAV virion. (1) indicates results of a normal control group; (2) indicates results of a pathological control group; (3) indicates results of an rAAV-mscFv-GS3-hI2S (1.0 × 10¹³ vg/kg) administration group; (4) indicates results of an rAAV-mscFv-GS3-hI2S (1.0 × 10¹² vg/kg) administration group; (5) indicates results of an rAAV-mscFv-GS3-hI2S (1.0 × 10¹¹ vg/kg) administration group; (6) indicates results of an rAAV-mscFv2-GS3-hI2S (1.0 × 10¹³ vg/kg) administration group; (7) indicates results of an rAAV-mscFv2-GS3-hI2S (1.0 × 10¹² vg/kg) administration group; (8) indicates results of an rAAV-mscFv2-GS3-hI2S (1.0 × 10¹¹ vg/kg) administration group; (9) indicates results of an rAAV-hI2S-mscFv2 (1.0 × 10¹³ vg/kg) administration group; (10) indicates results of an rAAV-hI2S-mscFv2 (1.0 × 10¹² vg/kg) administration group; (11) indicates results of an rAAV-hI2S-mscFv2 (1.0 × 10¹¹ vg/kg) administration group; (12) indicates results of an rAAV-hI2S (1.0 × 10¹³ vg/kg) administration group; (13) indicates results of an rAAV-hI2S (1.0 × 10¹² vg/kg) administration group; and (14) indicates results of an rAAV-hI2S (1.0 × 10¹¹ vg/kg) administration group. Values are mean values and error bars indicate standard deviations (n = 3).

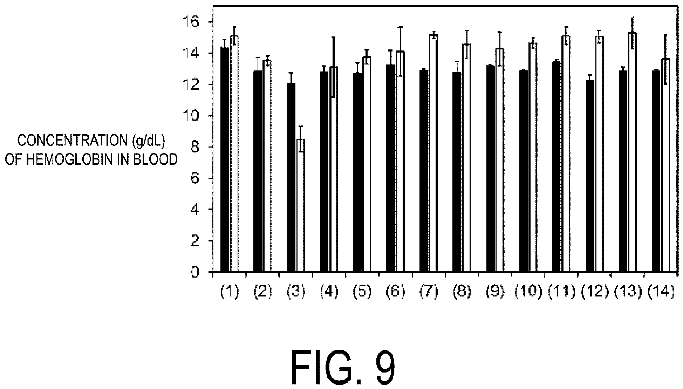

FIG. 9 is a graph illustrating results of measuring a concentration of hemoglobin in the blood (Example 19). The vertical axis of a bar graph represents the concentration (g/dL) of hemoglobin in the blood. A black bar represents a measured value of the concentration of hemoglobin in the blood one week after administration of each rAAV virion, and a white bar represents a measured value of the concentration of hemoglobin in the blood 6 weeks after administration of each rAAV virion. (1) indicates results of a normal control group; (2) indicates results of a pathological control group; (3) indicates results of an rAAV-mscFv-GS3-hI2S (1.0 × 10¹³ vg/kg) administration group; (4) indicates results of an rAAV-mscFv-GS3-hI2S (1.0 × 10¹² vg/kg) administration group; (5) indicates results of an rAAV-mscFv-GS3-hI2S (1.0 × 10¹¹ vg/kg) administration group; (6) indicates results of an rAAV-mscFv2-GS3-hI2S (1.0 × 10¹³ vg/kg) administration group; (7) indicates results of an rAAV-mscFv2-GS3-hI2S (1.0 × 10¹² vg/kg) administration group; (8) indicates results of an rAAV-mscFv2-GS3-hI2S (1.0 × 10¹¹ vg/kg) administration group; (9) indicates results of an rAAV-hI2S-mscFv2 (1.0 × 10¹³ vg/kg) administration group; (10) indicates results of an rAAV-hI2S-mscFv2 (1.0 × 10¹² vg/kg) administration group; (11) indicates results of an rAAV-hI2S-mscFv2 (1.0 × 10¹¹ vg/kg) administration group; (12) indicates results of an rAAV-hI2S (1.0 × 10¹³ vg/kg) administration group; (13) indicates results of an rAAV-hI2S (1.0 × 10¹² vg/kg) administration group; and (14) indicates results of an rAAV-hI2S (1.0 × 10¹¹ vg/kg) administration group. Values are mean values and error bars indicate standard deviations (n = 3).

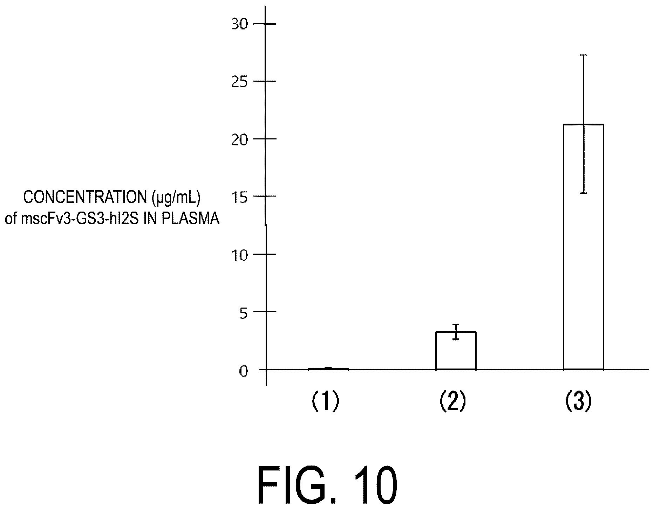

FIG. 10 is a graph illustrating results of measuring a concentration of mscFv3-GS3-hI2S in plasma (Example 26). The vertical axis of a bar graph represents the concentration (µg/mL) of mscFv3-GS3-hI2S in plasma. The vertical bar indicates the concentration of mscFv3-GS3-hI2S in plasma collected 6 weeks after rAAV-mscFv3-GS3-hI2S administration. (1) indicates results of a 1.0 × 10¹¹ vg/kg administration group, (2) indicates results of a 1.0 × 10¹² vg/kg administration group, and (3) indicates results of a 1.0 × 10¹³ vg/kg administration group. Values are mean values and error bars indicate standard deviations.

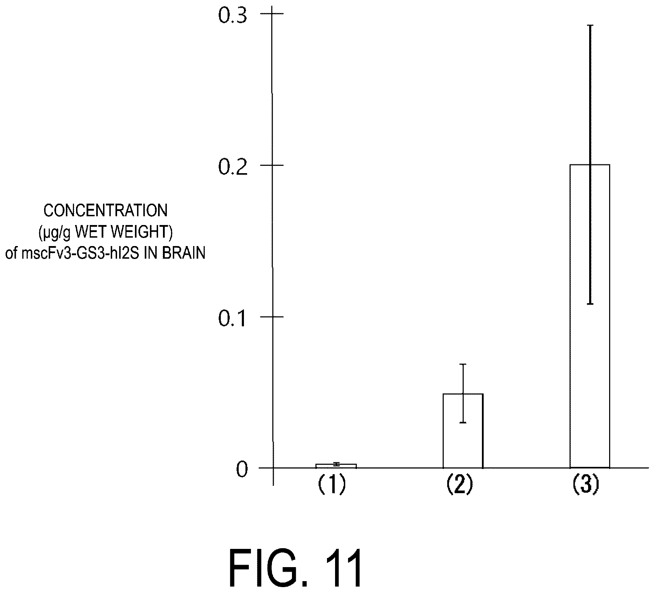

FIG. 11 is a graph illustrating results of measuring a concentration of mscFv3-GS3-hI2S in the brain (Example 26). The vertical axis of a bar graph represents the concentration (µg/g wet weight) of mscFv3-GS3-hI2S in the brain. The vertical bar indicates the concentration of rAAV-derived protein in the brain collected 6 weeks after rAAV-mscFv3-GS3-hI2S administration. (1) indicates results of a 1.0 × 10¹¹ vg/kg administration group, (2) indicates results of a 1.0 × 10¹² vg/kg administration group, and (3) indicates results of a 1.0 × 10¹³ vg/kg administration group. Values are mean values and error bars indicate standard deviations.

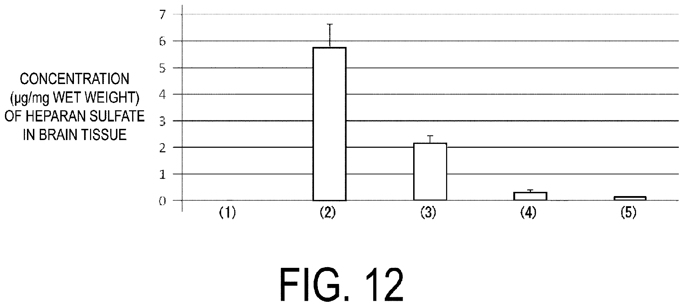

FIG. 12 is a graph illustrating results of measuring a concentration of heparan sulfate in the brain tissue (Example 27). The vertical axis of a bar graph represents the concentration (µg/mg wet weight) of heparan sulfate. The vertical bar indicates the concentration of heparan sulfate in the brain collected 6 weeks after rAAV-mscFv3-GS3-hI2S administration. (1) indicates results of a normal control group; (2) indicates results of a pathological control group; (3) indicates results of a 1.0 × 10¹¹ vg/kg administration group; (4) indicates results of a 1.0 × 10¹² vg/kg administration group; and (5) indicates results of a 1.0 × 10¹³ vg/kg administration group. Values are mean values and error bars indicate standard deviations.

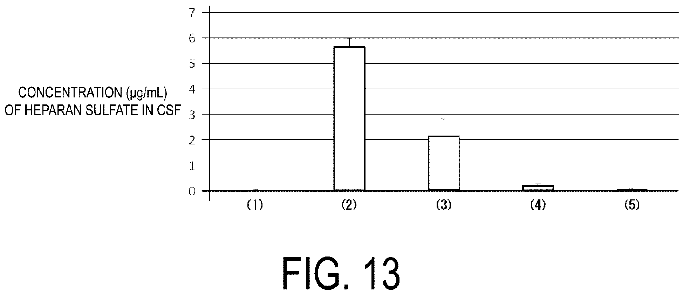

FIG. 13 is a graph illustrating results of measuring a concentration of heparan sulfate in CSF (Example 27). The vertical axis of a bar graph represents the concentration (µg/mg) of heparan sulfate. The vertical bar indicates the concentration of heparan sulfate in CSF collected 6 weeks after rAAV-mscFv3-GS3-hI2S administration. (1) indicates results of a normal control group; (2) indicates results of a pathological control group; (3) indicates results of a 1.0 × 10¹¹ vg/kg administration group; (4) indicates results of a 1.0 × 10¹² vg/kg administration group; and (5) indicates results of a 1.0 × 10¹³ vg/kg administration group. Values are mean values and error bars indicate standard deviations.

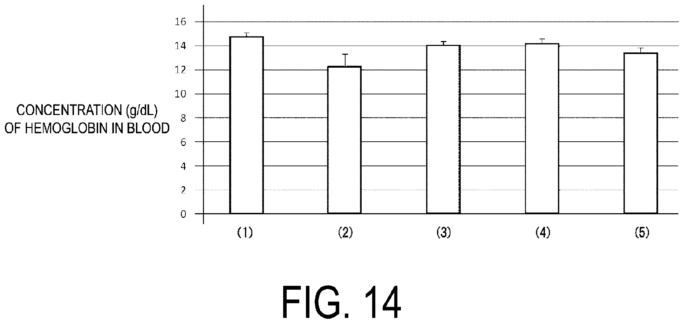

FIG. 14 is a graph illustrating results of measuring a concentration of hemoglobin in the blood (Example 28). The vertical axis of a bar graph represents the concentration (g/dL) of hemoglobin. The vertical bar indicates the concentration of hemoglobin in the blood collected 6 weeks after rAAV-mscFv3-GS3-hI2S administration. (1) indicates results of a normal control group; (2) indicates results of a pathological control group; (3) indicates results of a 1.0 × 10¹¹ vg/kg administration group; (4) indicates results of a 1.0 × 10¹² vg/kg administration group; and (5) indicates results of a 1.0 × 10¹³ vg/kg administration group. Values are mean values and error bars indicate standard deviations.

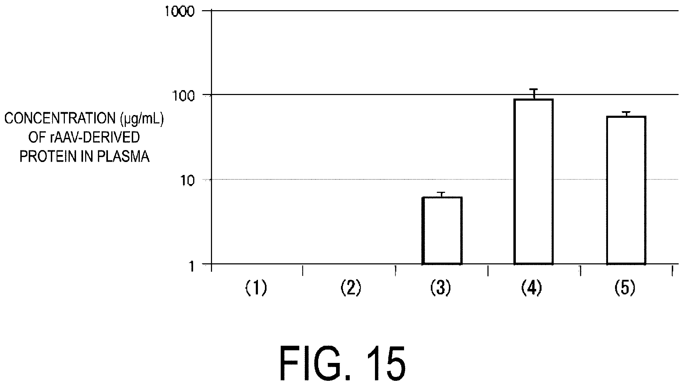

FIG. 15 is a graph illustrating results of measuring a concentration of rAAV-derived protein in plasma (Example 38). The vertical axis of a bar graph represents the concentration (µg/mL) of rAAV-derived protein in plasma. The vertical bar indicates the concentration of rAAV-derived protein in plasma collected 4 weeks after each rAAV virion administration. (1) indicates results of a normal control group; (2) indicates results of a pathological control group; (3) indicates results of an rAAV-hGAA (Pro)-GS-mscFv2 (1.0 × 10¹³ vg/kg) administration group, (4) indicates results of an rAAV-mscFv-GS3-hGAA (1.0 × 10¹³ vg/kg) administration group; and (5) indicates results of an rAAV-hGAA (1.0 × 10¹³ vg/kg) administration group. Values are mean values and error bars indicate standard deviations.

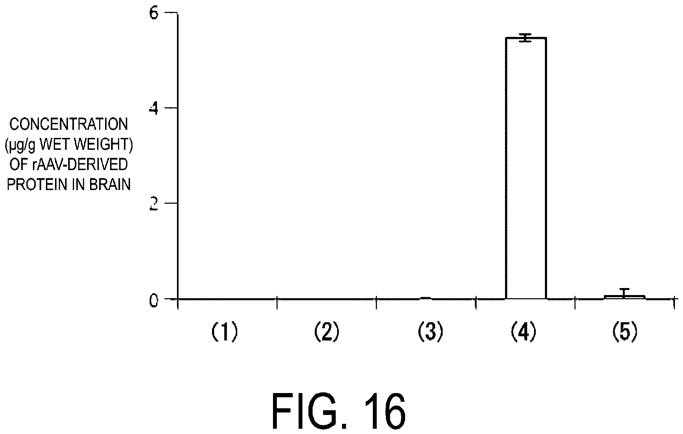

FIG. 16 is a graph illustrating results of measuring a concentration of rAAV-derived protein in the brain (Example 38). The vertical axis of a bar graph represents the concentration (µg/g wet weight). The vertical bar indicates the concentration of rAAV-derived protein in the brain collected 4 weeks after each rAAV virion administration. (1) indicates results of a normal control group; (2) indicates results of a pathological control group; (3) indicates results of an rAAV-hGAA (Pro)-GS-mscFv2 (1.0 × 10¹³ vg/kg) administration group, (4) indicates results of an rAAV-mscFv-GS3-hGAA (1.0 × 10¹³ vg/kg) administration group; and (5) indicates results of an rAAV-hGAA (1.0 × 10¹³ vg/kg) administration group. Values are mean values and error bars indicate standard deviations.

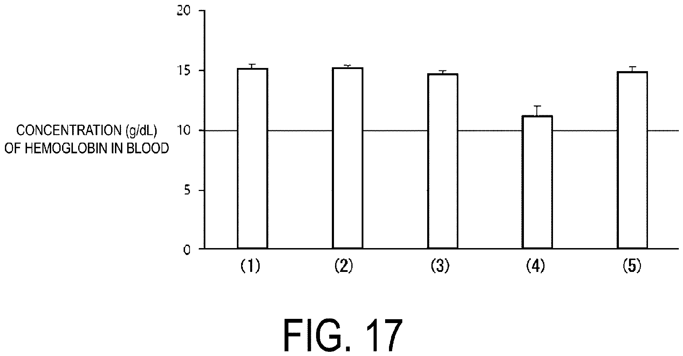

FIG. 17 is a graph illustrating results of measuring a concentration of hemoglobin in the blood (Example 40). The vertical axis of a bar graph represents the concentration (g/dL). The vertical bar indicates the concentration of hemoglobin in the blood collected 4 weeks after each rAAV virion administration. (1) indicates results of a normal control group; (2) indicates results of a pathological control group; (3) indicates results of an rAAV-hGAA (Pro)-GS-mscFv2 (1.0 × 10¹³ vg/kg) administration group, (4) indicates results of an rAAV-mscFv-GS3-hGAA (1.0 × 10¹³ vg/kg) administration group; and (5) indicates results of an rAAV-hGAA (1.0 × 10¹³ vg/kg) administration group. Values are mean values and error bars indicate standard deviations.

### Description of Embodiments

In the present invention, the term "nucleic acid molecule" mainly refers to either DNA in which deoxyribonucleotides are polymerized by phosphodiester bonds or RNA in which ribonucleotides are polymerized by phosphodiester bonds.

When the "nucleic acid molecule" is DNA, the DNA may be a single strand (single chain) or a double strand with a complementary strand. When the DNA is a single strand, the DNA may be a (+) strand or a (-) strand. Each deoxyribonucleotide contained in the DNA may be a naturally occurring deoxyribonucleotide or a modified deoxyribonucleotide of a naturally occurring deoxyribonucleotide, as long as a gene encoding a protein contained in the DNA can be translated into mRNA in mammalian (particularly, human) cells. In one embodiment of the present invention, each deoxyribonucleotide contained in the DNA may be a naturally occurring deoxyribonucleotide or a modified deoxyribonucleotide of a naturally occurring deoxyribonucleotide as long as a gene encoding a protein contained in the DNA can be translated into mRNA and all or a part of the DNA can be replicated in mammalian (particularly, human) cells.

Also, when the "nucleic acid molecule" is RNA, the RNA may be a single strand (single chain) or a double strand with a complementary strand. When the RNA is a single chain, the RNA may be a (+) strand or a (-) strand. In one embodiment of the present invention, each ribonucleotide contained in the RNA may be a naturally occurring ribonucleotide or a modified ribonucleotide as long as a gene encoding a protein contained in the RNA can be reverse-transcribed into DNA in mammalian (particularly, human) cells. Also, in one embodiment of the present invention, each ribonucleotide contained in the RNA may be a naturally occurring ribonucleotide or a modified ribonucleotide as long as a gene encoding a protein contained in the RNA can be translated into the protein in mammalian (particularly, human) cells. The ribonucleotide is modified, for example, for suppressing the degradation of the RNA by RNase and for enhancing the stability of the RNA in cells.

In one embodiment of the present invention, the term "inverted terminal repeat (ITR)" refers to a nucleotide sequence which is present at a terminus of a viral genome and in which a same sequence is repeated. Adeno-associated virus-derived and adenovirus-derived ITRs can be suitably used. For example, the adeno-associated viral ITR is a region having a chain length of about 145 nucleotides and functions as a replication origin or the like. In one embodiment of the present invention, there are two inverted terminal repeats (ITRs) in the nucleic acid molecule, which are referred to as a first inverted terminal repeat (ITR) and a second inverted terminal repeat (ITR), respectively. When a gene encoding a fusion protein is located between two ITRs, the ITR located on the 5' side is referred to as the first inverted terminal repeat (ITR), and the ITR located on the 3' side is referred to as the second inverted terminal repeat (ITR). In the present invention, the inverted terminal repeat (ITR) may be derived from any virus as long as it has at least one of the functions of the original ITR, such as the function as a replication origin and gene insertion into a host cell, and the adeno-associated viral ITR is one of suitable ITRs.

When the ITR is derived from adeno-associated virus, the serotype of AAV is not particularly limited, and may be any of serotypes 1, 2, 3, 4, 5, 6, 7, 8, 9, 10 and 11. For example, the inverted terminal repeat (ITR) of serotype 2 AAV, the first ITR contains a nucleotide sequence represented by SEQ ID NO: 5 and the second ITR contains a nucleotide sequence represented by SEQ ID NO: 6.

The ITR is not limited to a wild-type ITR, and may be a nucleotide sequence of the wild-type ITR modified by substitution, deletion, addition or the like. When a nucleotide in the nucleotide sequence of the wild-type ITR is substituted with another nucleotide, the number of nucleotides to be substituted is preferably from 1 to 20, more preferably from 1 to 10, and even more preferably from 1 to 3. When a nucleotide is deleted from the nucleotide sequence of the wild-type ITR, the number of nucleotides to be deleted is preferably from 1 to 20, more preferably from 1 to 10, and even more preferably from 1 to 3. In addition, a mutation combining such nucleotide substitution and deletion can also be performed. When a nucleotide is added to the wild-type ITR, preferably from 1 to 20, more preferably from 1 to 10, and even more preferably from 1 to 3 nucleotides are added into the nucleotide sequence or to the 5'-terminus or the 3'-terminus of the ITR. A mutation combining such nucleotide addition, substitution, and deletion can also be performed. The nucleotide sequence of the mutated ITR preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence of the wild-type ITR.

A functional equivalent of an ITR refers to an equivalent that can be used in place of an ITR from the functional viewpoint. An ITR artificially constructed based on the ITR is also a functional equivalent of the ITR as long as it can be substituted for the ITR.

A functional equivalent of an ITR of AAV refers to an equivalent that can be used in place of an ITR of AAV from the functional viewpoint. An ITR artificially constructed based on the ITR of AAV is also a functional equivalent of the ITR of AAV as long as it can be substituted for the ITR of AAV.

Examples of the artificially constructed functional equivalent of the first inverted terminal repeat of AAV (first AAV-ITR) include those having a nucleotide sequence represented by SEQ ID NO: 7 (functional equivalent of the first AAV-ITR). The functional equivalents of the first AAV-ITR also encompass a nucleotide sequence represented by SEQ ID NO: 7 to which substitution, deletion, or mutation is added as long as it can be used in place of the first AAV-ITR from the functional viewpoint. When a nucleotide in the nucleotide sequence represented by SEQ ID NO: 7 is substituted with another nucleotide, the number of nucleotides to be substituted is preferably from 1 to 20, more preferably from 1 to 10, even more preferably from 1 to 5, and even more preferably from 1 to 3. When a nucleotide in the nucleotide sequence represented by SEQ ID NO: 7 is deleted, the number of nucleotides to be deleted is preferably from 1 to 20, more preferably from 1 to 10, even more preferably from 1 to 5, and even more preferably from 1 to 3. The functional equivalents of the first AAV-ITR also encompass an ITR to which a mutation combining such nucleotide substitution and deletion has been added. When a nucleotide is added to the nucleotide sequence represented by SEQ ID NO: 7, preferably from 1 to 20, more preferably from 1 to 10, even more preferably from 1 to 5, and still more preferably from 1 to 3 nucleotides are added into the nucleotide sequence or to the 5'-terminus or the 3'-terminus. The functional equivalents of the first AAV-ITR also encompass an ITR to which a mutation combining such nucleotide addition, substitution and deletion is added. The nucleotide sequence of the mutated ITR preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 7.

Also, examples of the artificially constructed functional equivalent of the second inverted terminal repeat (second AAV-ITR) include those having a nucleotide sequence represented by SEQ ID NO: 8 (functional equivalent of the second AAV-ITR). The functional equivalents of the second AAV-ITR also encompass a nucleotide sequence represented by SEQ ID NO: 8 to which substitution, deletion, or mutation is added as long as it can be used in place of the second AAV-ITR from the functional viewpoint. When a nucleotide in the nucleotide sequence represented by SEQ ID NO: 8 is substituted with another nucleotide, the number of nucleotides to be substituted is preferably from 1 to 20, more preferably from 1 to 10, even more preferably from 1 to 5, and even more preferably from 1 to 3. When a nucleotide in the nucleotide sequence represented by SEQ ID NO: 8 is deleted, the number of nucleotides to be deleted is preferably from 1 to 20, more preferably from 1 to 10, even more preferably from 1 to 5, and even more preferably from 1 to 3. The functional equivalents of the second AAV-ITR also encompass an ITR to which a mutation combining such nucleotide substitution and deletion has been added. When a nucleotide is added to the nucleotide sequence represented by SEQ ID NO: 8, preferably from 1 to 20, more preferably from 1 to 10, even more preferably from 1 to 5, and still more preferably from 1 to 3 nucleotides are added into the nucleotide sequence or to the 5'-terminus or the 3'-terminus. The functional equivalents of the second AAV-ITR also encompass an ITR to which a mutation combining such nucleotide addition, substitution and deletion is added. The nucleotide sequence of the mutated ITR preferably exhibits an identity of 85% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 8.

In one embodiment of the present invention, the term "long terminal repeat (LTR)" refers to a nucleotide sequence which is present at a terminus of, for example, a eukaryotic retrotransposon, a retrovirus genome, a lentivirus genome, or the like and in which a same sequence is repeated several hundred to several thousand times. In one embodiment of the present invention, there are two long terminal repeats (LTRs) in the nucleic acid molecule, which are referred to as a first long terminal repeat (LTR) and a second long terminal repeat (LTR), respectively. When a gene encoding a fusion protein is located between two LTRs, the LTR located on the 5' side is referred to as the first long terminal repeat (LTR), and the LTR located on the 3' side is referred to as the second long terminal repeat (LTR). In the present invention, the long terminal repeat (LTR) may be derived from any virus as long as it has at least one of the functions that the LTR is supposed to have, such as the function as a replication origin and gene insertion into a host cell, and suitable examples thereof include a retrovirus genome and a lentivirus. The LTR is not limited to a wild-type LTR, and may be a nucleotide sequence of the wild-type LTR modified by substitution, deletion, addition, or the like.

When a nucleotide in the nucleotide sequence of the wild-type LTR is substituted with another nucleotide, the number of nucleotides to be substituted is preferably from 1 to 20, more preferably from 1 to 10, and even more preferably from 1 to 3. When a nucleotide is deleted from the nucleotide sequence of the wild-type LTR, the number of nucleotides to be deleted is preferably from 1 to 20, more preferably from 1 to 10, and even more preferably from 1 to 3. In addition, a mutation combining such nucleotide substitution and deletion can also be performed. When a nucleotide is added to the wild-type LTR, preferably from 1 to 20, more preferably from 1 to 10, and even more preferably from 1 to 3 nucleotides are added into the nucleotide sequence or to the 5'-terminus or the 3'-terminus of the LTR. A mutation combining such nucleotide addition, substitution, and deletion can also be performed. The nucleotide sequence of the mutated LTR preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence of the wild-type LTR.

A functional equivalent of an LTR refers to an equivalent that can be used in place of an LTR from the functional viewpoint. An LTR artificially constructed based on the LTR is also a functional equivalent of the LTR as long as it can be substituted for the LTR.

A functional equivalent of a lentiviral LTR refers to an equivalent that can be used in place of a lentiviral LTR from the functional viewpoint. An LTR artificially constructed based on the lentiviral LTR is also a functional equivalent of the lentiviral LTR as long as it can be substituted for the lentiviral LTR.

A functional equivalent of a retroviral LTR refers to an equivalent that can be used in place of a retroviral LTR from the functional viewpoint. An LTR artificially constructed based on the retroviral LTR is also a functional equivalent of the retroviral LTR as long as it can be substituted for the retroviral LTR.

Examples of functional equivalents of the first LTR include those having a base sequence represented by SEQ ID NO: 52. The functional equivalents of the first LTR also encompass a base sequence represented by SEQ ID NO: 52 to which substitution, deletion, or mutation is added as long as it can be used as the first LTR from the functional viewpoint. When a nucleotide in the nucleotide sequence represented by SEQ ID NO: 52 is substituted with another nucleotide, the number of nucleotides to be substituted is preferably from 1 to 20, more preferably from 1 to 20, even more preferably from 1 to 5, and even more preferably from 1 to 3. When a nucleotide in the nucleotide sequence represented by SEQ ID NO: 52 is deleted, the number of nucleotides to be deleted is preferably from 1 to 20, more preferably from 1 to 10, even more preferably from 1 to 5, and even more preferably from 1 to 3. The functional equivalents of the first LTR also encompass an LTR to which a mutation combining such nucleotide substitution and deletion has been added. When a nucleotide is added to the nucleotide sequence represented by SEQ ID NO: 52, preferably from 1 to 20, more preferably from 1 to 10, even more preferably from 1 to 5, and still more preferably from 1 to 3 nucleotides are added into the nucleotide sequence or to the 5'-terminus or the 3'-terminus. The functional equivalents of the first LTR also encompass an LTR to which a mutation combining such nucleotide addition, substitution and deletion is added. The nucleotide sequence of the mutated LTR preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 52.

Examples of functional equivalents of the second LTR include those having a nucleotide sequence represented by SEQ ID NO: 53. The functional equivalents of the second LTR also encompass a nucleotide sequence represented by SEQ ID NO: 53 to which substitution, deletion, or mutation is added as long as it can be used as the second LTR from the functional viewpoint. When a nucleotide in the nucleotide sequence represented by SEQ ID NO: 53 is substituted with another nucleotide, the number of nucleotides to be substituted is preferably from 1 to 20, more preferably from 1 to 10, even more preferably from 1 to 5, and even more preferably from 1 to 3. When a nucleotide in the nucleotide sequence represented by SEQ ID NO: 53 is deleted, the number of nucleotides to be deleted is preferably from 1 to 20, more preferably from 1 to 10, even more preferably from 1 to 5, and even more preferably from 1 to 3. The functional equivalents of the second LTR also encompass an LTR to which a mutation combining such nucleotide substitution and deletion has been added. When a nucleotide is added to the nucleotide sequence represented by SEQ ID NO: 53, preferably from 1 to 20, more preferably from 1 to 10, even more preferably from 1 to 5, and still more preferably from 1 to 3 nucleotides are added into the nucleotide sequence or to the 5'-terminus or the 3'-terminus. The functional equivalents of the second LTR also encompass an LTR to which a mutation combining such nucleotide addition, substitution and deletion is added. The nucleotide sequence of the mutated LTR preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 53.

In one embodiment of the present invention, the terms "leader" and "trailer" refer to nucleotide sequences that are present at terminals of a viral genome and are partially complementary to each other. As the leader and trailer, those derived from Sendai virus can be suitably used. Both the Sendai viral leader and trailer are regions having a chain length of about 50 nucleotides. Usually, the leader is located on the 5' side and the trailer is located on the 3' side.

A leader derived from Sendai virus having a nucleotide sequence represented by SEQ ID NO: 54 and a trailer derived from Sendai virus having a nucleotide sequence represented by SEQ ID NO: 55 are suitably used in one embodiment of the present invention.

Wild-type Sendai viral leader and trailer to which a mutation is added can also be suitably used in the present invention. When a nucleotide in the nucleotide sequence is substituted with another nucleotide, the number of nucleotides to be substituted is preferably from 1 to 20, more preferably from 1 to 10, and even more preferably from 1 to 3. When a nucleotide is deleted from the nucleotide sequence of the wild-type leader and/or trailer, the number of nucleotides to be deleted is preferably from 1 to 20, more preferably from 1 to 10, and even more preferably from 1 to 3. In addition, a mutation combining such nucleotide substitution and deletion can also be performed. When a nucleotide is added, preferably from 1 to 20, more preferably from 1 to 10, and even more preferably from 1 to 3 nucleotides are added into the nucleotide sequence(s) or to the 5'-terminus or the 3'-terminus of the leader and/or trailer. A mutation combining such nucleotide addition, substitution, and deletion can also be performed. The mutated nucleotide sequence preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the wild-type nucleotide sequence.

In one embodiment of the present invention, a nucleotide sequence that can be used as a nucleotide sequence containing a gene expression regulatory site that regulates expression of a gene of a fusion protein is not particularly limited as long as it is capable of expressing the fusion protein in a mammal (particularly, human) cell, tissue, or living body into which the gene encoding the fusion protein is introduced, but those including a cytomegalovirus-derived promoter (optionally including an enhancer), SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, a mouse albumin promoter, a human albumin promoter, and a human α-1 antitrypsin promoter are suitable. For example, a synthetic promoter having a nucleotide sequence represented by SEQ ID NO: 9 containing a mouse albumin promoter downstream of a mouse α-fetoprotein enhancer (mouse α-fetoprotein enhancer/mouse albumin promoter) can be suitably used as the gene expression regulatory site. A chicken β-actin/MVM chimeric intron having a nucleotide sequence represented by SEQ ID NO: 10 may be located downstream of the mouse α-fetoprotein enhancer/mouse albumin promoter. Such an intron is located, thereby making it possible to increase the expression level of the protein regulated at the gene expression regulatory site. In the nucleotide sequence represented by SEQ ID NO: 9, a nucleotide sequence at positions 1 to 219 is the mouse α-fetoprotein enhancer, and a nucleotide sequence at positions 241 to 549 is the mouse albumin promoter.

The gene regulatory site may be a promoter of a gene that is expressed in an organ-specific or cell-type-specific manner. The use of an organ-specific expression promoter or a cell-type-specific expression promoter can express, specifically in a desired organ or cell, the gene encoding the fusion protein incorporated into the nucleic acid molecule.

In one embodiment of the present invention, an "internal ribosome binding site" is a region (structure) which is present inside an mRNA chain, to which a ribosome can directly bind, and which initiates translation independent of a cap structure, or a region (structure) of a DNA chain which generates such a region when transcribed. In the present invention, a "gene encoding the internal ribosome binding site" is a region (structure) of a DNA chain which generates such a region when transcribed. The internal ribosome binding site is generally referred to as IRES (internal ribosome entry site) and is found in the 5' untranslated regions of viruses such as virus belonging to the family *Picornaviridae* (poliovirus, rhinovirus, mouse encephalomyocarditis virus, etc.), foot-and-mouth disease virus, hepatitis A virus, hepatitis C virus, coronavirus, bovine enterovirus, Theiler's murine encephalomyelitis virus, and Coxsackie type B virus and the 5' untranslated regions of genes of human immunoglobulin heavy chain binding protein, Drosophila antennapedia, Drosophila ultrabithorax, and the like. In the case of picornaviruses, the IRES is a region consisting of about the 450 bp, which is present in the 5' untranslated region of the mRNA. As used herein, the term "5' untranslated region of a virus" refers to the 5' untranslated region of a viral mRNA, or a region (structure) of a DNA chain which generates such a region when transcribed.

In one embodiment, the internal ribosome binding site is not particularly limited as long as it functions as the internal ribosome binding site in a mammalian (particularly, human) cell, tissue or living body, and any internal ribosome binding site can be used. Among them, preferred is an internal ribosome binding site derived from the 5' untranslated region of a virus, more preferred is an internal ribosome binding site derived from the 5' untranslated region of a virus belonging to the family *Picornaviridae,* and even more preferred is an internal ribosome binding site derived from the 5' untranslated region of mouse encephalomyocarditis virus. One embodiment of the internal ribosome binding site derived from the 5' untranslated region of mouse encephalomyocarditis virus has a nucleotide sequence represented by SEQ ID NO: 11.

In one embodiment, the internal ribosome binding site having a wild-type nucleotide sequence may be used as it is. Also, any mutant internal ribosome binding site in which one or two or more mutations (for example, substitution, deletion, and/or insertion) are added to the nucleotide sequence of the wild-type internal ribosome binding site can be used as long as it functions as the internal ribosome binding site in a mammalian (particularly, human) cell, tissue or living body. A chimera type internal ribosome binding site in which two or more internal ribosome binding sites are fused can also be used.

In one embodiment of the present invention, the physiologically active protein that binds to an anti-transferrin receptor antibody and forms a fusion protein is not particularly limited as long as it exhibits physiological activity in vivo. Suitable examples of the physiologically active protein include those to be administered to a patient as a medicament over a long period of time. Examples of such a medicament include a growth hormone, a lysosomal enzyme, a neurotrophic factor, a somatomedin, an insulin, a glucagon, a cytokine, a lymphokine, a blood coagulation factor, an antibody, a fusion protein of an antibody with another protein, granulocyte-macrophage colony-stimulating factor (GM-CSF), granulocyte-colony stimulating factor (G-CSF), macrophage-colony stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, DNasel, thyroid stimulating hormone (TSH), brain-derived neurotrophic factor (BDNF), nerve growth factor (NGF), ciliary neurotrophic factor (CNTF), glial-cell derived neurotrophic factor (GDNF), neurotrophin-3, neurotrophin-4/5, neurotrophin-6, neuregulin-1, activin, basic fibroblast growth factor (bFGF), fibroblast growth factor 2 (FGF2), epidermal growth factor (EGF), vascular endothelial growth factor (VEGF), interferon α, interferon β, interferon γ, interleukin-6, PD-1, a PD-1 ligand, tumor necrosis factor-α receptor (TNF-α receptor), an enzyme having beta-amyloid-degrading activity, etanercept, pegvisomant, metreleptin, abatacept, asfotase, a GLP-1 receptor agonist, and any one of antibody medicaments.

Suitable examples when the physiologically active protein is a lysosomal enzyme include α-L-iduronidase, iduronate-2-sulfatase, acid α-glucosidase, glucocerebrosidase, β-galactosidase, GM2 activating proteins, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoA α-glucosaminide N-acetyltransferase, N-acetylglucosamine-6-sulfatase, acid ceramidase, amylo-1,6-glucosidase, sialidase, palmitoylprotein thioesterase-1, tripeptidylpeptidase-1, hyaluronidase-1, CLN1 and CLN2.

In one embodiment of the present invention, the physiologically active protein is a human protein. Here, the protein may be a wild-type protein, or may be a mutated protein as long as it has an inherent physiological activity of the protein. As used herein, the expression that "a protein has an inherent physiological activity" means that the protein has a physiological activity that is 10% or more of the physiological activity of the wild-type protein of the protein. The physiological activity relative to the physiological activity of the wild-type protein is more preferably 20% or more, even more preferably 40% or more, still more preferably 50% or more, yet more preferably 80% or more, and even yet more preferably 90% or more.

When a nucleotide in the nucleotide sequence of the wild-type physiologically active protein is substituted with another nucleotide, the number of nucleotides to be substituted is preferably from 1 to 20, more preferably from 1 to 10, and even more preferably from 1 to 3. When a nucleotide in the nucleotide sequence of the wild-type physiologically active protein is deleted, the number of nucleotides to be deleted is preferably from 1 to 20, more preferably from 1 to 10, and even more preferably from 1 to 3. In addition, a mutation combining such nucleotide substitution and deletion can also be performed. When a nucleotide is added into the nucleotide sequence of the wild-type physiologically active protein, preferably from 1 to 20, more preferably from 1 to 10, and even more preferably from 1 to 3 nucleotides are added into the nucleotide sequence or to the 5'-terminus or the 3'-terminus of the protein. A mutation combining such nucleotide addition, substitution, and deletion can also be performed. The nucleotide sequence of the mutated physiologically active protein preferably exhibits an identity of 80% or more, preferably exhibits an identity of 85% or more, more preferably exhibits an identity of 90% or more, even more preferably exhibits an identity of 95% or more, and still more preferably exhibits an identity of 98% or more, with the nucleotide sequence of the wild-type physiologically active protein.

In the present invention, the position and manner (deletion, substitution, addition) of each mutation as compared with those of the wild-type protein can be easily confirmed by the alignment of the amino acid sequences of the wild-type and mutant proteins. Note that, in the present invention, the identity of the amino acid sequence of a mutant protein with the amino acid sequence of a wild-type protein can be easily calculated using a known homology calculation algorithm. Examples of such an algorithm include BLAST (Altschul SF. J Mol. Biol. 215. 403-10, (1990)), the similarity search method of Pearson and Lipman (Proc. Natl. Acad. Sci. USA. 85. 2444 (1988)), and the local homology algorithm of Smith and Waterman (Adv. Appl. Math. 2. 482-9 (1981)).

Furthermore, substitution of an amino acid in the amino acid sequence of the protein with another amino acid can occur, for example, in a family of amino acids that are related in their side chains and chemical properties. Such a substitution in a family of amino acids is expected not to result in a significant change in the function of the protein (i.e., to be a conservative amino acid substitution). Examples of such a family of amino acids include:
(1) aspartic acid and glutamic acid, which are acidic amino acids;
(2) histidine, lysine, and arginine, which are basic amino acids;
(3) phenylalanine, tyrosine, tryptophan, which are aromatic amino acids;
(4) serine and threonine, which are amino acids with a hydroxyl group (hydroxyamino acids);
(5) methionine, alanine, valine, leucine, and isoleucine, which are hydrophobic amino acids;
(6) cysteine, serine, threonine, asparagine, and glutamine, which are neutral hydrophilic amino acids;
(7) glycine and proline, which are amino acids affecting the orientation of peptide chains;
(8) asparagine and glutamine, which are amide amino acids (polar amino acids);
(9) alanine, leucine, isoleucine, and valine, which are aliphatic amino acids;
(10) alanine, glycine, serine, and threonine, which are amino acids with a small side chain;
(11) alanine and glycine, which are amino acids with a particularly small side chain.

The anti-transferrin receptor antibody contained in the fusion protein of the anti-transferrin receptor antibody and the physiologically active protein in the present invention will be described in detail below.

The anti-transferrin receptor antibody (anti-TfR antibody) is not particularly limited in animal species or the like of the anti-TfR antibody as long as it has a property of specifically binding to a transferrin receptor (TfR) as an antigen, but is particularly a human anti-TfR antibody or a humanized anti-TfR antibody. For example, the anti-TfR antibody may be an anti-TfR antibody of a mammal other than human, or may be a chimeric anti-TfR antibody of a human anti-TfR antibody and an anti-TfR antibody of another mammal other than human.

In one embodiment of the present invention, the mere term "anti-TfR antibody" is a generic concept of antibodies having a property of specifically binding to a transferrin receptor (TfR) of any one of animal species. Therefore, the antigen binding fragment which will be described below is also encompassed in the anti-TfR antibody as long as it has the property of specifically binding to the transferrin receptor (TfR).

The human anti-transferrin receptor antibody (human anti-TfR antibody) refers to an anti-TfR antibody entirely encoded by a human-derived gene. However, an anti-TfR antibody encoded by a gene in which an original human gene has been mutated for the purpose, for example, of increasing the expression efficiency of the gene is also a human anti-TfR antibody. In addition, an anti-TfR antibody in which a part of one human anti-TfR antibody has been replaced by a part of another human anti-TfR antibody by combining two or more genes encoding the human anti-TfR antibody is also a human anti-TfR antibody. The human anti-TfR antibody normally has three complementarity determining regions (CDRs) of an immunoglobulin light chain and three complementarity determining regions (CDRs) of an immunoglobulin heavy chain. The three CDRs of the immunoglobulin light chain are referred to as CDR1, CDR2, and CDR3 in the order from the N-terminus. The three CDRs of the immunoglobulin heavy chain are referred to as CDR1, CDR2, and CDR3 in the order from the N-terminus. An anti-TfR antibody in which the antigen specificity, affinity, and the like of a human anti-TfR antibody have been modified by replacing a CDR of one human anti-TfR antibody by a CDR of another human anti-TfR antibody is also a human anti-TfR antibody.

In the present invention, an antibody in which a mutation such as substitution, deletion, or addition has been added to the amino acid sequence of the original human anti-TfR antibody by modifying the gene of the original human anti-TfR antibody is also referred to as a human anti-TfR antibody. When an amino acid in the amino acid sequence of the original human anti-TfR antibody is substituted with another amino acid, the number of amino acids to be substituted is preferably from 1 to 20, more preferably from 1 to 10, even more preferably from 1 to 5, and still more preferably 1 to 3. When an amino acid in the amino acid sequence of the original human anti-TfR antibody is deleted, the number of amino acids to be deleted is preferably from 1 to 20, more preferably from 1 to 10, even more preferably from 1 to 5, and still more preferably 1 to 3. In addition, an antibody to which a mutation combining such amino acid substitution and deletion has been added is also the human anti-TfR antibody. When an amino acid is added, preferably from 1 to 20, more preferably from 1 to 10, even more preferably from 1 to 5, and still more preferably 1 to 3 amino acids are added into the amino acid sequence, or to the N-terminus or the C-terminus of the original human anti-TfR antibody. An antibody to which a mutation combining such amino acid addition, substitution and deletion has been added is also the human anti-TfR antibody. The amino acid sequence of the mutated human anti-TfR antibody preferably exhibits an identity of 80% or more, more preferably exhibits an identity of 90% or more, even more preferably exhibits an identity of 95% or more, and still more preferably exhibits an identity of 98% or more, with the amino acid sequence of the original human anti-TfR antibody. That is, the term "human-derived gene" in the present invention encompasses not only the original human-derived gene but also a gene in which the original human-derived gene has been modified.

In the present invention, the term "humanized anti-TfR antibody" refers to an anti-TfR antibody in which the amino acid sequence of a part of the variable region (e.g., in particular, all or some of the CDRs) is derived from a non-human mammal and another region is derived from human. Examples of the humanized anti-TfR antibody include an anti-TfR antibody prepared by replacing the three complementarity determining regions (CDRs) of an immunoglobulin light chain and the three complementarity determining regions (CDRs) of an immunoglobulin heavy chain contained in a human anti-TfR antibody by CDRs of another mammal. The organism species of another mammal from which the CDRs to be grafted at an appropriate position of the human anti-TfR antibody is derived is any non-human mammal without particular limitation but is preferably a mouse, a rat, a rabbit, a horse, or a non-human primate, more preferably a mouse and a rat, and for example, a mouse.

The case where the anti-TfR antibody is a human anti-TfR antibody or a humanized anti-TfR antibody in the present invention will be described in detail below. The light chains of human anti-TfR antibodies and humanized anti-TfR antibodies include a λ chain and a κ chain. The light chain contained in the anti-TfR antibody may be either a λ chain or a κ chain. In addition, the heavy chains of human anti-TfR antibodies and humanized anti-TfR antibodies include a γ chain, a µ chain, an α chain, a σ chain, and an ε chain, which correspond to IgG, IgM, IgA, IgD, and IgE, respectively. The heavy chain contained in the anti-TfR antibody may be a γ chain, a µ chain, an α chain, a σ chain, or an ε chain but is preferably a γ chain. Furthermore, γ chains of the heavy chains of anti-TfR antibodies include a γ1 chain, a γ2 chain, a γ3 chain, and a γ4 chain, which correspond to IgG1, IgG2, IgG3, and IgG4, respectively. When the heavy chain contained in the anti-TfR antibody is a γ chain, the γ chain may be a γ1 chain, a γ2 chain, a γ3 chain, or a γ4 chain but is preferably a γ1 chain or a γ4 chain. When the anti-TfR antibody is a humanized anti-TfR antibody or a human anti-TfR antibody and is IgG, the light chain of the anti-TfR antibody may be either a λ chain or a κ chain, and the heavy chain of the anti-TfR antibody may be a γ1 chain, a γ2 chain, a γ3 chain, or a γ4 chain but is preferably a γ1 chain or a γ4 chain. For example, a preferable embodiment of anti-TfR antibody includes an antibody in which the light chain is a λ chain and the heavy chain is a γ1 chain.

In the present invention, the term "chimeric anti-TfR antibody" refers to an anti-TfR antibody in which fragments of two or more different anti-TfR antibodies derived from two or more different species are linked.

A chimeric anti-TfR antibody between a human anti-TfR antibody and an anti-TfR antibody of another mammal is an anti-TfR antibody in which a part of a human anti-TfR antibody is replaced with a part of an anti-TfR antibody of a mammal other than human. An anti-TfR antibody is composed of an Fc region, a Fab region and a hinge region as described below. A specific example of such chimeric anti-TfR antibodies is that in which the Fc region is derived from a human anti-TfR antibody, while the Fab region is derived from an anti-TfR antibody of another mammal. The hinge region may be derived from either a human anti-TfR antibody or an anti-TfR antibody of another mammal. Conversely, a specific example of such chimeric anti-TfR antibodies also is a chimeric anti-TfR antibody in which the Fc region is derived from another mammal while the Fab region is derived from a human anti-TfR antibody. The hinge region may be derived from either a human anti-TfR antibody or an anti-TfR antibody of another mammal. The same applies to humanized anti-TfR antibodies.

It can also be said that the anti-TfR antibody is composed of a variable region and a constant region. Other specific examples of the chimeric anti-TfR antibody is that in which the constant region (CH) of the heavy chain and the constant region (CL) of the light chain are derived from a human anti-TfR antibody, while the variable region (VH) of the heavy chain and the variable region (VL) of the light chain are derived from an anti-TfR antibody of another mammal, and, conversely, that in which the constant region (CH) of the heavy chain and the constant region (CL) of the light chain are derived from an anti-TfR antibody of another mammal, while the variable region (VH) of the heavy chain and the variable region (VL) of the light chain are derived from a human anti-TfR antibody. The organism species of another mammal is derived is any non-human mammal without particular limitation but is preferably a mouse, a rat, a rabbit, a horse, or a non-human primate, and more preferably a mouse. The same applies to humanized anti-TfR antibodies. In one embodiment of the present invention, the mere term "heavy chain" can be appropriately rephrased as "a portion containing the variable region of the heavy chain", and the mere term "light chain" can be appropriately rephrased as "a portion containing the variable region of the light chain".

A chimeric anti-TfR antibody of a human anti-TfR antibody and a mouse anti-TfR antibody is particularly referred to as a "human/mouse chimeric anti-TfR antibody". The human/mouse chimeric anti-TfR antibody includes chimeric anti-TfR antibodies in which the Fc region is derived from a human anti-TfR antibody while the Fab region is derived from a mouse anti-TfR antibody, and, conversely, chimeric anti-TfR antibodies in which the Fc region is derived from a mouse anti-TfR antibody while the Fab region is derived from a human anti-TfR antibody. The hinge region may be derived from either a human anti-TfR antibody or a mouse anti-TfR antibody. Other specific examples of the human/mouse chimeric anti-TfR antibody is that in which the constant region (CH) of the heavy chain and the constant region (CL) of the light chain are derived from a human anti-TfR antibody, while the variable region (VH) of the heavy chain and the variable region (VL) of the light chain are derived from a mouse anti-TfR antibody, and, conversely, that in which the constant region (CH) of the heavy chain and the constant region (CL) of the light chain are derived from a mouse anti-TfR antibody, while the variable region (VH) of the heavy chain and the variable region (VL) of the light chain are derived from a human anti-TfR antibody. The same applies to humanized anti-TfR antibodies.

The anti-TfR antibody originally has a basic structure composed of a total of four polypeptide chains, i.e., two immunoglobulin light chains and two immunoglobulin heavy chains. However, in the present invention, the term "anti-TfR antibody" encompasses, in addition to an antibody having this basic structure:
(1) an antibody composed of a total of two polypeptide chains, i.e., one immunoglobulin light chain and one immunoglobulin heavy chain, and
(2) a single chain antibody in which a linker is bound to the C-terminus of an immunoglobulin light chain and further an immunoglobulin heavy chain is bound to the C-terminus of the linker,
(3) a single chain antibody in which a linker is bound to the C-terminus of an immunoglobulin heavy chain and further an immunoglobulin light chain is bound to the C-terminus of the linker,
(4) a single chain antibody (scFv) in which a linker is bound to the C-terminus of a variable region of an immunoglobulin heavy chain and further a variable region of an immunoglobulin light chain is bound to the C-terminus of the linker, and
(5) a single chain antibody (scFv) in which a linker is bound to the C-terminus of a variable region of an immunoglobulin light chain and further a variable region of an immunoglobulin heavy chain is bound to the C-terminus of the linker, as will be described in detail below. Also, the "antibody" of the present invention encompasses
(6) an antibody composed of a Fab region in which the Fc region has been deleted from the original basic structure of the antibody, and an antibody composed of the Fab region and all or a part of the hinge region (Fab, F(ab') and F(ab')₂), and
(7) a single domain antibody. Furthermore, the "antibody" of the present invention also encompasses scFv in which a variable region of a light chain and a variable region of a heavy chain are linked via a linker to form a single chain antibody. Furthermore, the "antibody" of the present invention also encompasses antibodies containing all or a part of an Fc region having a modified amino acid sequence and thus having affinity for TfR.

In the present invention, the term "linker" refers to, for example, a linker composed of a peptide chain in which a plurality of amino acids are bound by peptide bonds. A linker composed of such a peptide chain can also be referred to as a "peptide linker". The "linker" may also be rephrased as "linker sequence" in the context of the present specification. The N-terminus of the linker and the C-terminus of a certain protein are bound by a peptide bond, and the N-terminus of another protein is further bound to the C-terminus of the linker, whereby the two proteins form a conjugate via the linker.

An antibody having a basic structure composed of a total of four polypeptide chains, i.e., two light chains and two heavy chains has three complementarity determining regions (CDRs) in the variable region of the light chain (V_{L}) and three complementarity determining regions (CDRs) in the variable region of the heavy chain (V_{H}). The three CDRs of the light chain are referred to as CDR1, CDR2, and CDR3 in the order from the N-terminus. The three CDRs of the heavy chain are also referred to as CDR1, CDR2, and CDR3 in the order from the N-terminus. However, even if a part or all of these CDRs are incomplete or absent, they are encompassed in the antibody as long as they have a property of specifically binding to a specific antigen. The regions, other than the CDRs, of the light and heavy chain variable regions (V_{L} and V_{H}) are referred to as framework regions (FRs). The FRs are referred to as FR1, FR2, FR3 and FR4 in order from the N-terminus. Usually, the CDRs and FRs are present in the order of FR1, CDR1, FR2, CDR2, FR3, CDR3, and FR4 from the N-terminus.

In one embodiment of the present invention, the Fab refers to a molecule in which one light chain containing a variable region and a constant region of the light chain (C_{L} region) and one heavy chain containing a variable region and part 1 of a constant region of the heavy chain (C_{H}1 region) are linked by a disulfide bond between cysteine residues present in each chain. In the Fab, the heavy chain may further contain a part of a hinge region in addition to the variable region and the part 1 of the constant region of the heavy chain (C_{H}1 region), but in this case the hinge region lacks a cysteine residue that is otherwise present in the hinge region and links the heavy chains of the antibody to each other. In the Fab, the light chain and the heavy chain are linked by a disulfide bond formed between a cysteine residue present in the constant region (C_{L} region) of the light chain and a cysteine residue present in the constant region (C_{H}1 region) or the hinge region of the heavy chain. The heavy chain forming the Fab is referred to as a Fab heavy chain. The Fab lacks the cysteine residue, which is otherwise present in the hinge region and links the heavy chains of the antibody to each other, and thus is composed of one light chain and one heavy chain. The light chain contained in the Fab contains the variable region and the C_{L} region. The heavy chain contained in the Fab may be composed of the variable region and the C_{H}1 region or may contain a part of the hinge region in addition to the variable region and the C_{H}1 region. In this case, however, the hinge region is selected to contain no cysteine residue for linking between the heavy chains and thus does not form a disulfide bond between the two heavy chains in the hinge region. In F(ab'), the heavy chain contains, in addition to the variable region and the C_{H}1 region, all or a part of the hinge region containing a cysteine residue that links the heavy chains. F(ab')₂ refers to a molecule in which two F(ab')s are bound by a disulfide bond between cysteine residues present in their hinge regions. The heavy chain forming F(ab') or F(ab')₂ is referred to as a Fab' heavy chain. Further, a polymer such as a dimer or a trimer formed by binding a plurality of antibodies directly or via a linker is also an antibody. Furthermore, without being limited to these, the "antibody" referred to in the present invention encompasses any substance that contains a part of an antibody molecule and has the property of specifically binding to an antigen. That is, the "light chain" referred to in the present invention encompasses those derived from a light chain and having an amino acid sequence of all or a part of the variable region thereof. Also, the "heavy chain" includes those derived from a heavy chain and having an amino acid sequence of all or a part of the variable region thereof. Therefore, for example, a substance lacking the Fc region is also the heavy chain, as long as it has an amino acid sequence of all or a part of the variable region.

As used herein, the term "Fc" or "Fc region" refers to a region containing a fragment composed of the C_{H}2 region (part 2 of the constant region of the heavy chain) and the C_{H}3 region (part 3 of the constant region of the heavy chain) in an antibody molecule.

Furthermore, an antibody according to one embodiment of the present invention contains:
(8) scFab, scF(ab'), and scF(ab')₂ in which a light chain and a heavy chain contained in Fab, F(ab'), or F(ab')₂ indicated in (6) above are bound via a linker sequence to form single chain antibodies, respectively. Here in scFab, scF(ab'), and scF(ab')₂, the linker sequence may be bound to the C-terminus of the light chain, and the heavy chain may be further bound to the C-terminus thereof, or the linker may be bound to the C-terminus of the heavy chain, and the light chain may be further bound to the C-terminus thereof. Furthermore, the antibody of the present invention also encompasses scFv in which a variable region of a light chain and a variable region of a heavy chain are linked via a linker to form a single chain antibody. In the scFv, the linker sequence may be bound to the C-terminus of the variable region of the light chain and the variable region of the heavy chain may be further linked to the C-terminus of the linker, or the linker sequence may be bound to the C-terminus of the variable region of the heavy chain and the variable region of the light chain may be further bound to the C-terminus of the linker.

Furthermore, the "antibody" referred to herein includes, in addition to full-length antibodies and those described in (1) to (8) above, all forms in which a part of a full-length antibody has been deleted, which is a broader concept encompassing (1) to (8).

The term "antigen binding fragment (antibody fragment)" refers to a fragment of an antibody that retains at least a part of its specific binding activity with an antigen. Examples of the binding fragment include Fab, Fab', F(ab')₂, a variable region (Fv), a single chain antibody (scFv) in which a heavy chain variable region (V_{H}) and a light chain variable region (V_{L}) are linked by an appropriate linker, a diabody that is a dimer of a polypeptide including a heavy chain variable region (V_{H}) and a light chain variable region (V_{L}), a minibody that is a dimer of a molecule in which a part of a constant region (C_{H}3) binds to a heavy chain (H chain) of scFv, and any other low-molecular-weight antibodies. The antigen binding fragment also encompasses heavy chain antibodies, light chain antibodies, VHH, VNAR , as well as the same lacking portions thereof. However, it is not limited to these molecules as long as it has the ability to bind to an antigen.

In the present invention, the term "single chain antibody" refers to a protein in which a linker sequence binds to the C-terminus of an amino acid sequence including all or a part of a variable region of an immunoglobulin light chain, and an amino acid sequence including all or a part of a variable region of an immunoglobulin heavy chain binds to the C-terminus of the linker sequence, and which can specifically bind to a specific antigen. The "single chain antibody" in the present also encompasses a protein in which a linker sequence binds to the C-terminus of an amino acid sequence including all or a part of a variable region of an immunoglobulin heavy chain, and an amino acid sequence including all or a part of a variable region of an immunoglobulin light chain binds to the C-terminus of the linker sequence, and which can specifically bind to a specific antigen. For example, those indicated in (2) and (3) above are encompassed in the single chain antibody. In the single chain antibody in which an immunoglobulin light chain binds to the C-terminus of an immunoglobulin heavy chain via a linker sequence, the immunoglobulin heavy chain usually lacks the Fc region. The variable region of an immunoglobulin light chain has three complementarity determining regions (CDRs) that are responsible for the antigen specificity of an antibody. Similarly, the variable region of an immunoglobulin heavy chain has three CDRs. These CDRs are main regions that determine the antigen specificity of an antibody. Thus, the single chain antibody preferably contains all three CDRs of an immunoglobulin heavy chain and all three CDRs of an immunoglobulin light chain. However, as long as the antigen-specific affinity of the anti-transferrin receptor antibody is maintained, a single chain antibody in which one or more CDRs have been deleted can also be used.

In the single chain antibody, the linker situated between the light chain and the heavy chain of an immunoglobulin is a peptide chain composed of preferably from 2 to 50, more preferably from 8 to 50, even more preferably from 10 to 30, still more preferably from 12 to 18 or from 15 to 25, and, for example, from 15 or 25 amino acid residues. Such a linker is not limited in its amino acid sequence as long as the anti-transferrin receptor antibody formed by linking both chains thereby retains the affinity for the transferrin receptor, but is preferably composed of glycine alone or glycine and serine, and has, for example, an amino acid sequence Gly-Ser, an amino acid sequence Gly-Gly-Ser, an amino acid sequence Gly-Gly-Gly, SEQ ID NO: 1, SEQ ID NO: 2, SEQ ID NO: 3, SEQ ID NO: 4, or a sequence in which these amino acid sequences are repeated 2 to 10 times or 2 to 5 times. For example, when the variable region of the immunoglobulin light chain is bound to the C-terminus of the amino acid sequence composed of the entire variable region of the immunoglobulin heavy chain via a linker to form an scFv, a linker having the sequence of SEQ ID NO: 4 is suitably used.

The antibody in one embodiment of the present invention is an antibody derived from a camelid (including alpaca). Some camelid antibodies are composed of two heavy chains linked by a disulfide bond. The antibody composed of two heavy chains is referred to as a heavy chain antibody. The VHH is an antibody composed of one heavy chain containing the variable region of the heavy chain contained in the heavy chain antibody or an antibody composed of one heavy chain lacking the constant region (CH) contained in the heavy chain antibody. The VHH is also one of the antibodies in the embodiments of the present invention. The antibody according to one embodiment of the present invention also encompasses an antibody in which, for reducing the antigenicity of an antibody (including VHH) derived from a camelid when administered to a human, a mutation has been added to the amino acid sequence of a camelid antibody. When a mutation is added to the amino acids of the camelid antibody, a mutation can be added that is similar to the mutation that can be added to the antibodies described herein. In addition, an antibody composed of two light chains linked by a disulfide bond is also one of the antibodies in the embodiments of the present invention. The antibody composed of two light chains is referred to as a light chain antibody.

The antibody in one embodiment of the present invention is an antibody derived from shark. The shark antibody is composed of two heavy chains linked by a disulfide bond. The antibody composed of two heavy chains is referred to as a heavy chain antibody. The VNAR is an antibody composed of one heavy chain containing the variable region of the heavy chain contained in the heavy chain antibody or an antibody composed of one heavy chain lacking the constant region (CH) contained in the heavy chain antibody. The VNAR is also one of the antibodies in the embodiments of the present invention. The antibody according to one embodiment of the present invention also encompasses an antibody in which, for reducing the antigenicity of an antibody (including VNAR) derived from shark when administered to a human, a mutation has been added to the amino acid sequence of a shark antibody. When a mutation is added to the amino acids of the shark antibody, a mutation can be added that is similar to the mutation that can be added to the antibodies described herein. A humanized shark antibody is also one of the antibodies in the embodiments of the present invention.

In one embodiment of the present invention, the "single domain antibody" refers to an antibody having the property of specifically binding to an antigen in a single variable region. The single domain antibody encompasses antibodies whose variable region consists only of the variable region of the heavy chain (heavy chain single domain antibodies) and antibodies whose variable region consists only of the variable region of the light chain (light chain single domain antibodies). VHH and VNAR are types of single domain antibodies.

In one embodiment of the present invention, the anti-TfR antibody has a specific affinity for TfR and is therefore capable of binding to TfR present on surfaces of cerebrovascular endothelial cells. The anti-TfR antibody bound to TfR can pass through the blood-brain barrier (BBB). Therefore, by forming a fusion protein in which a protein having a desired physiological activity is bound to TfR, the fusion protein can pass through the blood-brain barrier (BBB) and the physiological activity of the protein can be exhibited in the brain. In other words, such a fusion protein can be used as a medicament that should exert its efficacy in the brain.

In the present invention, the term "human transferrin receptor" or "hTfR" refers to a membrane protein having an amino acid sequence shown in SEQ ID NO: 12. In one embodiment, the anti-transferrin receptor antibody of the present invention specifically binds to a portion (extracellular region of hTfR) from the cysteine residue at position 89 from the N-terminus to phenylalanine at the C-terminus in the amino acid sequence represented by SEQ ID NO: 12, but is not limited thereto.

In one embodiment, the light chain of the anti-hTfR antibody, which is a Fab, has an amino acid sequence represented by SEQ ID NO: 40 or 41 as the amino acid sequence of the light chain CDR1 of the anti-hTfR antibody, an amino acid sequence represented by SEQ ID NO: 42 or 43 as the amino acid sequence of the light chain CDR2 of the hTfR antibody, and an amino acid sequence represented by SEQ ID NO: 44 as the amino acid sequence of the light chain CDR3 of the hTfR antibody. In one embodiment, the heavy chain of the anti-hTfR antibody, which is a Fab, has an amino acid sequence represented by SEQ ID NO: 45 or 46 as the amino acid sequence of the heavy chain CDR1 of the anti-hTfR antibody, an amino acid sequence represented by SEQ ID NO: 47 or 48 as the amino acid sequence of the heavy chain CDR2 of the hTfR antibody, and an amino acid sequence represented by SEQ ID NO: 49 or 50 as the amino acid sequence of the heavy chain CDR3 of the hTfR antibody.

In one embodiment, the Fab anti-hTfR antibody contains a light chain having an amino acid sequence represented by SEQ ID NO: 15 and a heavy chain having an amino acid sequence represented by SEQ ID NO: 16.

Examples of a binding form between the anti-TfR antibody and the physiologically active protein in the fusion protein of the anti-TfR antibody and the physiologically active protein can include the following (a) to (h):
(a) the anti-TfR antibody is a single chain antibody in which the light chain of the antibody binds to the C-terminus of the heavy chain of the antibody, and the physiologically active protein binds to the N-terminus of the single chain antibody directly or via a linker;
(b) the anti-TfR antibody is a single chain antibody in which the light chain of the antibody binds to the C-terminus of the heavy chain of the antibody, and the physiologically active protein binds to the C-terminus of the single chain antibody directly or via a linker;
(c) the anti-TfR antibody is a single chain antibody in which the heavy chain of the antibody binds to the C-terminus of the light chain of the antibody, and the physiologically active protein binds to the N-terminus of the single chain antibody directly or via a linker;
(d) the anti-TfR antibody is a single chain antibody in which the heavy chain of the antibody binds to the C-terminus of the light chain of the antibody, and the physiologically active protein binds to the C-terminus of the single chain antibody directly or via a linker;
(e) the anti-TfR antibody is an antibody containing at least one light chain and at least one heavy chain, and the physiologically active protein binds to the C-terminus of the light chain of the antibody directly or via a linker;
(f) the anti-TfR antibody is an antibody containing at least one light chain and at least one heavy chain, and the physiologically active protein binds to the N-terminus of the light chain of the antibody directly or via a linker;
(g) the anti-TfR antibody is an antibody containing at least one light chain and at least one heavy chain, and the physiologically active protein binds to the C-terminus of the heavy chain of the antibody directly or via a linker;
(h) the anti-TfR antibody is an antibody containing at least one light chain and at least one heavy chain, and the physiologically active protein binds to the N-terminus of the heavy chain of the antibody directly or via a linker.

In the above (a) to (h), as needed, "the light chain" and "the variable region of the light chain" are interchangeable, and "the heavy chain" and "the variable region of the light chain" are interchangeable.

In (a) to (h) above, when a linker sequence is disposed between the anti-TfR antibody and the fusion protein of the physiologically active protein, the sequence is composed of preferably from 1 to 50 amino acids, more preferably from 1 to 17 amino acids, even more preferably from 1 to 10 amino acids, and still more preferably from 1 to 5 amino acids. Depending on the protein to be bound to the anti-TfR antibody, the number of amino acids contained in the linker sequence can be appropriately adjusted to 1, 2, 3, from 1 to 17, from 1 to 10, from 10 to 40, from 20 to 34, from 23 to 31, from 25 to 29, or the like. The amino acid sequence of such a linker sequence is not particularly limited as long as the anti-TfR antibody linked by the linker sequence retains the affinity for TfR and the protein linked by the linker sequence can exhibit the physiological activity of the protein under physiological conditions. The amino acid sequence is preferably composed of glycine and serine, for example, composed of one amino acid of either glycine or serine, or has an amino acid sequence Gly-Ser, an amino acid sequence Gly-Gly-Ser, SEQ ID NO: 1, SEQ ID NO: 2, SEQ ID NO: 3, SEQ ID NO: 4, or a sequence composed of from 1 to 50, from 2 to 17, from 2 to 10, from 10 to 40, from 20 to 34, from 23 to 31, or from 25 to 29 amino acids, which contains successively from 1 to 10 or from 2 to 5 of these amino acid sequences. For example, those having the amino acid sequence Gly-Ser, those having the amino acid sequence represented by SEQ ID NO: 4, and those composed of 17 amino acids with Gly-Ser added at the C-terminus of the amino acid sequence represented by SEQ ID NO: 4 can be suitably used as the linker sequence.

For convenience, in the present invention, a linker sequence (linker) that binds the light chain or heavy chain of the anti-TfR antibody to the physiologically active protein is referred to as a linker sequence (linker) or a first linker sequence (first linker), and a linker sequence (linker) that binds the light chain and the heavy chain in the anti-TfR antibody that is a single chain antibody is referred to as a second linker sequence (second linker).

A nucleic acid molecule in one embodiment of the present invention contains a gene encoding a fusion protein of an anti-TfR antibody and a physiologically active protein between a first inverted terminal repeat (ITR) and a second inverted terminal repeat (ITR). The following (1) to (4) are examples of the nucleic acid molecule:
(1) a nucleic acid molecule wherein a fusion protein contains a conjugate in which a physiologically active protein is bound to the C-terminus or the N-terminus of a heavy chain of an anti-TfR antibody directly or via a linker and a light chain of the anti-TfR antibody, and the nucleic acid molecule contains a nucleotide sequence encoding an internal ribosome binding site downstream of a gene encoding the light chain, and downstream thereof a nucleotide sequence encoding the conjugate;
(2) a nucleic acid molecule wherein a fusion protein contains a conjugate in which a physiologically active protein is bound to the C-terminus or the N-terminus of a heavy chain of an anti-TfR antibody directly or via a linker and a light chain of the anti-TfR antibody, and the nucleic acid molecule contains a nucleotide sequence encoding an internal ribosome binding site downstream of a gene encoding the conjugate, and downstream thereof a nucleotide sequence encoding the light chain;
(3) a nucleic acid molecule wherein a fusion protein contains a conjugate in which a physiologically active protein is bound to the C-terminus or the N-terminus of a light chain of an anti-TfR antibody directly or via a linker and a heavy chain of the anti-TfR antibody, and the nucleic acid molecule contains a nucleotide sequence encoding an internal ribosome binding site downstream of a gene encoding the heavy chain, and downstream thereof a nucleotide sequence encoding the conjugate;
(4) a nucleic acid molecule wherein a fusion protein contains a conjugate in which a physiologically active protein is bound to the C-terminus or the N-terminus of a light chain of an anti-TfR antibody directly or via a linker and a heavy chain of the anti-TfR antibody, and the nucleic acid molecule contains a nucleotide sequence encoding an internal ribosome binding site downstream of a gene encoding the conjugate, and downstream thereof a nucleotide sequence encoding the heavy chain.

In (1) to (4) above, the nucleic acid molecule may contain a nucleotide sequence containing a gene expression regulatory site between the first inverted terminal repeat (ITR) and the gene encoding the fusion protein. Also, in (1) to (4) above, the nucleotide sequence encoding the internal ribosome binding site may be replaced with a nucleotide sequence containing a gene expression regulatory site. Without any limitation to these cases, when the nucleic acid molecule has two gene expression regulatory sites, the first gene expression regulatory site and the first gene expression regulatory site are defined in this order from the first inverted terminal repeat (ITR) side for convenience. In (1) to (4) above, the nucleotide sequence encoding the internal ribosome binding site may be replaced with a nucleotide sequence encoding a 2A self-cleaving peptide. A suitable example of the 2A self-cleaving peptide is a 2A peptide derived from porcine teschovirus. In the above (1) to (4), the light chain can be appropriately read as the variable region of the light chain, and the heavy chain can be appropriately read as the variable region of the heavy chain.

When the anti-TfR antibody is a single chain antibody, a nucleic acid molecule in one embodiment of the present invention contains a gene encoding a fusion protein of a single chain anti-TfR antibody and a physiologically active protein between a first inverted terminal repeat (ITR) and a second inverted terminal repeat (ITR). The following (1) to (4) are examples of the nucleic acid molecule:
(1) a nucleic acid molecule containing a gene encoding a fusion protein in which a light chain is bound to the C-terminus of a heavy chain of an anti-TfR antibody via a second linker and further a physiologically active protein is bound to the C-terminus of the light chain directly or via a linker;
(2) a nucleic acid molecule containing a gene encoding a fusion protein in which a heavy chain is bound to the C-terminus of a light chain of an anti-TfR antibody via a second linker and further a physiologically active protein is bound to the C-terminus of the light chain directly or via a linker;
(3) a nucleic acid molecule containing a gene encoding a fusion protein in which a heavy chain of an anti-TfR antibody is bound to the C-terminus of a physiologically active protein directly or via a linker and further a light chain is bound to the C-terminus of the heavy chain;
(4) a nucleic acid molecule containing a gene encoding a fusion protein in which a light chain of an anti-TfR antibody is bound to the C-terminus of a physiologically active protein directly or via a linker and further a heavy chain is bound to the C-terminus of a heavy chain.

In (1) to (4) above, the nucleic acid molecule may contain a nucleotide sequence containing a gene expression regulatory site between the first inverted terminal repeat (ITR) and the gene encoding the fusion protein. In the above (1) to (4), as needed, "the light chain" and "the variable region of the light chain" are interchangeable, and "the heavy chain" and "the variable region of the light chain" are interchangeable.

In one embodiment of the present invention, the fusion protein contains human iduronate-2-sulfatase (hI2S) bound via a linker to the downstream of the Fab region of the heavy chain of the anti-TfR antibody, and the light chain of the anti-TfR antibody. A specific example of the fusion protein includes one in which the Fab region of the heavy chain of the anti-TfR antibody contains an amino acid sequence represented by SEQ ID NO: 13, the linker contains the amino acid sequence represented by SEQ ID NO: 4, the hI2S contains an amino acid sequence represented by SEQ ID NO: 14, and the light chain of the anti-TfR antibodies contains an amino acid sequence represented by SEQ ID NO: 15. Here, the conjugate contains an amino acid sequence represented by SEQ ID NO: 51 as a whole.

A nucleic acid molecule in one embodiment of the present invention contains a gene encoding a fusion protein of an anti-TfR antibody and a physiologically active protein between a first long terminal repeat (LTR) and a second long terminal repeat (LTR). The following (1) to (4) are examples of the nucleic acid molecule:
(1) a nucleic acid molecule wherein a fusion protein contains a conjugate in which a physiologically active protein is bound to the C-terminus or the N-terminus of a heavy chain of an anti-TfR antibody and a light chain of the anti-TfR antibody, and the nucleic acid molecule contains a nucleotide sequence encoding an internal ribosome binding site downstream of a gene encoding the light chain, and downstream thereof a nucleotide sequence encoding the conjugate;
(2) a nucleic acid molecule wherein a fusion protein contains a conjugate in which a physiologically active protein is bound to the C-terminus or the N-terminus of a heavy chain of an anti-TfR antibody and a light chain of the anti-TfR antibody, and the nucleic acid molecule contains a nucleotide sequence encoding an internal ribosome binding site downstream of a gene encoding the conjugate, and downstream thereof a nucleotide sequence encoding the light chain;
(3) a nucleic acid molecule wherein a fusion protein contains a conjugate in which a physiologically active protein is bound to the C-terminus or the N-terminus of a light chain of an anti-TfR antibody and a heavy chain of the anti-TfR antibody, and the nucleic acid molecule contains a nucleotide sequence encoding an internal ribosome binding site downstream of a gene encoding the heavy chain, and downstream thereof a nucleotide sequence encoding the conjugate;
(4) a nucleic acid molecule wherein a fusion protein contains a conjugate in which a physiologically active protein is bound to the C-terminus or the N-terminus of a light chain of an anti-TfR antibody and a heavy chain of the anti-TfR antibody, and the nucleic acid molecule contains a nucleotide sequence encoding an internal ribosome binding site downstream of a gene encoding the conjugate, and downstream thereof a nucleotide sequence encoding the heavy chain.

In (1) to (4) above, the nucleic acid molecule may contain a nucleotide sequence containing a gene expression regulatory site between the first inverted terminal repeat (LTR) and the gene encoding the fusion protein. Also, in (1) to (4) above, the nucleotide sequence encoding the internal ribosome binding site may be replaced with a nucleotide sequence containing a gene expression regulatory site. When the nucleic acid molecule has two gene expression regulatory sites, the gene expression regulatory sites are referred to as a first gene expression regulatory site and a first gene expression control site in the order from the first inverted terminal repeat (LTR) side for convenience. In (1) to (4) above, the nucleotide sequence encoding the internal ribosome binding site may be replaced with a nucleotide sequence encoding a 2A self-cleaving peptide. A suitable example of the 2A self-cleaving peptide is a 2A peptide derived from porcine teschovirus. In the above (1) to (4), as needed, "the light chain" and "the variable region of the light chain" are interchangeable, and "the heavy chain" and "the variable region of the light chain" are interchangeable.

When the anti-TfR antibody is a single chain antibody, a nucleic acid molecule in one embodiment of the present invention contains a gene encoding a fusion protein of a single chain anti-TfR antibody and a physiologically active protein between a first long terminal repeat (LTR) and a second long terminal repeat (LTR). The following (1) to (4) are examples of the nucleic acid molecule:
(1) a nucleic acid molecule containing a gene encoding a fusion protein in which a light chain is bound to the C-terminus of a heavy chain of an anti-TfR antibody via a second linker and further a physiologically active protein is bound to the C-terminus of the light chain directly or via a linker;
(2) a nucleic acid molecule containing a gene encoding a fusion protein in which a heavy chain is bound to the C-terminus of a light chain of an anti-TfR antibody via a second linker and further a physiologically active protein is bound to the C-terminus of the light chain directly or via a linker;
(3) a nucleic acid molecule containing a gene encoding a fusion protein in which a heavy chain of an anti-TfR antibody is bound to the C-terminus of a physiologically active protein directly or via a linker and further a light chain is bound to the C-terminus of the heavy chain;
(4) a nucleic acid molecule containing a gene encoding a fusion protein in which a light chain of an anti-TfR antibody is bound to the C-terminus of a physiologically active protein directly or via a linker and further a heavy chain is bound to the C-terminus of a heavy chain.

In (1) to (4) above, the nucleic acid molecule may contain a nucleotide sequence containing a gene expression regulatory site between the first long terminal repeat (LTR) and the gene encoding the fusion protein. In the above (1) to (4), as needed, "the light chain" and "the variable region of the light chain" are interchangeable, and "the heavy chain" and "the variable region of the light chain" are interchangeable.

In the nucleic acid molecule containing the nucleotide sequence encoding the internal ribosome binding site, the expression of one peptide chain contained in the fusion protein is regulated from the gene expression regulatory site, and the expression of the other peptide chain is regulated from the nucleotide sequence encoding the internal ribosome binding site. Both peptide chains are paired in a cell to form a fusion protein of the anti-TfR antibody and the physiologically active protein.

In one embodiment of the present invention, the affinity (binding activity) for TfR of the fusion protein of the anti-TfR antibody and the physiologically active protein can be adjusted by appropriately selecting the amino acid sequence of the anti-TfR antibody portion contained in the fusion protein and/or by appropriately selecting the binding form between the anti-TfR antibody and the physiologically active protein. Here, the expression "appropriately selecting the binding form between the anti-TfR antibody and the physiologically active protein" means, for example, (1) when the original fusion protein is one in which the physiologically active protein binds to the heavy chain of the anti-TfR antibody, binding the physiologically active protein to the light chain instead of the heavy chain of the anti-TfR antibody; (2) when the original fusion protein is one in which the physiologically active protein binds to the light chain of the anti-TfR antibody, binding the physiologically active protein to the heavy chain instead of the light chain of the anti-TfR antibody; (3) when the physiologically active protein binds to the N-terminus of the heavy chain or the light chain of the anti-TfR antibody, binding the physiologically active protein to the C-terminus of the heavy chain or the light chain of the anti-TfR antibody; (4) when the physiologically active protein binds to the C-terminus of the heavy chain or light chain of the anti-TfR antibody, binding the physiologically active protein to the N-terminus of the heavy chain or light chain of the anti-TfR antibody; (5) when the physiologically active protein binds to the heavy chain or light chain of the anti-transferrin receptor antibody via a linker, changing the amino acid sequence of the linker (including changing the length of the linker) or deleting the linker. The bonding form can also be changed by combining these (1) to (5). Due to these changes in binding form, for example, steric hindrance is generated, the binding between the anti-TfR antibody portion of the fusion protein and TfR is inhibited, and, as a result, the affinity (binding activity) for TfR of the fusion protein as a whole is reduced.

In the case of a fusion protein whose affinity (binding activity) for TfR as a whole is adjusted and which exhibits a certain value, the effect of impairing the function of reticulocytes caused by binding of the fusion protein to reticulocytes expressing a large amount of TfR on the cell surface is suppressed. When the function of reticulocytes is impaired, the number of erythrocytes in the blood decreases and an anemia symptom occurs. In the case of a fusion protein exhibiting a certain value of affinity for TfR, the anemia symptom that occurs when the fusion protein is administered in vivo is suppressed. Severity of the anemia symptom can be objectively judged by comparing the concentration of hemoglobin in the blood with that in a normal individual. The concentration of hemoglobin and the severity of the anemia symptom have an inverse correlation. The lower the concentration of hemoglobin, the more severe the anemia symptom.

In addition, when the affinity (binding activity) for TfR of the fusion protein of the anti-TfR antibody and the physiologically active protein as a whole is extremely high, the binding of endogenous transferrin to TfR is strongly inhibited, the transport of the transferrin into a cell via TfR is inhibited, and the cell may become iron deficient. The fusion protein of one embodiment of the present invention can reduce the risk of iron deficiency of a cell when the anti-TfR antibody is administered in vivo.

In administration of the fusion protein of the anti-TfR antibody and the physiologically active protein as a drug by subcutaneous, intramuscular, intravenous or any other injection, if an anemia symptom is observed as a side reaction, administration only has to be discontinued. However, when gene therapy is performed using a gene encoding a fusion protein, the fusion protein will be continuously released from the administered gene into the living body for a long period of time. Thus, even if the side reaction occurs, the expression of the fusion protein cannot be interrupted. In one embodiment of the present invention, by appropriately adjusting the affinity (binding activity) for TfR, the occurrence of side reactions expected to occur due to the continuous expression of the fusion protein in the body of a patient is suppressed.

Especially, when the gene encoding the fusion protein of the anti-TfR antibody and the physiologically active protein is used in gene therapy, the affinity (binding activity) for TfR of the fusion protein as a whole becomes an issue. Therefore, a gene encoding a fusion protein having an adjusted affinity (binding activity) as a whole for TfR is used, upon appropriate adjustment of the amino acid sequence and/or the binding form between TfR and the physiologically active protein, for example, by mutating the anti-TfR antibody portion contained in the fusion protein. The use of such a gene makes it possible to develop a safe therapeutic agent for gene therapy, in which an anemia symptom caused by the expression of the fusion protein in vivo is suppressed, and the fusion protein can be transferred into cells via TfR on the cells and exert its physiological activity. Thus, a gene encoding such a fusion protein can be used as a part of a safe gene therapy medicament. In particular, if the fusion protein is to pass through the blood-brain barrier (BBB) by binding to TfR present on the surfaces of cerebrovascular endothelial cells, the gene encoding the fusion protein can be used as a part of a highly safe medicament for gene therapy for disorders in the brain and central nervous system.

When the gene encoding the fusion protein is used as a part of a medicament for gene therapy, the binding activity to TfR of the anti-TfR antibody or the fusion protein of the anti-TfR antibody and the physiologically active protein can be evaluated by, for example, 50% effective concentration (EC₅₀) based on measurement using an ELISA method. The 50% effective concentration can be measured, for example, by the method which will be described in Example 15. In one embodiment of the present invention, for the binding activity between TfR and the fusion protein of the anti-TfR antibody and the physiologically active protein, the 50% effective concentration is preferably from 3 nM to 10 µM, preferably from 3 nM to 5 µM, and, for example, from 3 nM to 2 µM, from 3 nM to 1 µM, from 3 nM to 500 nM, from 3 nM to 200 nM, from 3 nM to 100 nM, from 3.5 nM to 2 µM, from 3.5 nM to 1 µM, from 3.5 nM to 500 nM, from 3.5 nM to 200 nM, from 3.5 nM to 100 n, from 4 nM to 2 µM, from 4 nM to 1 µM, from 4 nM to 500 nM, from 4 nM to 200 nM, from 4 nM to 100 nM, from 5 nM to 5 µM, from 5 nM to 1 µM, from 5 nM to 500 nM, from 5 nM to 200 nM, from 5 nM to 100 nM, from 10 nM to 5 µM, from 10 nM to 1 µM, from 5 nM to 500 nM, from 5 nM to 200 nM 5 nM to 100 nM, or from 10 to 100 nM, when measured by the measurement method. When the anti-TfR antibody is an anti-human TfR, the 50% effective concentration (EC₅₀) may be determined by using, for example, a human transferrin receptor extracellular domain having an amino acid sequence from position 87 to position 763 of the amino acid sequence represented by SEQ ID NO: 12 instead of the mouse transferrin receptor extracellular domain in the method described in Example 15.

The nucleic acid molecule according to one embodiment of the present invention can be used as a part of a therapeutic agent for gene therapy to which AAV is applied. When wild-type AAV alone infects a human cell as a host cell, the viral genome is site-specifically integrated into chromosome 19 via the inverted terminal repeats (ITRs) at both ends of the viral genome. The gene of the viral genome taken up into the genome of the host cell is hardly expressed. However, when the cell is infected with a helper virus, AAV is excised from the host genome and replication of the infectious virus is initiated. When the helper virus is an adenovirus, the genes responsible for helper action are E1A, E1B, E2A, VA1, and E4. Here, when the host cell is a HEK293 cell which is a human embryonic kidney tissue-derived cell transformed with adenoviral E1A and E1B, E1A and E1B genes are originally expressed in the host cell. In one embodiment of the present invention, the AAV vector refers to a vector used for preparing rAAV virions, which has a nucleotide sequence containing a first inverted terminal repeat (ITR) and a nucleotide sequence containing a second inverted terminal repeat (ITR) derived from an AAV genome and into which a foreign gene can be integrated. The place where a foreign gene can be integrated in the AAV vector can be located between the first and second inverted terminal repeats (ITRs). An AAV vector into which a foreign gene is integrated is also the AAV vector.

The wild-type AAV genome contains two genes, rep and cap. The rep proteins (rep78, rep68, rep52 and rep40) produced from the rep gene are essential for capsid formation and mediate integration of the viral genome into the chromosome. The cap gene is responsible for the production of three capsid proteins (VP1, VP2 and VP3).

In the genome of wild-type AAV, ITRs are present at both ends, and the rep gene and the cap gene are present between the ITRs. This genome is encapsulated in a capsid to form an AAV virion. In one embodiment, a recombinant AAV virion (rAAV virion) has a genome encapsulated in a capsid encoded by the cap gene, in which a region including the rep gene and the cap gene of the wild-type AAV genome is replaced with a gene encoding a foreign protein. However, the recombinant AAV virion is not limited to this, and the recombinant AAV virion (rAAV virion) also encompasses one having a genome encapsulated in a capsid, in which a part of the wild-type AAV genome is replaced with a gene encoding a foreign protein.

The rAAV virion can be used for therapeutic purposes as the vector for delivering a foreign gene to cells. In a specific aspect of the present invention, there is provided a pharmaceutical composition containing an rAAV virion and a pharmaceutically acceptable carrier.

For the production of a recombinant AAV virion used for introducing a foreign gene into a cell, a tissue or a living body, three types of plasmids are usually used: (1) a plasmid (plasmid 1) having a nucleotide sequence containing a first inverted terminal repeat (ITR) and a nucleotide sequence containing a second inverted terminal repeat (ITR) derived from a virus such as AAV, and a structure containing a gene encoding a desired protein located between the two ITRs; (2) a plasmid (plasmid 2) containing an AAV Rep gene having a function necessary for integrating a nucleotide sequence of a region (including an ITR sequence) flanked by the ITR sequences into a genome of a host cell and a gene encoding a capsid protein of AAV; and (3) a plasmid (plasmid 3) containing an E2A region, an E4 region, and a VA1 RNA region of adenovirus. However, the plasmid is not limited to these plasmids, and two types of plasmids, i.e., one plasmid in which two of the plasmids 1 to 3 are linked to each other and the remaining one plasmid, can also be used. Furthermore, one plasmid in which the plasmids 1 to 3 are linked can also be used. The desired protein in the present invention is a fusion protein of a ligand and a physiologically active protein.

Generally, for the production of the recombinant AAV virion, these three types of plasmids are first introduced, by a general transfection technique, into a host cell such as a HEK293 cell in which adenoviral E1a and E1b genes are integrated into the genome. Then, the region containing the nucleotide sequence containing the first inverted terminal repeat (ITR), the nucleotide sequence containing the second inverted terminal repeat (ITR), and the gene encoding the desired protein located between these two ITRs is replicated in the host cell, and the resulting single-stranded DNA is packaged into the capsid protein of AAV to form a recombinant AAV virion. The recombinant AAV virion is infectious, and thus can be used to introduce a foreign gene into a cell, a tissue, or a living body. In one embodiment of the present invention, the foreign gene is the gene encoding the fusion protein of the anti-TfR antibody and the physiologically active protein. The fusion protein is expressed from this gene in the cell or the like to which the gene is introduced.

The Rep protein of adeno-associated virus (AAV) is encoded by the rep gene of AAV The Rep protein has a function necessary for integrating, for example, an AAV genome into the genome of a host cell via an ITR present in the genome. Although there are multiple subtypes of Rep proteins, two types, Rep68 and Rep78, are required for integration of the AAV genome into the genome of the host cell. Rep68 and Rep78 are translation products of two types of mRNAs transcribed by alternative splicing from the same genes. In the present invention, the "Rep protein of AAV" includes at least two types of proteins, Rep68 and Rep78.

In one embodiment of the present invention, the nucleotide sequence encoding the Rep protein of adeno-associated virus (the nucleotide sequence of the Rep region) refers to a nucleotide sequence encoding at least Rep68 and Rep78, or a mutated nucleotide sequence thereof. The Rep protein is preferably that of serotype 2 AAV, but is not limited thereto and may be that of serotype 1, 3, 4, 5, 6, 7, 8, 9, 10 or 11. A preferred example of the nucleotide sequence encoding the Rep protein of wild-type serotype 2 AAV is a nucleotide sequence represented by SEQ ID NO: 32.

Rep68 may be a mutant in which the amino acid sequence of the wild-type Rep68 of AAV of serotype 1, 2, 3, 4, 5, 6, 7, 8, 9, 10 or 11 has been modified by substitution, deletion, addition, or the like, as long as it exhibits its function. In the present invention, Rep68 also encompasses such mutated Rep68.

When an amino acid in the amino acid sequence of the wild-type Rep68 is substituted with another amino acid, the number of amino acids to be substituted is preferably from 1 to 10, more preferably from 1 to 5, and even more preferably from 1 to 3. When an amino acid in the amino acid sequence of the wild-type Rep68 is deleted, the number of amino acids to be deleted is preferably from 1 to 10, more preferably from 1 to 5, and even more preferably from 1 to 3. In addition, Rep68 also encompasses Rep68 to which a mutation combining such amino acid substitution and deletion has been added. When an amino acid is added, preferably from 1 to 10, more preferably from 1 to 5, and even more preferably from 1 to 3 amino acids are added into the amino acid sequence or to the N-terminus or the C-terminus of the wild-type Rep68. In addition, Rep68 also encompasses Rep68 to which a mutation combining such amino acid addition, substitution and deletion has been added. The amino acid sequence of the mutated Rep68 preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the amino acid sequence of the wild-type Rep68.

Rep78 may be a mutant in which the amino acid sequence of the wild-type Rep78 of AAV of serotype 1, 2, 3, 4, 5, 6, 7, 8, 9, 10 or 11 has been modified by substitution, deletion, addition, or the like, as long as it exhibits its function. In the present invention, Rep78 also encompasses such mutated Rep78.

When an amino acid in the amino acid sequence of the wild-type Rep78 is substituted with another amino acid, the number of amino acids to be substituted is preferably from 1 to 10, more preferably from 1 to 5, and even more preferably from 1 to 3. When an amino acid in the amino acid sequence of the wild-type Rep78 is deleted, the number of amino acids to be deleted is preferably from 1 to 10, more preferably from 1 to 5, and even more preferably from 1 to 3. In addition, Rep78 also encompasses Rep78 to which a mutation combining such amino acid substitution and deletion has been added. When an amino acid is added, preferably from 1 to 10, more preferably from 1 to 5, and even more preferably from 1 to 3 amino acids are added into the amino acid sequence or to the N-terminus or the C-terminus of the wild-type Rep78. In addition, Rep78 to which a mutation combining such amino acid addition, substitution and deletion has been added is also included in Rep78. The amino acid sequence of the mutated Rep78 preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the amino acid sequence of the wild-type Rep78.

A functional equivalent of the Rep protein of AAV refers to an equivalent that can be used in place of Rep68 from the functional viewpoint for Rep68, and refers to an equivalent that can be used in place of Rep78 from the functional viewpoint for Rep78. The functional equivalent of the Rep protein may be a mutant of the wild-type Rep protein.

The nucleotide sequence represented by SEQ ID NO: 32 may be modified by substitution, deletion, addition or the like as long as it encodes functional Rep68 and Rep78. When a nucleotide in the nucleotide sequence represented by SEQ ID NO: 32 is substituted with another nucleotide, the number of nucleotides to be substituted is preferably from 1 to 20, more preferably from 1 to 10, and even more preferably from 1 to 3. When a nucleotide in the nucleotide sequence represented by SEQ ID NO: 32 is deleted, the number of nucleotides to be deleted is preferably from 1 to 20, more preferably from 1 to 10, and even more preferably from 1 to 3. In addition, a sequence to which a mutation combining such nucleotide substitution and deletion is added can also be used as a nucleotide sequence encoding Rep protein. When a nucleotide is added, preferably from 1 to 20, more preferably from 1 to 10, and even more preferably from 1 to 3 nucleotides are added into the nucleotide sequence represented by SEQ ID NO: 32 or to the 5'-terminus or the 3'-terminus. A sequence to which a mutation combining such nucleotide addition, substitution and deletion is added can also be used as a nucleic acid molecule encoding Rep protein. The mutated nucleotide sequence preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 32. However, when these mutations are added, the start codons of the genes encoding the Rep proteins, Rep68 and Rep78, are preferably ACG.

In one embodiment of the present invention, the term "nucleotide sequence encoding Cap protein of adeno-associated virus (nucleotide sequence of Cap region)" refers to a nucleotide sequence containing at least a nucleotide sequence encoding VP1, which is one of the proteins contained in the capsid of AAV, or a mutated nucleotide sequence thereof. The VP1 is preferably that of serotype 8 AAV, but is not limited thereto and may be that of serotype 1, 2, 3, 4, 5, 6, 7, 8, 9, 10 or 11. A suitable example of the nucleotide sequence of the Cap region of serotype 8 includes a nucleotide sequence represented by SEQ ID NO: 31.

VP1 may be one in which the amino acid sequence of the wild-type VP1 of AAV of serotype 1, 2, 3, 4, 5, 6, 7, 8, 9, 10 or 11 is modified by substitution, deletion, addition, or the like, as long as it exhibits its function. In one embodiment of the present invention, VP1 also encompasses such mutated VP1.

When a nucleotide in the nucleotide sequence represented by SEQ ID NO: 31 is substituted with another nucleotide, the number of nucleotides to be substituted is preferably from 1 to 20, more preferably from 1 to 10, and even more preferably from 1 to 3. When a nucleotide in the nucleotide sequence represented by SEQ ID NO: 31 is deleted, the number of nucleotides to be deleted is preferably from 1 to 20, more preferably from 1 to 10, and even more preferably from 1 to 3. In addition, a sequence to which a mutation combining such nucleotide substitution and deletion is added can also be used as a nucleotide sequence encoding Cap protein. When a nucleotide is added, preferably from 1 to 20, more preferably from 1 to 10, and even more preferably from 1 to 3 nucleotides are added into the nucleotide sequence represented by SEQ ID NO: 31 or to the 5'-terminus or the 3'-terminus. A sequence to which a mutation combining such nucleotide addition, substitution and deletion is added can also be used as a nucleic acid molecule encoding Cap protein. The mutated nucleotide sequence preferably exhibits an identity of 80% or more, more preferably exhibits an identity of 85% or more, even more preferably exhibits an identity of 90% or more, still more preferably exhibits an identity of 95% or more, and yet more preferably exhibits an identity of 98% or more, with the nucleotide sequence represented by SEQ ID NO: 31.

In one embodiment of the present invention, an AAV vector can be used to form a recombinant AAV virion in which a nucleic acid molecule containing a foreign gene between a first ITR and a second ITR is packaged. The recombinant AAV virion is infectious, and thus can be used to introduce a foreign gene into a cell, a tissue, or a living body. In the present invention, the foreign gene is the gene encoding the fusion protein of the anti-TfR antibody and the physiologically active protein. The fusion protein is expressed from this gene in the cell or the like to which the gene is introduced. An example of the recombinant virion in which a nucleic acid molecule containing a foreign gene between the first ITR and the second ITR is packaged, and which can be used for introducing a gene into a cell or the like includes a recombinant virion in which AAV is applied (AAV vector system) and a recombinant virion in which adenovirus is applied (adenoviral vector system). In the adenoviral vector system, a recombinant adenoviral virion is formed in which a nucleic acid molecule containing a foreign gene between the first ITR and the second ITR is packaged. The recombinant adenoviral virion is infectious, and thus can be used to introduce a foreign gene into a cell, a tissue, or a living body.

A preferred aspect of the binding activity between TfR and a fusion protein of an anti-TfR antibody and a physiologically active protein, when a gene encoding the fusion protein is used as a part of a medicament for gene therapy to which AAV virus is applied, will be described in detail below. Note that these aspects can also be applied to those to which adenovirus is applied, those to which lentivirus is applied, and those to which retrovirus is applied. That is, for the binding activity between the fusion protein and TfR is determined by, for example, the 50% effective concentration (EC₅₀) value is preferably from 0.5 nM to 10 µM, more preferably from 3 nM to 5 µM, and, for example, from 0.5 nM to 5 µM, from 0.5 nM to 1 µM, from 1 nM to 2 µM, from 1 nM to 1 µM, from 3 nM to 2 µM, from 3 nM to 1 µM, from 3 to 500 nM, from 3 to 200 nM, from 3 to 100 nM, from 3.5 nM to 2 µM, from 3.5 nM to 1 µM, from 3.5 to 500 nM, from 3.5 to 200 nM, from 3.5 to 100 n, from 4 nM to 2 µM, from 4 nM to 1 µM, from 4 to 500 nM, from 4 to 200 nM, from 4 to 100 nM, from 5 nM to 5 µM, from 5 nM to 1 µM, from 5 to 500 nM, from 5 to 200 nM, from 5 to 100 nM, from 10 nM to 5 µM, from 10 nM to 1 µM, from 5 to 500 nM, from 5 to 200 nM, from 5 to 100 nM, or from 10 to 100 nM, as measured by the ELISA method described in Example 15. These EC₅₀ values are particularly preferred when the fusion protein is a fusion protein of an anti-TfR antibody and a lysosomal enzyme, e.g., a fusion protein of a TfR antibody and human I2S or a fusion protein of a TfR antibody and human GAA. When the anti-TfR antibody is an anti-human TfR, the 50% effective concentration (EC₅₀) may be determined by using, for example, a human transferrin receptor extracellular domain having an amino acid sequence from position 87 to position 763 of the amino acid sequence represented by SEQ ID NO: 12 instead of the mouse transferrin receptor extracellular domain in the method described in Example 15. An rAAV virion in which a DNA fragment containing the gene encoding the fusion protein is packaged is one embodiment of the medicament for gene therapy to which AAV virus is applied.

A dosage of the medicament should be appropriately adjusted in accordance with symptoms of the patient and the like. The dosage of the rAAV virion in which the gene encoding the fusion protein of the anti-TfR antibody and the physiologically active protein is packaged, the fusion protein having the EC₅₀ value described in the previous paragraph, is preferably from 1.0 × 10¹⁰ to 1.0 × 10¹⁴ vg/kg, and for example, from 1.0 × 10¹⁰ to 5.0 × 10¹³ vg/kg, from 1.0 × 10¹⁰ to 2.0 × 10¹³ vg/kg, from 1.0 × 10¹⁰ to 1.0 × 10¹³ vg/kg, from 1.0 × 10¹¹ to 1.0 × 10¹³ vg/kg, 1.0 × 10¹¹ vg/kg, 1.0 × 10¹² vg/kg, 2.0 × 10¹² vg/kg, 5.0 × 10¹² vg/kg, 6.0 × 10¹² vg/kg, or 1.0 × 10¹³ vg/kg. That is, the dosage can be safely increased or decreased over a wide range without causing anemia symptoms. In addition, the administration interval of the medicament is not particularly limited, and, for example, when the concentration of the fusion protein in the blood becomes a predetermined concentration or less, the medicament can be administered again. Here, the expression that "the concentration becomes a predetermined concentration or less" means that the concentration of the fusion protein becomes 5% or less, 10% or less, 20% or less, or 50% or less as compared with the blood concentration as measured on any day from one week to 10 years after administration, for example, from 3 weeks to 6 weeks after administration. As long as the concentration of the fusion protein in the blood does not decrease, the number of administrations can be limited to one.

For the binding activity to TfR of a fusion protein of an anti-TfR antibody and a physiologically active protein when a gene encoding the fusion protein is used as a part of the medicament for gene therapy to which AAV virus is applied, the 50% effective concentration (EC₅₀) value, as measured by the ELISA method described in Example 15, can also be defined as less than 3 nM, for example, from 0.5 to 3 nM, from 0.8 to 3 nM, or from 1 to 3 nM, by limiting the dosage per administration, in addition to those applied to all the fusion proteins of the anti-TfR antibody and the physiologically active protein. The dosage per administration in this case should not exceed 1.0 × 10¹³ vg/kg, and is adjusted to preferably 7.5 × 10¹² vg/kg or less, more preferably 6.0 × 10¹² vg/kg or less, even more preferably 5.0 × 10¹² vg/kg or less, 3.0 × 10¹² vg/kg or less, 2.5 × 10¹² vg/kg or less, or the like. For example, the dosage per administration is adjusted to from 1.0 × 10¹⁰ to 7.5 × 10¹² vg/kg, from 1.0 × 10¹¹ to 7.5 × 10¹² vg/kg, from 5.0 × 10¹¹ to 7.5 × 10¹² vg/kg, from 1.0 × 10¹⁰ to 6.0 × 10¹² vg/kg, from 1.0 × 10¹¹ to 6.0 × 10¹² vg/kg, from 5.0 × 10¹¹ to 6.0 × 10¹² vg/kg, from 1.0 × 10¹⁰ to 5.0 × 10¹² vg/kg, from 1.0 × 10¹¹ to 5.0 × 10¹² vg/kg, from 5.0 × 10¹¹ to 5.0 × 10¹² vg/kg, from 1.0 × 10¹⁰ to 3.0 × 10¹² vg/kg, from 1.0 × 10¹¹ to 3.0 × 10¹² vg/kg, from 5.0 × 10¹¹ to 3.0 × 10¹² vg/kg, from 1.0 × 10¹⁰ to 2.5 × 10¹² vg/kg, from 1.0 × 10¹¹ to 2.5 × 10¹² vg/kg, from 5.0 × 10¹¹ to 2.5 × 10¹² vg/kg, from 1.0 × 10¹⁰ to 2.0 × 10¹² vg/kg, from 1.0 × 10¹¹ to 2.0 × 10¹² vg/kg, or from 5.0 × 10¹¹ to 2.0 × 10¹² vg/kg. However, since administration of a fusion protein having an EC₅₀ value of less than 3 nM may cause anemia symptoms, it is necessary to carefully observe the physical condition of the patient after administration. From this point of view, a fusion protein having an EC₅₀ value of 3 nM or more can be said to be excellent as the medicament for gene therapy. Nevertheless, administration of a fusion protein having an EC₅₀ value of less than 3 nM can be said to be clinically valuable because it may bring about an efficacy.

For the binding activity to TfR of a fusion protein of an anti-TfR antibody and a physiologically active protein when a gene encoding the fusion protein is used as a part of the medicament for gene therapy to which AAV virus is applied and the physiologically active protein is human I2S, the EC₅₀ value, as measured by the ELISA method described in Example 15 is less than 3 nM, for example, from 0.5 to 3 nM, from 0.8 to 3 nM, or from 1 to 3 nM (from 0.5 to 4 nM, from 0.8 to 4 nM, or from 1 to 4 nM), in addition to the above-described ones. However, the dosage per administration in this case should not exceed 1.0 × 10¹¹ vg/kg, and is adjusted to preferably 7.5 × 10¹² vg/kg or less, more preferably 6.0 × 10¹² vg/kg or less, even more preferably 5.0 × 10¹² vg/kg or less, still more preferably 3.0 × 10¹² vg/kg or less, 2.5 × 10¹² vg/kg or less, or the like. For example, the dosage per administration is adjusted to from 1.0 × 10¹⁰ to 7.5 × 10¹² vg/kg, from 1.0 × 10¹¹ to 7.5 × 10¹² vg/kg, from 5.0 × 10¹¹ to 7.5 × 10¹² vg/kg, from 1.0 × 10¹⁰ to 6.0 × 10¹² vg/kg, from 1.0 × 10¹¹ to 6.0 × 10¹² vg/kg, from 5.0 × 10¹¹ to 6.0 × 10¹² vg/kg, from 1.0 × 10¹⁰ to 5.0 × 10¹² vg/kg, from 1.0 × 10¹¹ to 5.0 × 10¹² vg/kg, from 5.0 × 10¹¹ to 5.0 × 10¹² vg/kg, from 1.0 × 10¹⁰ to 3.0 × 10¹² vg/kg, from 1.0 × 10¹¹ to 3.0 × 10¹² vg/kg, from 5.0 × 10¹¹ to 3.0 × 10¹² vg/kg, from 1.0 × 10¹⁰ to 2.5 × 10¹² vg/kg, from 1.0 × 10¹¹ to 2.5 × 10¹² vg/kg, from 5.0 × 10¹¹ to 2.5 × 10¹² vg/kg, from 1.0 × 10¹⁰ to 2.0 × 10¹² vg/kg, from 1.0 × 10¹¹ to 2.0 × 10¹² vg/kg, or from 5.0 × 10¹¹ to 2.0 × 10¹² vg/kg.

That is, in the case of using a gene encoding a fusion protein of an anti-TfR antibody and human I2S, a fusion protein having an EC₅₀ value, with respect to TfR, of 0.5 nM or more, 0.8 nM or more, or 1 nM or more can also be used under the condition that the dosage per administration is adjusted to preferably from 7.5 × 10¹² vg/kg or less, more preferably from 6.0 × 10¹² vg/kg or less, even more preferably from 5.0 × 10¹² vg/kg or less, still more preferably from 3.0 × 10¹² vg/kg or less, from 2.5 × 10¹² vg/kg or less, or the like. For example, the EC₅₀ value is preferably from 0.5 nM to 1 µM, more preferably from 0.5 to 100 nM, even more preferably from 0.5 to 10 nM, still more preferably from 0.5 to 5 nM, and for example, from 0.5 to 4 nM, from 0.5 to 3 nM, from 0.8 nM to 1 µM, from 0.8 to 500 nM, from 0.8 to 100 nM, from 0.8 to 10 nM, from 0.8 to 5 nM, from 0.8 to 4 nM, from 0.8 to 3 nM, from 1.0 nM to 1 µM, from 1.0 to 500 nM, from 1.0 to 100 nM, from 1.0 to 10 nM, from 1.0 to 5 nM, from 1.0 to 4 nM, from 1.0 to 3 nM, from 1 nM to 5 µM, from 1 nM to 1 µM, from 1 to 500 nM, from 1 to 200 nM, from 1 to 100 nM, from 1 nM to 5 µM, from 1 nM to 1 µM, from 1 to 500 nM, from 1 to 200 nM, or from 1 to 100 nM.

However, when the gene encoding the fusion protein of the anti-TfR antibody and human I2S is used, administration of a fusion protein having an EC₅₀ value, with respect to TfR, of less than 3 nM (less than 4 nM) has a disadvantage of limitation on setting of the dosage because there is a possibility of causing anemia symptoms. In addition, it is necessary to carefully observe the physical condition of the patient after administration. From this point of view, a fusion protein having an EC₅₀ value of 3 nM or more (or 4 nM or more) can be said to be excellent as the medicament for gene therapy. Nevertheless, administration of a fusion protein having an EC₅₀ value of less than 3 nM (less than 4 nM) can be said to be clinically valuable because it may bring about an efficacy.

In the case of using the gene encoding the fusion protein of the anti-TfR antibody and human I2S, the administration interval is not particularly limited. For example, when the concentration of the fusion protein in the blood becomes a predetermined concentration or less, administration can be performed again. Here, the expression that "the concentration becomes a predetermined concentration or less" means that the concentration of the fusion protein becomes 5% or less, 10% or less, 20% or less, or 50% or less as compared with the blood concentration as measured on any day from one week to 10 years after administration, for example, from 3 weeks to 6 weeks after administration. As long as the concentration of the fusion protein in the blood does not decrease, the number of administrations can be limited to one.

The above-described dosage when the gene encoding the fusion protein of the anti-TfR antibodies and human I2S is used can also be applied to a fusion protein of the anti-TfR antibody and another physiologically active substance, for example, a fusion protein of the anti-TfR antibody and another lysosomal enzyme.

Lentiviruses are viruses belonging to the genus Lentivirus of the subfamily Orthoretrovirinae, which is a subfamily of the family Retroviridae, and have a single-stranded (+) strand RNA genome (ssRNA). The genome of lentivirus contains, as essential genes, gag (a region encoding structural proteins including capsid protein), pol (a region encoding a group of enzymes including reverse transcriptase), and env (a region encoding an envelope protein necessary for binding to a host cell), which are flanked by two LTRs (5' LTR and 3' LTR). In addition to these, the genome of lentivirus contains, as auxiliary genes, Rev (a region encoding a protein having a function of binding to RRE (rev responsive element) present in viral RNA to transport viral RNA from the nucleus to the cytoplasm), tat (a region encoding a protein having a function of binding to TAR present in 5' LTR to increase the promoter activity of LTR), vif, vpr, vpu, nef, and the like.

Lentivirus is an enveloped virus and infects a cell by fusion of the envelope with the cell membrane. Lentivirus is an RNA virus, and reverse transcriptase is present in the virion. After infection with lentivirus, single chain plus strand DNA is replicated from the (+) strand RNA genome by reverse transcriptase, and further double-stranded DNA is synthesized. A protein which is a component of the virion is expressed from the double-stranded DNA, and the (+) strand RNA genome is packaged in the protein, thereby propagating the virion. In one embodiment of the present invention, the lentiviral vector system may be developed based on the genome of HIV-1, which is a type of lentivirus, but is not limited thereto.

The first generation lentiviral vector system consists of three plasmids: a packaging plasmid, an Env plasmid, and a transfer plasmid. The packaging plasmid contains the gag and pol genes under the regulation of a CMV promoter or the like. The Env plasmid carries the env gene under the regulation of the CMV promoter. The transfer plasmid has a 5' LTR, an RRE, a gene encoding a desired protein under the regulation of the CMV promoter, and a 3' LTR. These are introduced into a host cell by a general transfection technique to form a recombinant virion in which a nucleic acid molecule containing a foreign gene between the first ITR and the second ITR is packaged in a capsid protein. The packaging plasmids for the first generation lentiviral vector systems also contain the virus-derived auxiliary genes rev, tat, vif, vpr, vpu, and nef. The desired protein in the present invention is a fusion protein of an anti-TfR antibody and a physiologically active protein. Note that a promoter that controls a gene encoding the desired protein is preferably a promoter other than the CMV promoter, for example, a promoter including, for example, SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, a mouse albumin promoter, a human albumin promoter, and a human α-1 antitrypsin promoter. For example, a synthetic promoter having a nucleotide sequence represented by SEQ ID NO: 9 containing a mouse albumin promoter downstream of a mouse α-fetoprotein enhancer (mouse α-fetoprotein enhancer/mouse albumin promoter) can also be used.

The second generation lentiviral vector system also consists of three plasmids: a packaging plasmid, an Env plasmid (envelope plasmid), and a transfer plasmid, similarly to the first generation lentiviral vector system. However, the auxiliary genes vif, vpr, vpu and nef, which are not essential genes, are deleted from the packaging plasmid.

The third generation lentiviral vector system consists of four plasmids: a packaging plasmid, an Env plasmid (envelope plasmid), a Rev plasmid, and a transfer plasmid. In the third generation, rev, which is present in the packaging plasmid in the second generation, is used independently as the Rev plasmid. In addition, tat is deleted from the packaging plasmid. In addition, the TAR in the 5' LTR of the transfer plasmid is replaced by the CMV promoter.

The recombinant lentiviral virion is infectious, and thus can be used to introduce a foreign gene into a cell, a tissue, or a living body. In the present invention, the foreign gene is the gene encoding the fusion protein of the anti-transferrin receptor antibody and the physiologically active protein. The fusion protein is expressed from this gene in the cell or the like to which the gene is introduced.

A retrovirus has a single chain (+) strand RNA genome (ssRNA). In the viral genome, genes of gag (encoding structural proteins including capsid protein), pol (encoding a group of enzymes including reverse transcriptase), env (encoding an envelope protein necessary for binding to a host cell), and packaging signal (Ψ) are encoded. These genes are flanked by two long terminal repeats (LTRs, 5' LTR and 3' LTR). When a retrovirus infects a host cell, (+) strand RNA and reverse transcriptase are transferred into the cell and reverse transcribed into double chain DNA.

The retroviral vector system has been developed mainly on the basis of murine leukemia virus. For the purpose of eliminating pathogenicity and enhancing safety, the viral genome is divided to eliminate self-replication ability while maintaining its infectivity. The first generation retroviral vector system consists of a packaging plasmid (viral genome from which the packaging signal is removed) and a transfer plasmid (packaging signal, a part of gag, and foreign gene flanked at both ends by 5' LTR and 3' LTR). Therefore, when homologous recombination occurs at the gag sequence portion commonly present in the packaging plasmid and the transfer plasmid, a retrovirus capable of self-replication, i.e., a replication competent (RC) virus appears. In the second generation retroviral vector system, the LTR on the 3' side of the first generation packaging plasmid is replaced with a poly A addition signal. As a result, in order to generate RC virus, homologous recombination should occur simultaneously at two sites upstream of the gag sequence and the poly A addition signal, and the probability of occurrence thereof is very low and safety is enhanced. The third generation is composed of three plasmids and the second generation packaging plasmid is further divided into a plasmid encoding gag/pol and a plasmid encoding env. Thus, in order to generate RC virus, homologous recombination should occur simultaneously at three sites, the probability of occurrence is extremely low, and the safety is further enhanced.

In general, for production of recombinant retroviral virions, these packaging plasmids and transfer plasmids are first introduced into a host cell by common transfection techniques. Then, the 5' LTR and 3' LTR, and the region containing the gene encoding the desired protein located between these two LTRs are replicated in the host cell, and the resulting single chain (+) strand RNA is packaged in the retroviral capsid protein to form a recombinant retroviral virion. The recombinant retroviral virion is infectious, and thus can be used to introduce a foreign gene into a cell, a tissue, or a living body. In the present invention, the foreign gene is the gene encoding the fusion protein of the anti-transferrin receptor antibody and the physiologically active protein. The fusion protein is expressed from this gene in the cell or the like to which the gene is introduced.

In one embodiment of the present invention, the nucleic acid molecule may be encapsulated in a liposome, a lipid nanoparticle (LNP), or the like. Liposomes are spherical vesicles with a lipid bilayer and are mainly composed of phospholipids, in particular phosphatidylcholine. However, the liposome is not limited to these, and other lipids such as egg yolk phosphatidylethanolamine may be added to the liposome as long as a lipid bilayer is formed. Since the cell membrane is mainly composed of a phospholipid bilayer, the liposome has an advantage of excellent biocompatibility. The lipid nanoparticle refers to a particle having a diameter of from 10 nm to 1000 nm, typically less than about 200 nm, and containing a lipid as a main component. The lipid nanoparticle can encapsulate a hydrophobic (lipophilic) molecule, and contains a biocompatible lipid such as a triglyceride, a diglyceride, a monoglyceride, a fatty acid, or a steroid as a main component. It is considered that, when a gene encapsulated in a liposome or a lipid nanoparticle is administered to a living body, the gene is taken up into the cell by direct fusion with the cell membrane of the cell, endocytosis, or the like, then transferred to the nucleus, and introduced into the cell. Gene transfer methods using liposomes or lipid nanoparticles are superior to gene transfer methods using viral vectors in that there is no limitation on the size of the gene to be transferred and in that safety is high. The liposome, lipid nanoparticle or the like encapsulating the nucleic acid molecule of the present invention can be used for introducing a gene of a fusion protein of a ligand and a physiologically active protein into a cell, a tissue or a living body. The fusion protein is expressed from this gene in the cell or the like to which the gene is introduced. As used herein, the term "liposome, lipid nanoparticle or the like" encompasses, in addition to the above-mentioned liposome and lipid nanoparticle, polymer nanoparticle, micelle, emulsion, nanoemulsion, microsphere, nanosphere, microcapsule, nanocapsule, dendrimer, nanogel, metal nanoparticle, and any other nano/micro particles that can be used as drug delivery systems (DDSs).

In the present invention, the behavior of a nucleic acid molecule introduced into a cell, a tissue, or a living body in the form of a plasmid, a form of being encapsulated in a recombinant viral virion, or a form of being encapsulated in a liposome, a lipid nanoparticle, or the like is exemplified in (1) to (6) below. However, the behavior of the nucleic acid molecule is not limited to these.
(1) The nucleic acid molecule is a single chain (+) strand RNA. When it is introduced into a cell, a gene encoding a fusion protein of an anti-transferrin receptor antibody and a physiologically active protein contained in the nucleic acid molecule is translated to express the fusion protein.
(2) The nucleic acid molecule is a single chain (+) strand RNA. When introduced into a cell, the nucleic acid molecule is reverse-transcribed to form a single chain (+) strand DNA, which is then transcribed and translated to express the fusion protein.
(3) The nucleic acid molecule is a single chain (+) strand RNA or (-) strand RNA. When introduced into a cell, the nucleic acid molecule is reverse-transcribed into a double chain DNA, which is then transcribed and translated to express the fusion protein.
(4) The nucleic acid molecule is a single chain (+) strand or (-) RNA. When introduced into a cell, the nucleic acid molecule is reverse-transcribed to form a double chain DNA. Then, the DNA undergoes random recombination or homologous recombination with the genome of the host cell and is integrated into the genome. The integrated DNA is transcribed and translated to express the fusion protein.
(5) The nucleic acid molecule is a single chain (+) strand DNA. When introduced into a cell, the nucleic acid molecule is transcribed and translated to express the fusion protein.
(6) The nucleic acid molecule is a double-stranded DNA. When introduced into a cell, the nucleic acid molecule is transcribed and translated to express the fusion protein.

The nucleic acid molecule can be introduced into a cell, a tissue, or a living body in the form of a plasmid, the form of being encapsulated in a recombinant viral virion, or the form of being encapsulated in a liposome, a lipid nanoparticle, or the like.

When the nucleic acid molecule is introduced into a living body, the nucleic acid molecule is administered in the form of a plasmid, the form of being encapsulated in a recombinant viral virion, or the form of being encapsulated in a liposome, a lipid nanoparticle or the like, together with a pharmaceutically acceptable excipient, that is, as a pharmaceutical composition, by parenteral means such as subcutaneous injection, intramuscular injection, intravenous injection or the like. Such a pharmaceutical composition is provided to medical institutions, for example, as an aqueous liquid filled in a vial or as a pre-filled syringe-type or cartridge-type aqueous liquid filled in a syringe in advance.

The nucleic acid molecule in the form of a plasmid, in the form of being encapsulated in a recombinant viral virion, or in the form of being encapsulated in a liposome, a lipid nanoparticle or the like can be used as various medicaments. The use of the nucleic acid molecule in which the fusion protein encoded by the nucleic acid molecule specifically recognizes the transferrin receptor and the physiologically active protein is a human lysosomal enzyme will be described in detail below.

The nucleic acid can be used as: a therapeutic agent for central nervous system disorder in Hurler's syndrome or Hurler-Scheie syndrome when the human lysosomal enzyme is α-L-iduronidase; a therapeutic agent for central nervous system disorder in Hunter syndrome when the human lysosomal enzyme is iduronate-2-sulfatase; a therapeutic agent for central nervous system disorder in Pompe disease when the human lysosomal enzyme is acid α-glucosidase; a therapeutic agent for central nervous system disorder in Gaucher disease when the human lysosomal enzyme is glucocerebrosidase; a therapeutic agent for central nervous system disorder in GM1-gangliosidosis types 1 to 3 when the human lysosomal enzyme is β-galactosidase; a therapeutic agent for central nervous system disorder in GM2-gangliosidosis AB variant when the human lysosomal enzyme is GM2 activating protein; a therapeutic agent for central nervous system disorder in Sandhoff disease and Tay-Sachs disease when the human lysosomal enzyme is β-hexosaminidase A; a therapeutic agent for central nervous system disorder in Sandhoff disease when the human lysosomal enzyme is β-hexosaminidase B; a therapeutic agent for central nervous system disorder in I-cell disease when the human lysosomal enzyme is N-acetylglucosamine-1-phosphotransferase; a therapeutic agent for central nervous system disorder in α-mannosidosis when the human lysosomal enzyme is α-mannosidase; a therapeutic agent for central nervous system disorder in β-mannosidosis when the human lysosomal enzyme is β-mannosidase; a therapeutic agent for central nervous system disorder in Krabbe disease when the human lysosomal enzyme is galactosylceramidase; a therapeutic agent for central nervous system disorder in Gaucher's disease-like storage disease when the human lysosomal enzyme is saposin C; a therapeutic agent for central nervous system disorder in metachromatic white matter degeneration (metachromatic leukodystrophy) when the human lysosomal enzyme is arylsulfatase A; a therapeutic agent for central nervous system disorder in fucosidosis when the human lysosomal enzyme is α-L-fucosidase; a therapeutic agent for central nervous system disorder in aspartylglucosaminuria when the human lysosomal enzyme is aspartylglucosaminidase; a therapeutic agent for a central nervous system disorder in Schindler disease and Kawasaki disease when the human lysosomal enzyme is α-N-acetylgalactosaminidase; a therapeutic agent for a central nervous system disorder in Niemann-Pick disease when the human lysosomal enzyme is acid sphingomyelinase; a therapeutic agent for a central nervous system disorder in Fabry disease when the human lysosomal enzyme is α-galactosidase A; a therapeutic agent for a central nervous system disorder in Sly syndrome when the human lysosomal enzyme is β-glucuronidase; a therapeutic agent for a central nervous system disorder in Sanfilippo syndrome when the human lysosomal enzyme is heparan N-sulfatase, α-N-acetylglucosaminidase, acetyl-CoA α-glucosaminide N-acetyltransferase, or N-acetylglucosamine-6-sulfatase (in particular, Sanfilippo syndrome A when the human lysosomal enzyme is heparan N-sulfatase, Sanfilippo syndrome B when the human lysosomal enzyme is α-N-acetylglucosaminidase, Sanfilippo syndrome C when the human lysosomal enzyme is acetyl-CoA α-glucosaminide N-acetyltransferase, and Sanfilippo syndrome D when the human lysosomal enzyme is N-acetylglucosamine-6-sulfatase); a therapeutic agent for a central nervous system disorder in Farber disease when the human lysosomal enzyme is acid ceramidase; a therapeutic agent for a central nervous system disorder in Cori's disease (Forbes-Cori's disease) when the human lysosomal enzyme is amylo-1,6-glucosidase; a therapeutic agent for central nervous system disorder in sialidase deficiency when the human lysosomal enzyme is sialidase; a therapeutic agent for central nervous system disorder in aspartylglucosaminuria when the human lysosomal enzyme is aspartylglucosaminidase; a therapeutic agent for central nervous system disorder in neuronal ceroid lipofuscinosis or Santavuori-Haltia disease when the human lysosomal enzyme is palmitoylprotein thioesterase-1 (PPT-1); a therapeutic agent for central nervous system disorder in neuronal ceroid lipofuscinosis or Jansky-Bielschowsky disease when the human lysosomal enzyme is tripeptidylpeptidase-1 (TPP-1); a therapeutic agent for central nervous system disorder in hyaluronidase deficiency when the human lysosomal enzyme is hyaluronidase-1; and a therapeutic agent for central nervous system disorder in Batten disease when the human lysosomal enzyme is either CLN1 or CLN2.

When the nucleic acid molecule is introduced into a cell, the type of the cell is not particularly limited, and examples thereof include a mesenchymal stem cell, a dental pulp-derived stem cell, a hematopoietic stem cell, an embryonic stem cell, an endothelial stem cell, a mammary stem cell, an intestinal stem cell, a hepatic stem cell, a pancreatic stem cell, a neural stem cell, and an iPS cell.

The cell into which the nucleic acid molecule is introduced will express the fusion protein and thus can be transplanted into a patient for therapeutic purposes. For example, the cell into which a nucleic acid molecule in which the physiologically active protein is a human lysosomal enzyme is introduced can be used for the above-mentioned applications.

### Examples

Hereinafter, the present invention will be described in more detail with reference to examples, but the present invention is not intended to be limited to the examples.

### Example 1: Construction of pAAV-mMAP-mscFv-GS3-hI2S vector

A DNA fragment containing a nucleotide sequence represented by SEQ ID NO: 18 and containing, from the 5' side, a ClaI site, a mouse α-fetoprotein enhancer/mouse albumin promoter, a chicken β-actin/MVM chimeric intron, a gene encoding a conjugate in which human iduronate-2-sulfatase (hI2S) bound to the C-terminus of a mouse anti-mouse transferrin receptor single chain antibody (mouse anti-mTfRscFv antibody) via a linker sequence represented by SEQ ID NO: 4, the conjugate having the amino acid sequence represented by SEQ ID NO: 19 (mscFv-GS3-hI2S), a bovine growth hormone polyA sequence, and a BglII site was synthesized. This DNA fragment was digested with ClaI and BglII. The pAAV-CMV vector (Takara Bio Inc.) was digested with ClaI and BglII, and the DNA fragment treated with the above restriction enzymes was inserted into the vector. The resulting plasmid was designated as a pAAV-mMAP-mscFv-GS3-hI2S vector (FIG. 1). The pAAV-mMAP-mscFv-GS3-hI2S vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding mscFv-GS3-hI2S (SEQ ID NO: 20), the bovine growth hormone polyA signal (SEQ ID NO: 17), and a functional equivalent of the second AAV-ITR (SEQ ID NO: 8). Furthermore, the pAAV-mMAP-mscFv-GS3-hI2S vector contains an ampicillin-resistance gene and a replication origin (ColE1 ori). The mouse anti-mTfRscFv antibody was obtained by binding the light chain variable region of mouse anti-mTfR to the C-terminus of the heavy chain variable region of mouse anti-mTfR via a linker having the amino acid sequence represented by SEQ ID NO: 4.

### Example 2: Construction of pAAV-mMAP-mscFv2-GS3-hI2S vector

A DNA fragment containing a nucleotide sequence represented by SEQ ID NO: 21 and containing, from the 5' side, a ClaI site, a mouse α-fetoprotein enhancer/mouse albumin promoter, a chicken β-actin/MVM chimeric intron, a gene encoding a conjugate in which hI2S bound to the C-terminus of a mouse anti-mouse transferrin receptor single chain antibody (mouse anti-mTfRscFv antibody 2) with amino acid substitutions introduced into two positions of CDRs of the heavy chain and two positions of CDRs of the light chain of mscFv via a linker sequence represented by SEQ ID NO: 4, the conjugate having the amino acid sequence represented by SEQ ID NO: 22 (mscFv2-GS3-hI2S), a bovine growth hormone polyA sequence, and a BglII site was synthesized. This DNA fragment was digested with ClaI and BglII. The pAAV-CMV vector (Takara Bio Inc.) was digested with ClaI and BglII, and the DNA fragment treated with the above restriction enzymes was inserted into the vector. The resulting plasmid was designated as a pAAV-mMAP-mscFv2-GS3-hI2S vector (FIG. 2). The pAAV-mMAP-mscFv2-GS3-hI2S vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding mscFv2-GS3-hI2S (SEQ ID NO: 23), the bovine growth hormone polyA signal (SEQ ID NO: 17), and a functional equivalent of the second AAV-ITR (SEQ ID NO: 8). Furthermore, the pAAV-mMAP-mscFv2-GS3-hI2S vector contains an ampicillin-resistance gene and a replication origin (ColE1 ori).

### Example 3: Construction of pAAV-mMAP-hI2S-mscFv2 vector

A DNA fragment containing, from the 5' side, a ClaI site, a mouse α-fetoprotein enhancer/mouse albumin promoter, a chicken β-actin/MVM chimeric intron, a gene encoding a conjugate (hI2S-mscFv2) in which a mouse anti-mTfRscFv antibody 2 bound to the C-terminus of hI2S, the conjugate having the amino acid sequence represented by SEQ ID NO: 25, a bovine growth hormone polyA sequence, and a nucleotide sequence represented by SEQ ID NO: 24 and containing a BglII site was synthesized. This DNA fragment was digested with ClaI and BglII. The pAAV-CMV vector (Takara Bio Inc.) was digested with ClaI and BglII, and the DNA fragment treated with the above restriction enzymes was inserted into the vector. The resulting plasmid was designated as a pAAV-mMAP-hI2S-mscFv2 vector (FIG. 3). The pAAV-mMAP-hI2S-mscFv2 vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding hI2S-mscFv2 (SEQ ID NO: 26), the bovine growth hormone poly A signal (SEQ ID NO: 17), and a functional equivalent of the second AAV-ITR (SEQ ID NO: 8). Furthermore, the pAAV-mMAP-hI2S-mscFv2 vector contains an ampicillin-resistance gene and a replication origin (ColE1 ori).

### Example 4: Construction of pAAV-mMAP-hI2S vector

A DNA fragment containing a nucleotide sequence represented by SEQ ID NO: 27 and containing, from the 5' side, a ClaI site, a mouse α-fetoprotein enhancer/mouse albumin promoter, a chicken β-actin/MVM chimeric intron, a gene encoding hI2S having the amino acid sequence represented by SEQ ID NO: 14, a bovine growth hormone polyA sequence, and a BglII site was synthesized. This DNA fragment was digested with ClaI and BglII. The pAAV-CMV vector (Takara Bio Inc.) was digested with ClaI and BglII, and the DNA fragment treated with the above restriction enzymes was inserted into the vector. The resulting plasmid was designated as a pAAV-mMAP-hI2S vector (FIG. 4). The pAAV-mMAP-hI2S vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding hI2S (SEQ ID NO: 28), the bovine growth hormone polyA signal (SEQ ID NO: 17), and a functional equivalent of the second AAV-ITR (SEQ ID NO: 8). Furthermore, the pAAV-mMAP-hI2S vector contains an ampicillin-resistance gene and a replication origin (ColE1 ori).

### Example 5: Construction of pR2 (mod) C6 vector

A DNA fragment containing a nucleotide sequence represented by SEQ ID NO: 29 and containing, from the 5' side, an AfeI site, a RsrII site, a BsrGI site, an AvrII site, an ampicillin-resistance gene, a replication origin (ColE1 ori), a RsrII site, a 5' portion of an AAV2 Rep region (including a p5 promoter), and a SacII site was synthesized. This DNA fragment was digested with AfeI and SacII. A pRC6 vector (Takara Bio Inc.) was digested with AfeI and SacII, and the above restricted synthetic genes were inserted into the digested vector. The resulting plasmid was designated as a pR2 (mod) C6 vector.

### Example 6: Construction of pR2 (mod) C8 vector

A DNA fragment containing a nucleotide sequence represented by SEQ ID NO: 30 and containing, from the 5' side, a HindIII site, a 3' portion of an AAV2 Rep region, an AAV8 Cap region, a p5 promoter, a RsrII site, and a BsrGI site was synthesized. This DNA fragment was digested with HindIII and BsrGI. The pR2 (mod) C6 vector was digested with HindIII and BsrGI, and the synthetic genes treated with the above restriction enzymes were inserted into the vector. The resulting plasmid was designated as a pR2 (mod) C8 vector (FIG. 5).

### Example 7: Production of rAAV virion

HEK293 cells, which are cell lines derived from human embryonic kidney cells, were used as host cells. The HEK293 cells were seeded at 1.59 × 10⁸ per cell stack chamber in a 10-layer cell stack chamber (Corning) and cultured in a 10% FBS-containing DMEM medium (Thermo Fisher Scientific) at 37°C under 5% CO₂ for 3 days. As a DNA solution for transfection, a solution containing plasmids of the following combinations (1) to (4) was prepared:
(1) a pHelper vector (Takara Bio Inc.), a pR2 (mod) C8 vector, and a pAAV-mMAP-mscFv-GS3-hI2S vector;
(2) a pHelper vector (Takara Bio Inc.), a pR2 (mod) C8 vector, and a pAAV-mMAP-mscFv2-GS3-hI2S vector;
(3) a pHelper vector (Takara Bio Inc.), a pR2 (mod) C8 vector, and a pAAV-mMAP-hI2S-mscFv2 vector;
(4) a pHelper vector (Takara Bio Inc.), a pR2 (mod) C8 vector, and a pAAV-mMAP-hI2S vector.

Each solution contained the three plasmids at equimolar concentrations, and was adjusted to have a total DNA concentration of 1.0 mg/mL and to have a total liquid volume of 2.2 mL. To this solution, polyethyleneimine was added at a weight ratio of DNA (µg): polyethyleneimine (µg) = 1:2. ADMEM medium was further added to adjust a liquid volume to 1.0 L, and a transfection solution was prepared. After removing all of the culture supernatant from the 10-layer cell stack chamber 3 days after the start of culture, the total amount, 1.0 L, of the transfection solution was added. The cells were cultured at 37°C in the presence of 5% CO₂ for 3 days to produce rAAV virions. The rAAV virions produced in the host cell are partially released into the culture supernatant and partially retained within the host cell. The resulting rAAV virions were designated rAAV-mscFv-GS3-hI2S, rAAV-mscFv2-GS3-hI2S, rAAV-hI2S-mscFv2, and rAAV-hI2S, respectively. rAAV-mscFv-GS3-hI2S is expected to express mscFv-GS3-hI2S when cells are infected, rAAV-mscFv2-GS3-hI2S is expected to express mscFv2-GS3-hI2S when cells are infected, rAAV-hI2S-mscFv2 is expected to express hI2S-mscFv2 when cells are infected, and rAAV-hI2S is expected to express hI2S when cells are infected.

### Example 8: Cell lysis and free nucleic acid removal steps

After completion of the culture in Example 7, an aqueous solution containing 0.5 M HEPES, 20 mM MgCl₂, and 10% (w/v) polysorbate 20 in an amount of 1/9 of the volume of each cell culture solution was added to each cell culture solution, and benzonase (Merck) as an endonuclease was further added thereto at a concentration of 50 U/mL and mixed to uniformly disperse the solution in the cell stack, followed by standing at room temperature for 30 minutes. Thereafter, the solution was transferred from the cell stack to a bottle and stirred at room temperature for 3 hours with a stir bar and a stirrer. Then, a 5M NaCl aqueous solution containing 10% (w/v) sucrose in an amount of 1/9 of the liquid volume was added, and the mixture was stirred to obtain a cell lysate.

### Example 9: Preparation of affinity step loading solution

Each cell lysate obtained in Example 8 was aliquoted into 1000 mL flat bottom centrifuge containers and centrifuged at 3000 × g and 4°C for 20 minutes in a Sorvall LYNX 6000 high capacity high speed refrigerated centrifuge (Thermo Fisher Scientific). The centrifuged supernatant was filtered using a Nalgene Rapid-Flow PES filter unit (Thermo Fisher Scientific) having a pore size of 0.2 µm to obtain a cell lysate containing rAAV virions. To this was added 20 mmol/L Tris buffer (pH 7.5) containing 2 mmol/L MgCh and 2000 mmol/L-Arginine in a 1/4 amount, and this was used as an affinity step loading solution.

### Example 10: Chromatographic purification step of rAAV virion using affinity resin

A column (diameter: 1.6 cm, height: 10 cm (column volume: about 20 mL)) packed with POROS Capture Select AAVX resin (Thermo Fisher Scientific) was equilibrated with 20 mmol/L Tris buffer (pH 7.5) containing 2 mmol/L MgCh and 150 mmol/L NaCl. Each affinity step loading solution obtained in Example 9 was passed through the column at a flow rate of 5 mL/min to adsorb the rAAV virions to the resin. Then, 20 mmol/L Tris buffer (pH 7.5) containing 2 mmol/L MgCh and 400 mmol/L-Arginine in an amount of 5 times or more the column volume was passed through the column to wash the column, and 20 mmol/L Tris buffer (pH 7.5) containing 2 mmol/L MgCh and 150 mmol/L NaCl in an amount of 3 times or more the column volume was passed through the column to wash the column. Then, 10 mM citrate buffer (pH 3.5) containing 2 mM MgCh in an amount of 3 times or more the column volume was passed through the column to elute the rAAV virions adsorbed onto the resin, and a fraction containing the rAAV virions was obtained. To this fraction was added 500 mmol/L Tris buffer (pH 8.5) containing 2 mM MgCh to adjust the pH to 7.0, and an affinity step-eluted fraction was obtained.

### Example 11: Concentration step of rAAV virion by ultrafiltration membrane

Each affinity step-eluted fraction obtained in Example 10 were circulated at a flux of 7 L/min/m² or less through Spectrum MicroKros Hollow Fiber Modules 100 kDa, 75 cm² (Repligen) equilibrated with 20 mmol/L Tris buffer (pH 7.5) containing 2 mmol/L MgCh and 150 mmol/L NaCl, concentrated to about 8 mL and collected. Next, the ultrafiltration membrane was washed twice with 2 mL of a 20 mmol/L Tris buffer (pH 7.5) containing 2 mmol/L MgCh and 150 mmol/L NaCl, and this washing solution was combined with the previously collected solution to obtain a concentrated solution of about 12 mL in total.

### Example 12: Purification step of rAAV virions by ultracentrifugation with iodixanol

PBS-MK buffer (20 mmol/L sodium phosphate buffer (pH 7.4) containing 136.9 mmol/L sodium chloride, 28.2 mmol/L potassium chloride and 1 mmol/L magnesium chloride) was added to 60% iodixanol (versatile density gradient centrifugation medium OptiPrep (trade name), Cosmo Bio Co., Ltd.) to prepare a 45% iodixanol solution. A 25% iodixanol solution was prepared by adding PBS-MK buffer to 60% iodixanol. Furthermore, 1 M NaCl/PBS-MK buffer (PBS-MK buffer containing 1 mol/L NaCl) was added to 60% iodixanol to prepare 15% iodixanol solutions.

From the bottom of a 39 mL Quick-Seal (trade name) Round-Top Polypropylene Tube, 25 × 89 mm (Beckman Coulter, Inc.), 5 mL, 8 mL, 5 mL, and 8 mL of a 60% iodixanol solution added with phenol red, a 45% iodixanol solution, a 25% iodixanol solution added with phenol red, and a 15% iodixanol solution, respectively, were layered in this order. About 12 mL of the concentrated solution obtained in Example 11 was added to this, and the tube was filled with 1 M NaCl/PBS-MK buffer. Then, the injection port of the tube was closed with a cordless tube topper sealer kit (Beckman Coulter, Inc.), and the tube was placed in a 70Ti rotor of an ultracentrifuge Optima XPN (Beckman Coulter, Inc.) and centrifuged at a rotational speed of 63000 rpm (408500 × g) for 2 hours. After centrifugation, a Tygon E-LFL tube with a needle was connected to the bottom of the tube using a fraction recovery system (Beckman Coulter, Inc.), an 18 G syringe needle was inserted into the top of the tube to form an air hole, and then 0.5 mL each of the solvent was fractionated from the bottom of the tube using a peristaltic pump. The 9th to 13th or 10th to 14th fractions were collected to obtain fractions containing purified rAAV virions.

### Example 13: Concentration step of rAAV virion by ultrafiltration membrane and buffer exchange step

The fraction containing each rAAV virion obtained in Example 12 was diluted 50-fold with 350 mmol/L NaCl/PBS buffer (20 mmol/L sodium phosphate buffer (pH 7.4) containing 486.9 mmol/L NaCl, 2.7 mmol/L KCl, and 0.001% F-68) and concentrated to about 15 mL by being circulated through Spectrum MicroKros Hollow Fiber Modules 100 kDa, 75 cm² (Repligen) equilibrated with 350 mmol/L NaCl/PBS buffer at a flow rate of 7 L/min/m² or less. A 10 DV buffer exchange was then performed with 350 mmol/L NaCl/PBS buffer. The solution was further concentrated to about 8 mL by circulation at a flow rate of 7 L/min/m² or less, and the concentrated solution was collected. Next, the ultrafiltration membrane was washed twice with 2 mL of 350 mmol/L NaCl/PBS buffer, and the washing solution was combined with the previously collected concentrated solution to obtain a total of about 12 mL of a concentrated solution. This concentrated solution was further concentrated by centrifugation using an Amicon Ultra 15 centrifugal filter unit 50 kDa (Merck) under conditions of 3200 rpm (2100 × g) or less and 20 minutes or less to obtain an rAAV virion solution in which the solvent was replaced with 350 mmol/L NaCl/PB S buffer. Each rAAV virion solution obtained was subjected to the following experiment.

### Example 14: Measurement of amount of rAAV genome contained in rAAV virion solution

The amount of the viral genome in the rAAV virion solution obtained in Example 13 was measured by the droplet digital PCR method. The measurement method will be described in detail below.

To 5 µL of the rAAV virion solution, 2 µL of Recombinant DNaseI (Takara Bio Inc.), 5 µL of 10 × DNaseI Buffer (attached to Recombinant DNaseI, Takara Bio Inc.), 5 µL of 10% F-68-contafining water for injection, and 33 µL of water for injection (Otsuka distilled water, Otsuka Pharmaceutical Factory, Inc.) were added in an 8-tube (TOHO). The mixed solution was incubated at 37°C for 30 minutes to digest DNA not encapsulated in rAAV virions.

The DNaseI-treated rAAV virion solution was appropriately diluted with a DNA-suspension buffer (10 mM Tris buffer solution (pH 8.0) containing 0.1 mM ethylenediaminetetraacetate, 100 µg/mL polyA, and 0.001% F-68). The diluted rAAV virion solution was then incubated at 95°C for 10 minutes to thermally disrupt the rAAV virions, thereby preparing a sample solution for droplet digital PCR.

A FAM-labeled 20 × primer/probe mix was prepared, containing 1.0 µM forward primer (primer SI-1, SEQ ID NO: 33), 1.0 µM reverse primer (primer SI-2, SEQ ID NO: 34), and 0.25 µM probe (probe SI-1, SEQ ID NO: 35 modified with FAM as a reporter dye at the 5' terminus and BHQ1 as a quencher dye at the 3' terminus). A HEX-labeled 20 × primer/probe mix was also prepared, containing 1.0 µM forward primer (primer SI-3, SEQ ID NO: 36), 1.0 µM reverse primer (primer SI-4, SEQ ID NO: 37), and 0.25 µM probe (probe SI-2, SEQ ID NO: 38 modified with HEX as a reporter dye at the 5' terminus and BHQ1 as a quencher dye at the 3' terminus).

To 4 µL of the sample solution for droplet digital PCR, 12 µL of ddPCR Supermix for probes (no dUTP) (BioRad), 1.2 µL of FAM-labeled 20 × primer/probe mix, 1.2 µL of HEX-labeled 20 × primer/probe mix, 0.3 µL of MspI (NEB), and 5.3 µL of water for injection were added to prepare a PCR reaction solution. Suspension droplets of 20 µL of the PCR reaction solution and 70 µL of Droplet Generator oil for Probes (BioRad) were prepared using a Droplet generator (BioRad). The droplets were subjected to QX200 Droplet Digital PCR System (BioRad). PCR was carried out under the following conditions: denaturation reaction (95°C, 10 minutes), 40 cycles of three-step PCR (95°C, 30 seconds → 60°C, 60 seconds → 72°C, 15 seconds), and PCR enzyme inactivation treatment (98°C, 10 minutes). FAM/HEX double positive droplets were defined as rAAV positive droplets, and the amount of rAAV genome (vg: viral genome) was determined using QuantaSoft Version 1.7 (BioRad). The DNA regions amplified in this PCR are bovine growth hormone polyA and the internal regions of the ITRs.

### Example 15: Evaluation of mouse TfR binding activity between conjugate of mouse anti-mTfRscFv antibody and hI2S

Genes encoding conjugates of three types of mouse anti-mTfRscFv antibodies, i.e., mscFv-GS3-hI2S, mscFv2-GS3-hI2S, and hI2S-mscFv2 and hI2S were integrated into expression vectors, respectively. The obtained expression vectors were introduced into CHO cells to obtain cells for expression of three types of conjugates. Each of the obtained cells for expression was cultured to express the conjugate as a recombinant protein, and each recombinant protein was collected from the culture supernatant.

The binding activities of the obtained three types of proteins, mscFv-GS3-hI2S, mscFv2-GS3-hI2S, and hI2S-mscFv2, to the mouse TfR receptor were measured by the ELISA method. The ELISA method was generally performed in the following manner. To each well of a 96-well microtiter plate (Nunc), 100 µL of a mouse transferrin receptor extracellular domain having an amino acid sequence from position 87 to position 763 of an amino acid sequence represented by SEQ ID NO: 39 diluted with 0.05 M bicarbonate buffer (pH 9.6) to a concentration of 10 µg/mL was added, and the plate was allowed to stand at room temperature for at least 1 hour to adsorb the antibody to the plate. Then, 300 µL of TBS containing 1% BSA and 0.05% Tween20 was added to each well, and the plate was allowed to stand at room temperature for 1 hour. Each well was washed three times with TBS containing 0.05% Tween20 (TBS-T), followed by dilution with TBS containing 0.1% BSA and 0.05% Tween20 to 300, 100, 33.33, 11.11, 3.70, 1.23, 0.41, or 0.14 nM for mscFv-GS3-hI2S, 600, 200, 66.67, 22.22, 7.41, 2.47, 0.82, or 0.27 nM for mscFv2-GS3-hI2S, and 1200, 400, 133.33, 44.44, 14.81, 4.94, 1.65, or 0.55 nM for hI2S-mscFv2, and 100 µL of each thereof was added to each well. The plate was allowed to stand at room temperature for at least 1 hour. After the plate was washed three times with TBS-T, anti-hI2S monoclonal antibodies were diluted to 0.5 µg/mL with TBS containing 0.1% BSA and 0.05% Tween20, 100 µL thereof was added to each well, and the plate was allowed to stand at room temperature for at least 1 hour. The plate was washed three times with the TBS-T, then 100 µL of an HRP-labeled anti-human IgG polyclonal antibody (Bethyl) solution diluted to 50 ng/mL with TBS containing 0.1% BSA and 0.05% Tween20 was added to each well, and the plate was allowed to stand at room temperature for at least 1 hour. Each well was washed three times with TBS-T, then 50 µL of a chromogenic substrate solution TMB Stabilized Substrate for Horseradish Peroxidase (Promega) was added to each well, and the mixture was allowed to stand at room temperature for from 5 to 10 minutes. Then, 100 µL of a stop solution (1 N hydrochloride) was added to each well, and the absorbance at 450 nm for each well was measured using a plate reader (SpectraMax iD3, MOLECULAR DEVICES). EC₅₀ was calculated from the measured values, and the binding activity of each protein to mTfR was evaluated.

The EC₅₀ values of mscFv-GS3-hI2S, mscFv2-GS3-hI2S, and hI2S-mscFv2 with respect to mouse TfR were 2.4 nM, 13.2 nM, and 96.7 nM, respectively.

### Example 16: Evaluation of efficacy of each rAAV virion using I2S-KO mouse

I2S gene-knockout mice (I2S-KO mice) were used to evaluate the efficacy of each rAAV when administered to living bodies. The I2S-KO mice used were male mice at 14 to 15 weeks of age (at the start of administration). As the rAAV virions, rAAV-mscFv-GS3-hI2S, rAAV-mscFv2-GS3-hI2S, rAAV-hI2S-mscFv2, and rAAV-hI2S were single-administered to the male I2S-KO mice, in a tail vein, at doses of 1.0 × 10¹¹ vg/kg, 1.0 × 10¹² vg/kg, and 1.0 × 10¹³ vg/kg, respectively (N = 3 for each group). In addition, a normal control group composed of male wild-type mice and a pathological control group composed of male I2S-KO mice) were set (N = 3 for each group). Physiological saline (Otsuka Pharmaceutical Factory, Inc.) was administered to the normal control group and the pathological control group.

One week and six weeks after the administration, the chest was opened to collect heart blood, under anesthesia with a mixture of three drugs, Vetorphale (Meiji Seika Pharma co., Ltd.), midazolam (Sandoz) and Domitor (Nippon Zenyaku Kogyo Co., Ltd.). The bloods were collected into EDTA-2K coated blood-collecting tubes (Capiject, Terumo Corporation) to obtain anticoagulated bloods, which were then subjected to measurement of hemoglobin concentrations. The remaining anticoagulated blood was allowed to stand on ice and then centrifuged (2000 × g, 20 minutes, 4°C), and plasma was collected. In addition, the mouse after heart blood collection was systemically perfused with physiological saline (Otsuka Pharmaceutical Factory, Inc.), and the brain was removed. A part of brain tissue was dispensed for quantification of heparan sulfate in the brain tissue and quickly immersed in liquid nitrogen for quick freezing. Separately, a part of the brain tissue was measured for wet weight and quickly frozen by immersion in liquid nitrogen, and the frozen brain tissue was homogenized in RIPA Buffer (Wako Pure Chemical Industries, Ltd.) containing 0.025% Protease Inhibitor Cocktail (Sigma-Aldrich), centrifuged, and the supernatant was collected.

### Example 17: Measurement of rAAV-derived protein concentration in plasma and brain tissue

Each of rAAV-derived proteins in rAAV-mscFv-GS3-hI2S, rAAV-mscFv2-GS3-hI2S, rAAV-hI2S-mscFv2, and rAAV-hI2S contained in the plasma prepared in Example 16 and the supernatant obtained by homogenizing brain tissue was quantified. Quantitation was generally performed in the following manner. One hundred and fifty (150) µL of Superblock Blocking Buffer in PBS (Thermo Fisher Scientific) was added to each well of a Streptavidin Gold plate (Meso scale diagnostics), and the plate was blocked by shaking for 1 hour or more. Then, to a mixed solution of biotinylated anti-hI2S monoclonal antibodies and SULFOnated anti-hI2S monoclonal antibodies, each standard sample for calibration curve containing a known concentration of mscFv-GS3-hI2S, hI2S-mscFv2, or hI2S obtained by expression in CHO cells by an ordinary method and each analyte were added and shaken for 1 hour to obtain an antibody reaction solution. The anti-hI2S monoclonal antibodies were obtained by culturing an anti-hI2S producing hybridoma prepared by an ordinary method using splenocytes obtained from a mouse immunized with hI2S having the amino acid sequence of SEQ ID NO: 14. One of the obtained monoclonal antibodies was modified using Biotin Labeling Kit-NH2 (Dojindo Laboratories) according to the attached protocol to obtain biotinylated anti-hI2S monoclonal antibodies. Another one of the obtained monoclonal antibodies was modified using MSD GOLD SULFO-TAG NHS-Ester (Meso Scale Diagnostics) according to the attached protocol to obtain SULFOnated anti-hI2S monoclonal antibodies. The blocking solution was removed from each well. After washing with PBS containing 0.05% Tween 20 (PBST, Sigma Aldrich), 25 µL of the antibody reaction solution was added to each well and shaken for 1 hour or more. Then, the antibody reaction solution was removed. After washing with PBST, 150 µL of 4 × Read Buffer (Meso scale diagnostics) mixed and diluted in an equal amount with water for injection (Otsuka Pharmaceutical Factory, Inc.) was added, and the amount of luminescence from each well was measured using Sector (trade name) Imager 6000 (Meso scale diagnostics). A calibration curve was prepared from the measured values of each standard sample for calibration curve, and the measured value of each analyte was interpolated into the calibration curve to calculate the amount of the rAAV-derived protein contained per mL of the analyte (concentration of each rAAV-derived protein). In the measurement of mscFv2-GS3-hI2S, mscFv-GS3-hI2S was used as the standard sample for calibration curve.

The measurement results of the rAAV-derived protein concentration in plasma are illustrated in FIG. 6. The concentration of each rAAV-derived protein in plasma increased in a dosage-dependent manner in all the rAAV virion administration groups. In each group, the expression level was maintained at the same level or slightly increased between one week and 6 weeks after the administration. The above results show that rAAV virions injected intravenously into mice were taken up into cells, and that at least rAAV-derived proteins expressed by transcription and translation of genes encoded by rAAV in the cells were released into blood.

The measurement results of the concentration of rAAV-derived protein in the brain tissue are illustrated in FIG. 7. The concentration of the expressed protein in the brain tissue increased in a dosage-dependent manner in all the rAAV virion administration groups. The expression concentration of mscFv-GS3-hI2S in the brain tissue in the rAAV-mscFv-GS3-hI2S administration group was very high when compared among the same dose groups of rAAV-mscFv2-GS3-hI2S, rAAV-hI2S-mscFv2, and rAAV-hI2S, and the concentration did not vary greatly between one week and 6 weeks after the administration. In the rAAV-mscFv2-GS3-hI2S and rAAV-hI2S-mscFv2 (1.0 × 10¹³ vg/kg) administration groups, the concentration in the brain tissue increased about 3.4 times and about 2.1 times, respectively, from one week to 6 weeks after the administration. The concentration in the brain tissue in the rAAV-hI2S (1.0 × 10¹³ vg/kg) administration group did not vary greatly between one week and 6 weeks after the administration, and was not more than half the concentration in the brain tissue in the other groups, in the case of the same dose.

The above results show that, by administering rAAV-mscFv-GS3-hI2S, rAAV-mscFv2-GS3-hI2S, and rAAV-hI2S-mscFv2, the conjugates of anti-mTfR antibody scFv and hI2S, which were expected to be expressed in the mouse body, were actually expressed as expected, and that the anti-mTfR antibody scFv was bound to mTfR, and thus passed through the BBB and reached the tissues of the central nervous system.

### Example 18: Quantification of heparan sulfate in brain tissue

Quantification of heparan sulfate (HS) in the brain was generally performed in the following manner. Heparan sulfate is a substrate for the enzyme I2S.

Solutions (a) to (l) used in the test were prepared by the following procedure.
(a) MeCN/water:
   Two (2) mL of water for injection (Otsuka Pharmaceutical Co., Ltd.) and 18 mL of acetonitrile (FUJIFILM Wako Pure Chemical Corporation) were mixed, and this was used as MeCN/water.
(b) Deuterium labeling solvent:
   Under an ice bath, 240 µL of acetyl chloride (Sigma-Aldrich) was added dropwise to 1.5 mL of methanol-d₄ (Sigma-Aldrich), and this was used as a deuterium labeling solvent.
(c) Mobile phase A:
   A mixture of 475 mL of water for injection and 25 mL of a 1 M ammonium formate aqueous solution (FUJIFILM Wako Pure Chemical Corporation) was used as a mobile phase A.
(d) Mobile phase B:
   A mixture of acetonitrile and mobile phase A at a ratio of 93:7 (v/v) was used as a mobile phase B.
(e) Heparan sulfate standard stock solution (HS standard stock solution):
   Heparan sulphate (Iduron) was dissolved in water for injection to prepare a 5.0 mg/mL solution. This solution was used as an HS standard stock solution.
(f) Heparan sulfate internal standard solution (HS internal standard solution):
   Into a borosilicate screw-top test tube, 40 µL of the HS standard stock solution was pipetted, and the solvent was distilled off under a nitrogen stream. To the dried product, 400 µL of the deuterium-labeling solvent was added, and the mixture was stirred and then reacted at 65°C for 75 minutes to perform deuteriomethanolysis (methanolysis). After the reaction, the solvent was distilled off under a nitrogen stream. To the dried product, 500 µL of the MeCN/water was added, and the mixture was ultrasonicated for 30 minutes. This solution was used as a heparan sulfate internal standard solution (HS internal standard solution).
(i) Internal standard solution:
   To 1 mL of methanol, 1 µL of the HS internal standard solution was added, and the mixture was stirred and then ultrasonicated for 30 minutes. This solution was used as an internal standard solution.
(j) Calibration curve sample:
   980 µL of water for injection was pipetted, and 10 µL of the HS standard stock solution was added thereto to prepare a solution containing 50 µg/mL of heparan sulfate. This solution was diluted with water for injection to prepare a solution containing 5000 ng/mL of heparan sulfate. This solution was serially diluted with water for injection to prepare solutions containing heparan sulfate at concentrations of 25, 50, 100, 250, 500, 1000, 2500 and 5000 ng/mL. Twenty (20) µL of these solutions were pipetted and individually dispensed into borosilicate screw cap test tubes. This solution was used as a calibration curve sample.
(k) Solution for tissue extraction
   One (1) mL of polyoxyethylene (10) octylphenyl ether was added to the physiological saline to bring the total volume to 500 mL. This solution was used as a solution for tissue extraction.
(l) 10% ammonium carbonate solution
   Five (5) g of ammonium carbonate was dissolved in 50 mL of water for injection. This solution was used as a 10% ammonium carbonate solution.

Twenty (20) µL of plasma was pipetted and dispensed into a borosilicate screw cap test tube. This solution was used as a blood sample solution.

The brain tissue dispensed in Example 16 was freeze-dried and the dry weight was measured. The freeze-dried tissue was disrupted in a solution for tissue extraction, and the supernatant was centrifuged. Twenty (20) µL of this was pipetted and dispensed into a borosilicate screw cap test tube. This solution was used as a sample solution.

The solvent in each prepared sample solution and calibration curve sample was distilled off under a nitrogen stream. To the dried product, 20 µL of 2,2-dimethoxypropane (Tokyo Chemical Industry Co., Ltd.) and 200 µL of methanol hydrochloride (Sigma-Aldrich) with a hydrochloride concentration of 3 mol/L were added, and the mixture was stirred and then subjected to a methanolysis reaction at a reaction temperature of 70°C for a reaction time of 90 minutes. After the reaction, the mixture was cooled on ice to stop the reaction, and 200 µL of the 10% ammonium carbonate solution and 50 µL of the internal standard solution were added thereto. After the solvent was distilled off under a nitrogen stream, 250 µL of water for injection was added to the dried product. This was loaded on a solid-phase cartridge (OASIS HLB (1 cc, 30 mg, Waters Corporation)) which had been conditioned by passing methanol and water for injection therethrough in advance, washed with water for injection, added with 500 µL of methanol, and eluted by centrifugation. After the solvent was distilled off under a nitrogen stream, 2 mL of water for injection and 18 mL of acetonitrile were added, and the mixture was dissolved again by ultrasonic treatment. After centrifugation, the supernatant was filled into an LC vial.

LC/MS/MS analysis was performed using a combination of hydrophilic interaction ultra-high performance liquid chromatography and a tandem quadrupole mass spectrometer. QTRAP5500 (AB Sciex) was used as a mass spectrometer (MS/MS instrument), and Nexera X2 (Shimadzu Corporation) was set as an HPLC instrument to this. In addition, Acquity UPLC (trade name) BEH Amide 1.7 µm (2.1 × 150 mm, Waters Corporation) was used as an LC column. The mobile phases A and B were used as mobile phases. Furthermore, the column temperature was set at 60°C.

After the column was equilibrated with the mobile phase B, 5 µL of a sample was injected, and the chromatography was performed under the gradient conditions of the mobile phases shown in Table 1. The flow rate of the mobile phases was set at 0.4 mL/min.

**[Table 1]**

| (Table 1) Conditions for liquid chromatography | | |
|---|---|---|
| Elapsed time (min) after sample injection | Mobile phase A (% (v/v)) | Mobile phase B (% (v/v)) |
| 0 | 0 | 100 |
| 5.00 | 0 | 100 |
| 7.00 | 20 | 80 |
| 12.5 | 20 | 80 |
| 13.0 | 70 | 30 |
| 15.0 | 70 | 30 |
| 15.5 | 0 | 100 |
| 20.0 | 0 | 100 |

The ion source parameters of the MS/MS instrument were set according to the instruction manual of QTRAP5500 (AB Sciex) as shown in Table 2.

**[Table 2]**

| (Table 2) Setting of various parameters of ion source of MS/MS instrument | |
|---|---|
| Ion Source | ESI (Turbo Spray) |
| Polarity | Positive |
| Scan type | MRM |
| IonSpray voltage | 5500 V |
| Heater gas temperature | 425 °C |
| Curtain gas (CUR) | 30.00 |
| Collision gas (CAD) | 8.00 |
| Ion source gas 1 (GS1) | 70.00 |
| Ion source gas 2 (GS2) | 70.00 |
| Entrance potential | 10.00 |
| Duration | 15.00 min |

MS internal parameters are shown in Table 3.

**[Table 3]**

| (Table 3) MS internal parameters | | | | | | |
|---|---|---|---|---|---|---|
| | Precursor ion (m/z) Q1 | Product ion (m/z) Q3 | Time (msec) | DP (volts) | collision energy (volts) | CXP (volts) |
| HS-derived substance | 384.100 | 162.100 | 100.00 | 45.00 | 21.00 | 15.00 |
| HS internal standard substance | 390.200 | 162.100 | 100.00 | 45.00 | 21.00 | 15.00 |

The calibration curve sample was subjected to LC/MS/MS analysis, and the area of the peak on the chromatogram chart corresponding to the product ion derived from heparan sulfate in the calibration curve sample (HS detection peak area) was determined. In addition, the area of the detection peak corresponding to the product ion derived from the HS internal standard solution (HS-IS detection peak area) was determined.

In each calibration curve sample, the area of the detection peak derived from heparan sulfate with respect to the detection peak area derived from the HS internal standard solution (HS detection peak area/HS-IS detection peak area) was taken as the vertical axis, the heparan sulfate concentration of each calibration curve sample was taken as the horizontal axis, and a regression equation was obtained using quadratic discriminant analysis.

The brain tissue sample solution was subjected to LC/MS/MS analysis, and heparan sulfate contained in the sample solution was interpolated into the regression equation and quantified.

The measurement results of the HS concentration (µg/mg dry weight) in the brain tissue are illustrated in FIG. 8. The concentration of HS in the brain tissue decreased in a dosage-dependent manner in all the rAAV virion administration groups. In the results 6 weeks after the administration, the HS concentrations of each of the rAAV-mscFv-GS3-hI2S, rAAV-mscFv2-GS3-hI2S, and rAAV-hI2S-mscFv2 (1.0 × 10¹³ vg/kg) administration groups was significantly decreased and was almost the same as that of the normal control group. In the rAAV-mscFv-GS3-hI2S administration group, the HS reduction effect was higher in the 1.0 × 10¹² vg/kg and 1.0 × 10¹¹ vg/kg administration groups than in the same dose groups of the other rAAV virions. The above results show that, when rAAV virions were intravenously administered, the conjugate of anti-mTfR antibody scFv and hI2S expressed in the liver was distributed to the brain, and that the HS accumulated in the brain tissue was reduced by I2S exhibiting its activity. The results also show that the effect of reducing HS accumulated in the brain tissue lasts for at least 6 weeks.

### Example 19: Measurement of concentration of hemoglobin in blood

Using the anticoagulated blood obtained in Example 16, the hemoglobin concentration was measured. The hemoglobin concentration was measured by an SLS hemoglobin method using a laboratory automatic hemocytometer (Procyte Dx Research, Sysmex Corporation). The blood hemoglobin concentration is an item usually used as an index of anaemia. The results are illustrated in FIG. 9.

Six weeks after the administration, a decrease in blood hemoglobin concentration was confirmed only in the rAAV-mscFv-GS3-hI2S (1.0 × 10¹³ vg/kg) administration group. In the other groups, no clear difference was observed between one week and 6 weeks after the administration as compared with the normal control group and the pathological control group. The above results show that anaemia can be avoided by adjusting the binding activity to TfR to, for example, from 10 to 100 nM.

### Example 20: Conclusion 1

The above results show that the anti-TfR antibody-fused hI2S can be expressed in vivo for a long period of time by single intravenous administration of rAAV virions having the DNA encoding the anti-TfR antibody-fused hI2S, and that the expressed anti-TfR antibody-fused hI2S can degrade the substrate distributed and accumulated in the central nervous system tissue of the pathological model mouse. In addition, the results show that, by producing rAAV virions containing genes encoding fusion proteins of anti-TfR antibodies and hI2S whose binding activity (EC₅₀) to TfR via anti-TfR is adjusted to 10 nM or more, for example, from 10 to 100 nM, rAAV virions for safe treatment, which do not cause anaemia, can be provided while maintaining high expression levels of the fusion proteins in the blood and exerting the efficacy of reducing HS accumulated in the central nervous system by virtue of the enzymatic activity of hI2S. Furthermore, the results show that, by producing rAAV virions containing genes encoding fusion proteins of anti-TfR antibodies and desired physiologically active proteins whose binding activity (EC₅₀) to TfR via anti-TfR is adjusted to 10 nM or more, for example, from 10 to 100 nM, not limited to hI2S, rAAV virions for safe treatment, which do not cause anaemia, can be provided, while maintaining high expression levels of the fusion proteins in the blood and exerting the activity of the physiologically active proteins in the central nervous system. Furthermore, in the case of the fusion proteins of anti-TfR antibodies and hI2S, even rAAV virions containing genes encoding the fusion proteins of anti-TfR antibodies and hI2S whose binding activity (EC₅₀) to TfR via anti-TfR is adjusted to 10 nM or less, for example, from 0.5 to 4 nM, can be used as safe therapeutic rAAV virions that do not cause anaemia when the dosage per administration is less than 1.0 × 10¹³ vg/kg, for example, 7.5 × 10¹² vg/kg.

### Example 21: Construction of pAAV-mMAP-mscFv3-GS3-hI2S vector

In order to verify the conclusions presented in Examples 19 and 20 above, the following experiments were further conducted. A DNA fragment containing a nucleotide sequence represented by SEQ ID NO: 57 and containing, from the 5' side, a ClaI site, a mouse α-fetoprotein enhancer/mouse albumin promoter, a chicken β-actin/MVM chimeric intron, a gene encoding a conjugate in which human iduronate-2-sulfatase (hI2S) bound to the C-terminus of a mouse anti-mouse transferrin receptor single chain antibody (mscFv 3) with two amino acid substitutions introduced into CDRs of the heavy chain of mscFv via a linker sequence represented by SEQ ID NO: 4, the conjugate having the amino acid sequence represented by SEQ ID NO: 56 (mscFv3-GS3-hI2S), a bovine growth hormone polyA sequence, and a BglII site was synthesized. This DNA fragment was digested with ClaI and BglII. The pAAV-CMV vector (Takara Bio Inc.) was digested with ClaI and BglII, and the DNA fragment treated with the above restriction enzymes was inserted into the vector. The resulting plasmid was designated as a pAAV-mMAP-mscFv3-GS3-hI2S vector. The pAAV-mMAP-mscFv3-GS3-hI2S vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding mscFv3-GS3-hI2S, the bovine growth hormone polyA signal (SEQ ID NO: 17), and a functional equivalent of the second AAV-ITR (SEQ ID NO: 8). The pAAV-mMAP-mscFv3-GS3-hI2S vector generally has a structure in which the genes encoding I2S are replaced with the genes encoding mscFv3-GS3-hI2S in the vector map illustrated in FIG. 4.

### Example 22: Production of rAAV virion

HEK293 cells, which are cell lines derived from human embryonic kidney cells, were used as host cells. The HEK293 cells were seeded at 1.59 × 10⁸ per cell stack chamber in a 10-layer cell stack chamber (Corning) and cultured in a 10% FBS-containing DMEM medium (Thermo Fisher Scientific) at 37°C under 5% CO₂ for 3 days. As a DNA solution for transfection, a solution containing the following plasmids was prepared:
a pHelper vector (Takara Bio Inc.), the pR2 (mod) C8 vector constructed in Example 6, and the pAAV-mMAP-mscFv3-GS3-hI2S vector constructed in Example 21.

The solution contained the three plasmids at equimolar concentrations, and adjusted to have a total DNA concentration of 1.0 mg/mL and to have a total liquid volume of 2.2 mL. To this solution, polyethyleneimine was added at a weight ratio of DNA (µg): polyethyleneimine (µg) = 1:2. ADMEM medium was further added to adjust a liquid volume to 1.0 L, and a transfection solution was prepared. After removing all of the culture supernatant from the 10-layer cell stack chamber 3 days after the start of culture, the total amount, 1.0 L, of the transfection solution was added. The cells were cultured at 37°C in the presence of 5% CO₂ for 3 days to produce rAAV virions. The rAAV virions produced in the host cell are partially released into the culture supernatant and partially retained within the host cell. The resulting rAAV virions were designated rAAV-mscFv3-GS3-hI2S. rAAV-mscFv3-GS3-hI2S is expected to express mscFv3-GS3-hI2S when cells are infected.

### Example 23: Preparation of rAAV virion solution containing rAAV-mscFv3-GS3-hI2S and measurement of rAAV genome amount

After completion of the culture in Example 22, rAAV virion solutions containing rAAV-mscFv3-GS3-hI2S (rAAV-mscFv3-GS3-hI2S solution) were prepared from the culture supernatants by the methods described in Examples 8 to 13. Further, the amount of rAAV genome contained in the rAAV virion solution was measured by the method described in Example 14.

### Example 24: mTfR binding activity evaluation of mscFv3-GS3-hI2S

The recombinant proteins of mscFv3-GS3-hI2S obtained by an ordinary method using CHO cells were measured for their binding activity to mouse transferrin receptor by the ELISA method. The ELISA method was generally performed in the following manner. To each well of a 96-well microtiter plate (Nunc), 100 µL of a mouse transferrin receptor extracellular domain having an amino acid sequence from position 87 to position 763 of an amino acid sequence represented by SEQ ID NO: 39 diluted with 0.05 M bicarbonate buffer (pH 9.6) to a concentration of 10 µg/mL was added, and the plate was allowed to stand at room temperature for at least 1 hour to adsorb the antibody to the plate. Then, 300 µL of TBS containing 1% BSA and 0.05% Tween20 was added to each well, and the plate was allowed to stand at room temperature for 1 hour. Each well was washed three times with TBS containing 0.05% Tween20 (TBS-T). Then, a solution containing mscFv3-GS3-hI2S was diluted using TBS containing 0.1% BSA and 0.05% Tween20 to a concentration of mscFv3-GS3-hI2S of 200, 66.7, 22.2, 7.41, 2.47, 0.82, and 0.27 nM, and 100 µL of the diluted solution was added to each well. The plate was allowed to stand at room temperature for at least 1 hour. After the plate was washed three times with TBS-T, 100 µL of anti-I2S monoclonal antibodies diluted to 0.5 µg/mL with TBS containing 0.1% BSAand 0.05% Tween20 was added to each well, and the plate was allowed to stand at room temperature for at least 1 hour. The plate was washed three times with the TBS-T, then 100 µL of an HRP-labeled anti-human IgG polyclonal antibody (Bethyl) solution diluted to 50 ng/mL with TBS containing 0.1% BSA and 0.05% Tween20 was added to each well, and the plate was allowed to stand at room temperature for at least 1 hour. Each well was washed three times with TBS-T, then 50 µL of a chromogenic substrate solution TMB Stabilized Substrate for Horseradish Peroxidase (Promega) was added to each well, and the mixture was allowed to stand at room temperature for from 5 to 10 minutes. Then, 100 µL of a stop solution (1 N hydrochloride) was added to each well, and the absorbance at 450 nm for each well was measured using a plate reader (MOLECULAR DEVICES). EC₅₀ was calculated from the measured values, and the binding activity of mscFv3-GS3-hI2S to mTfR was evaluated. mscFv3-GS3-hI2S had an EC₅₀ with respect to mouse TfR of 4.4 nM.

### Example 25: Evaluation of efficacy of rAAV-mscFv3-GS3-hI2S using I2S-KO mouse

I2S gene-knockout mice (I2S-KO mice) were used to evaluate the efficacy of each rAAV when administered to living bodies. The I2S-KO mice used were male mice at 14 to 15 weeks of age (at the start of administration). As rAAV virions, rAAV-mscFv3-GS3-hI2S was single-administered to male I2S-KO mice in a tail vein at doses of 1.0 × 10¹¹ vg/kg, 1.0 × 10¹² vg/kg and 1.0 × 10¹³ vg/kg (N = 5 or 6 for each group). In addition, a normal control group composed of male wild-type mice and a pathological control group composed of male I2S-KO mice) were set (N = 2 or 5 for each group). Physiological saline (Otsuka Pharmaceutical Factory, Inc.) was administered to the normal control group and the pathological control group.

Six weeks after the administration, cerebrospinal fluid (CSF) was collected from the large tank of the mice and the chest was opened to collect heart blood, under anesthesia with a mixture of three drugs, Vetorphale (Meiji Seika Pharma co., Ltd.), midazolam (Sandoz) and Domitor (Nippon Zenyaku Kogyo Co., Ltd.). The bloods were collected into EDTA-2K coated blood-collecting tubes (Capiject, Terumo Corporation) to obtain anticoagulated bloods, which were then subjected to measurement of hemoglobin concentrations. The hemoglobin concentration was measured by the SLS hemoglobin method described in Example 19. The remaining anticoagulated blood was allowed to stand on ice and then centrifuged (2000 × g, 20 minutes, 4°C), and plasma was collected. In addition, the mouse after heart blood collection was systemically perfused with physiological saline (Otsuka Pharmaceutical Factory, Inc.), and the brain was removed. Apart of brain tissue was dispensed for quantification of heparan sulfate in the brain tissue and quickly immersed in liquid nitrogen for quick freezing. Separately, a part of the brain tissue was measured for wet weight and quickly frozen by immersion in liquid nitrogen, and the frozen brain tissue was homogenized in RIPA Buffer (Wako Pure Chemical Industries, Ltd.) containing 0.025% Protease Inhibitor Cocktail (Sigma-Aldrich), centrifuged, and the supernatant was collected.

### Example 26: Measurement ofrAAV-derived protein concentration in CSF, plasma and brain tissue

The amounts of mscFv3-GS3-hI2S derived from rAAV-mscFv3-GS3-hI2S contained in the plasma prepared in Example 25 and the supernatant obtained by homogenizing brain tissue were measured by the method described in Example 17. The measurement results of mscFv3-GS3-hI2S contained in plasma are illustrated in FIG. 10. Since the concentration of mscFv3-GS3-hI2S in plasma increased in a dosage-dependent manner, it can be seen that rAAV virions injected intravenously into mice were taken up into cells, and that at least mscFv3-GS3-hI2S expressed by transcription and translation of genes encoding mscFv3-GS3-hI2S in the cells was released into the blood. Next, the measurement results of the concentration of mscFv3-GS3-hI2S contained in the brain tissue are illustrated in FIG. 11. Since the concentration of mscFv3-GS3-hI2S in plasma increased in a dosage-dependent manner, it can be seen that mscFv3-GS3-hI2S expressed in mouse cells was released into the blood, and that at least a part thereof was bound to mouse TfR, and thus passed through the BBB, and reached the tissues of the central nervous system.

### Example 27: Quantification of heparan sulfate in brain tissue and CSF

The brain tissue frozen in Example 25 was freeze-dried and the dry weight was then measured. The freeze-dried tissue was disrupted in a solution for tissue extraction, and the supernatant was centrifuged. Twenty (20) µL of this was pipetted and dispensed into a borosilicate screw cap test tube. This solution was used as a sample solution. In the measurement of CSF, 20 µL of CSF was pipetted and dispensed into a borosilicate screw-top test tube, which was used as a sample solution. Using the prepared sample solution, heparan sulfate (HS) in the brain tissue and CSF was quantified by the method described in Example 18.

The measurement results of the HS concentration (µg/mg dry weight) in the brain tissue and HS concentration (µg/mL) in CSF are illustrated in FIGS. 12 and 13, respectively. The HS concentrations in both brain tissue and CSF decreased in a dosage-dependent manner. Even in the 1.0 × 10¹¹ vg/kg administration group, the HS concentrations in both brain tissue and CSF were significantly reduced. These results show that, when rAAV-mscFv3-GS3-hI2S is intravenously administered, mscFv3-GS3-hI2S expressed in mouse cells is released into the blood, that at least a part thereof binds to mouse TfR, and thus passes through the BBB and reaches the tissues of the central nervous system, and furthermore that the enzymatic activity of hI2S to reduce the HS concentration can be exerted in the central nervous system. The results also show that the effect of reducing HS accumulated in the brain tissue lasts for at least 6 weeks.

### Example 28: Measurement of concentration of hemoglobin in blood

The hemoglobin concentration was measured using the anticoagulated blood obtained in Example 25. The hemoglobin concentration was measured by the SLS hemoglobin method described in Example 19. The blood hemoglobin concentration is an item usually used as an index of anaemia. The results are shown in FIG. 14.

Six weeks after the administration, in all of the rAAV-mscFv3-GS3-hI2S administration groups, there was no apparent difference in change in blood hemoglobin concentration as compared with the normal control group or the pathological control group. The above results show that, when the EC₅₀ of a fusion protein of an anti-TfR antibody and a physiologically active protein to the transferrin receptor via the anti-TfR antibody is about 4.4 nM, the function of the physiologically active protein can be exerted in the central nervous system by intravenous administration of rAAV virions encoding the fusion protein without decreasing the concentration of hemoglobin in the blood, i.e., while avoiding anaemia.

### Example 29: Conclusion 2

In consideration together with Conclusion 1 of Example 20, the above results show that, by producing rAAV virions containing genes encoding fusion proteins of anti-TfR antibodies and hI2S whose binding activity (EC₅₀) to TfR via anti-TfR is adjusted to 3 nM or more, for example, from 3 to 100 nM, rAAV virions for safe treatment, which do not cause anaemia, can be provided while maintaining high expression levels of the fusion proteins in the blood over a long period of time and exerting the efficacy of reducing HS accumulated in the central nervous system by virtue of the enzymatic activity of hI2S. Furthermore, by producing rAAV virions containing genes encoding fusion proteins of anti-TfR antibodies and desired physiologically active proteins whose anti-TfR-mediated binding activity (EC₅₀) to TfR is adjusted to 3 nM or more, for example, 3 to 100 nM, not limited to hI2S, it is possible to provide rAAV virions for safe treatment that do not cause anaemia while maintaining high expression levels of the fusion proteins in the blood and exerting the activity of the physiologically active proteins in the central nervous system. Furthermore, in the case of the fusion proteins of anti-TfR antibodies and hI2S, even rAAV virions containing genes encoding the fusion proteins of anti-TfR antibodies and hI2S whose binding activity (EC₅₀) to TfR via anti-TfR is adjusted to 4 nM or less, for example, from 0.5 to 4 nM, can be used as safe therapeutic rAAV virions that do not cause anaemia when the dosage per administration is less than 1.0 × 10¹³ vg/kg, for example, 7.5 × 10¹² vg/kg.

### Example 30: Construction of pAAV-mMAP-mscFv-GS3-hGAA vector

A DNA fragment containing a nucleotide sequence represented by SEQ ID NO: 59 and containing, from the 5' side, a BamHI site, a gene encoding hGAA having an amino acid sequence of SEQ ID NO: 58, and a NotI site was synthesized. This DNA fragment was digested with BamHI and NotI. The pAAV-mMAP-mscFv-GS3-hI2S constructed in Example 1 was digested with BamHI and NotI, and the DNA fragment treated with the above restriction enzymes was inserted into this. The resulting plasmid was designated as a pAAV-mMAP-mscFv-GS3-hGAA vector. The pAAV-mMAP-mscFv-GS3-hGAA vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), a gene encoding a conjugate (mscFv-GS3-hGAA) in which hGAA bound to the C-terminus of a mouse anti-mouse transferrin receptor single chain antibody (mouse anti-mTfRscFv antibody) via a linker represented by SEQ ID NO: 4, the conjugate having an amino acid sequence shown in SEQ ID NO: 60, the bovine growth hormone polyA signal (SEQ ID NO: 17), and a functional equivalent of the second AAV-ITR (SEQ ID NO: 8). The pAAV-mMAP-mscFv-GS3-hGAA vector generally has a structure in which the genes encoding I2S are replaced with the genes encoding mscFv-GS3-hGAA in the vector map illustrated in FIG. 4.

### Example 31: Construction of pAAV-mMAP-hGAA (Pro)-GS-mscFv2 vector

A DNA fragment containing a nucleotide sequence represented by SEQ ID NO: 62 and containing, from the 5' side, a MluI site, a gene encoding hGAA (Pro) having an amino acid sequence represented by SEQ ID NO: 61, a gene encoding a GS linker having an amino acid sequence represented by SEQ ID NO: 1, and a Bsu36I site were synthesized. This DNA fragment was digested with MluI, ScaI and Bsu36I. The pAAV-mMAP-hI2S-mscFv2 constructed in Example 3 was digested with MluI and Bsu36I, and the DNA fragment treated with the above restriction enzymes was inserted into this. The resulting plasmid was designated as a pAAV-mMAP-hGAA (Pro)-GS-mscFv2 vector. The pAAV-mMAP-hGAA (Pro)-GS-mscFv2 vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), a gene encoding a conjugate in which a mouse anti-mTfRscFv antibody 2 bound to the C-terminus of hGAA (Pro) via a linker represented by SEQ ID NO: 1, the conjugate having an amino acid sequence represented by SEQ ID NO: 63 (hGAA (Pro)-GS-mscFv2), the bovine growth hormone polyA signal (SEQ ID NO: 17), and a functional equivalent of the second AAV-ITR (SEQ ID NO: 8). The pAAV-mMAP-hGAA (Pro)-GS-mscFv2 vector generally has a structure in which the genes encoding I2S are replaced with the genes encoding hGAA (Pro)-GS-mscFv2 in the vector map illustrated in FIG. 4.

### Example 32: Construction of pAAV-mMAP-hGAA vector

A DNA fragment containing a nucleotide sequence represented by SEQ ID NO: 64 and containing, from the 5' side, a MluI site, a gene encoding hGAA, and a Not1 site was synthesized. This DNA fragment was digested with MluI and NotI. The pAAV-mMAP-hI2S-mscFv2 constructed in Example 3 was digested with MluI and NotI, and the DNA fragment treated with the above restriction enzymes was inserted into this. The resulting plasmid was designated as a pAAV-mMAP-hGAA vector. The pAAV-mMAP-hGAA vector has a structure containing, in order from the upstream, a functional equivalent of the first AAV-ITR (SEQ ID NO: 7), the mouse α-fetoprotein enhancer/mouse albumin promoter (SEQ ID NO: 9), the chicken β-actin/MVM chimeric intron (SEQ ID NO: 10), the gene encoding hGAA, the bovine growth hormone polyA signal (SEQ ID NO: 17), and a functional equivalent of the second AAV-ITR (SEQ ID NO: 8). The pAAV-mMAP-hGAA vector generally has a structure in which the genes encoding I2S are replaced with the genes encoding hGAA in the vector map illustrated in FIG. 4.

### Example 33: Production of rAAV virion

HEK293 cells, which are cell lines derived from human embryonic kidney cells, were used as host cells. The HEK293 cells were seeded at 1.59 × 10⁸ per cell stack chamber in a 10-layer cell stack chamber (Corning) and cultured in a 10% FBS-containing DMEM medium (Thermo Fisher Scientific) at 37°C under 5% CO₂ for 3 days. As a DNA solution for transfection, a solution containing plasmids of the following combinations (1) to (3) was prepared:
(1) a pHelper vector (Takara Bio Inc.), a pR2 (mod) C8 vector, and a pAAV-mMAP-mscFv-GS3-hGAA vector;
(2) a pHelper vector (Takara Bio Inc.), a pR2 (mod) C8 vector, and a pAAV-mMAP-hGAA (Pro)-GS-mscFv2 vector;
(3) a pHelper vector (Takara Bio Inc.), a pR2 (mod) C8 vector, and a pAAV-mMAP-hGAA vector.

Each of the solutions of (1) to (3) contained the three plasmids at equimolar concentrations, and was adjusted to have a total DNA concentration of 1.0 mg/mL and to have a total liquid volume of 2.2 mL. To this solution, polyethyleneimine was added at a weight ratio of DNA (µg): polyethyleneimine (µg) = 1:2. ADMEM medium was further added to adjust a liquid volume to 1.0 L, and a transfection solution was prepared. After removing all of the culture supernatant from the 10-layer cell stack chamber 3 days after the start of culture, the total amount, 1.0 L, of the transfection solution was added. The cells were cultured at 37°C in the presence of 5% CO₂ for 3 days to produce rAAV virions. The rAAV virions produced in the host cell are partially released into the culture supernatant and partially retained within the host cell. The resulting rAAV virions were designated rAAV-mscFv-GS3-hGAA, rAAV-hGAA (Pro)-GS-mscFv2, and rAAV-hGAA, respectively. For example, rAAV-mscFv-GS3-hGAAis expected to express mscFv-GS3-hGAA when cells are infected.

### Example 34: Preparation of rAAV virion solution and measurement of rAAV genome amount

After completion of the culture in Example 33, rAAV virion solutions containing rAAV-mscFv-GS3-hGAA, rAAV-hGAA (Pro)-GS-mscFv2, and rAAV-hGAA, respectively (rAAV-mscFv-GS3-hGAA solution, rAAV-hGAA (Pro)-GS-mscFv2 solution, and rAAV-hGAA solution) were prepared from the culture supernatants by the methods described in Examples 8 to 13. Further, the amount of rAAV genome contained in the rAAV virion solution was measured by the method described in Example 14.

### Example 35: Evaluation of mTfR binding activity of conjugate of mouse anti-mTfRscFv and hGAA

Two types of recombinant proteins, mscFv-GS3-hGAA and hGAA (Pro)-GS-mscFv2, obtained by an ordinary method using CHO cells were measured for their binding activity to mouse transferrin receptor by the ELISA method. The ELISA method was generally performed in the following manner. To each well of a 96-well microtiter plate (Nunc), 100 µL of a mouse transferrin receptor extracellular domain represented by an amino acid sequence from position 87 to position 763 from the N-terminus in an amino acid sequence represented by SEQ ID NO: 39 diluted with 0.05 M bicarbonate buffer (pH 9.6) to a concentration of 10 µg/mL was added, and the plate was allowed to stand at room temperature for at least 1 hour to adsorb the antibody to the plate. Then, 300 µL of TBS containing 1% BSA and 0.05% Tween20 was added to each well, and the plate was allowed to stand at room temperature for 1 hour. Each well was washed three times with TBS containing 0.05% Tween20 (TBS-T). Then, mscFv-GS3-hGAA and hGAA (Pro)-GS-mscFv2 were diluted using TBS containing 0.1% BSA and 0.05% Tween20 to a concentration of 33.33, 11.11, 3.70, 1.23, 0.41, 0.14, and 0.046 nM, and to a concentration of 200, 66.7, 133.33, 22.2, 7.41, 2.47, 0.82, and 0.27 nM, respectively, and 100 µL of each diluted product was added to each well. The plate was allowed to stand at room temperature for at least 1 hour. After the plate was washed three times with TBS-T, 100 µL of anti-hGAA monoclonal antibodies diluted to 0.5 µg/mL with TBS containing 0.1% BSA and 0.05% Tween20 was added to each well, and the plate was allowed to stand at room temperature for at least 1 hour. The plate was washed three times with the TBS-T, then 100 µL of an HRP-labeled anti-human IgG polyclonal antibody (Bethyl) solution diluted to 50 ng/mL with TBS containing 0.1% BSA and 0.05% Tween20 was added to each well, and the plate was allowed to stand at room temperature for at least 1 hour. Each well was washed three times with TBS-T, then 50 µL of a chromogenic substrate solution TMB Stabilized Substrate for Horseradish Peroxidase (Promega) was added to each well, and the mixture was allowed to stand at room temperature for from 5 to 10 minutes. Then, 100 µL of a stop solution (1 N hydrochloride) was added to each well, and the absorbance at 450 nm for each well was measured using a plate reader (MOLECULAR DEVICES). EC₅₀ was calculated from the measured values, and the binding activity of each recombinant protein to mTfR was evaluated.

The EC₅₀ values for the binding affinity of mscFv-GS3-hGAA and hGAA (Pro)-GS-mscFv2 for mouse TfR were 0.173 nM and 83.7 nM, respectively.

### Example 36: Preparation of GAAKO mice

The GAAKO mouse is a mouse homozygously deficient in acid α-glucosidase gene (Gaa gene). GAA KO mice were prepared generally in the following manner. Using the genomic DNA of the ES cell as a template,
two fragments, the 5' homologous region and the 3' homologous region, were amplified by the PCR method. Then, Exon6 of the Gaa gene into which a stop codon was inserted together with the loxP sequence and the PGK-neo region was constructed, and a homologous recombination vector for the Gaa genes was constructed by integrating a fragment of Exon6 by an ordinary method. This targeting vector was introduced into ES cells of the C57NBL/6N line by the electroporation method, and positive ES cells in the primary screening were obtained after drug selection. Homologous recombinants were selected from among the obtained primary screening positive ES cells to obtain homologous recombinant ES cell clones. Using the obtained homologous recombinant ES cell clone and an 8-cell stage embryo (host embryo) of an ICR mouse, a chimeric embryo was prepared by the aggregation method. The obtained chimeric embryo was transplanted into a pseudopregnant mouse (recipient mouse) obtained by mating with a mouse subjected to vasectomy. The obtained offspring (chimeric mice) was subjected to hair color determination to select an individual in which ES cells contributed to formation of a living body with high efficiency, i.e., an individual having a high ratio of white hair to the total hair. The chimeric mouse individuals were crossed with ICR mice to obtain F1 mice. White F1 mice were selected, DNAs extracted from tail tissues were analyzed, and mice in which mouse Gaa genes were heterozygous on the chromosome were designated as Gaa heterozygous mice. Based on this mouse, a mouse homozygously deficient in Gaa gene (GAAKO mouse) was prepared.

### Example 37: Evaluation of efficacy of each rAAV virion using GAA KO mouse

The GAA KO mice prepared in Example 36 were used to evaluate the efficacy of each rAAV when administered to living bodies. The GAA KO mice used were male mice at 10 to 11 weeks of age (at the start of administration). Each of the rAAV-mscFv-GS3-hGAA solution, rAAV-hGAA (Pro)-GS-mscFv2 solution and rAAV-hGAA solution obtained in Example 34 was single-administered to male GAA KO mice in a tail vein at a dose of 1.0 × 10¹³ vg/kg (N = 5 for each group). In addition, a normal control group composed of male wild-type mice and a pathological control group composed of male GAA KO mice were set (N = 5 for each group). Physiological saline (Otsuka Pharmaceutical Factory, Inc.) was administered to the normal control group and the pathological control group.

Four weeks after the administration, the chest was opened and heart blood was collected under anesthesia by inhalation of isoflurane. The bloods were collected into EDTA-2K coated blood-collecting tubes (Capiject, Terumo Corporation) to obtain anticoagulated bloods, which were then subjected to measurement of hemoglobin concentrations. The remaining anticoagulated blood was allowed to stand on ice and then centrifuged (2000 × g, 20 minutes), and plasma was collected. In addition, the mouse after heart blood collection was systemically perfused with physiological saline (Otsuka Pharmaceutical Factory, Inc.), and the brain and the anterior tibial muscle were removed. The brain was immersed in liquid nitrogen 3 minutes after the start of perfusion. After measuring the wet weight of skeletal muscle, it was quickly immersed in liquid nitrogen for quick freezing.

### Example 38: Measurement of rAAV-derived protein concentration in plasma and tissue

Each of rAAV-derived proteins in rAAV-mscFv-GS3-hGAA, rAAV-hGAA (Pro)-GS-mscFv2, and rAAV-hGAA contained in the plasma prepared in Example 37 and the supernatant obtained by homogenizing brain tissue was quantified. Quantitation was generally performed in the following manner. One hundred and fifty (150) µL of Superblock Blocking Buffer in PBS (Thermo Fisher Scientific) was added to each well of a Streptavidin Gold plate (Meso scale diagnostics), and the plate was blocked by shaking for 1 hour or more. Then, standard samples for calibration curve (standard samples for GAA calibration curve) containing known concentrations of mscFv-GS3-hGAA, hGAA (Pro)-GS-mscFv2, and hGAA, respectively, and each analyte were added to a mixed solution of biotinylated anti-hGAA polyclonal antibodies and SLTLFOnated anti-hGAA polyclonal antibodies, and shaken for 1 hour or more to obtain antibody reaction solutions. The anti-hGAA polyclonal antibody was obtained by obtaining antiserum from rabbit whole blood immunized multiple times with human acid α-glucosidase having the amino acid sequence represented by SEQ ID NO: 58, followed by affinity purification. One of the obtained polyclonal antibodies was modified using Biotin Labeling Kit-NH2 (Dojindo Laboratories) according to the attached protocol to obtain biotinylated anti-hGAA polyclonal antibodies. Another one of the obtained polyclonal antibodies was modified using MSD GOLD SULFO-TAG NHS-Ester (Meso Scale Diagnostics) according to the attached protocol to obtain SULFOnated anti-hGAA polyclonal antibodies. The blocking solution was removed from each well. After washing with PBS containing 0.05% Tween 20 (PBST, Sigma Aldrich), 25 µL of the antibody reaction solution was added to each well and shaken for 1 hour or more. Then, the antibody reaction solution was removed. After washing with PBST, 150 µL of 4 × Read Buffer (Meso scale diagnostics) mixed and diluted to 1/2 with water for injection (Otsuka Pharmaceutical Factory, Inc.) was added, and the amount of luminescence from each well was measured using Sector^{™} Imager 6000 (Meso scale diagnostics). A calibration curve was prepared from the measured values of each standard sample for GAA calibration curve, and the measured value of each analyte was interpolated into the calibration curve to calculate the amount of each protein contained per mL of the analyte. mscFv-GS3-hGAA and hGAA (Pro)-GS-mscFv2 contained in the standard sample for calibration curve were obtained by expression using CHO cells into which expression vectors incorporating genes encoding the respective proteins were introduced by an ordinary method.

The measurement results of the rAAV-derived protein concentration in plasma are illustrated in FIG. 15. The concentration of each rAAV-derived protein in plasma increased by the time point of 4 weeks after administration in all the rAAV virion administration groups. The above results show that rAAV virions injected intravenously into mice were taken up into cells, and that at least rAAV-derived proteins expressed by transcription and translation of genes encoded by rAAV in the cells were released into blood.

The measurement results of the concentration of rAAV-derived protein in the brain tissue are illustrated in FIG. 16. It was shown that the fusion protein encoded by each rAAV virion was detected in the brain tissue 4 weeks after administration in all of the rAAV virion administration groups. The expression concentration in the brain tissue was very high particularly in the rAAV-mscFv-GS3-hGAA administration group. Although the expression concentration in the brain tissue in the rAAV-hGAA (Pro)-GS-mscFv2 administration group was low, the concentration of glycogen in the brain tissue of the rAAV-hGAA (Pro)-GS-mscFv2 administration group decreased dramatically as shown in Table 4, indicating that rAAV-hGAA (Pro)-GS-mscFv2 administered to the mice passed through the BBB and exhibited the enzymatic activity as hGAAin the brain tissue to degrade glycogen.

The above results show that, by administering rAAV-mscFv-GS3-hGAA and rAAV-hGAA (Pro)-GS-mscFv2, the conjugate of anti-mTfR antibody scFv and hGAA, which was expected to be expressed in the mouse body, was actually expressed as expected, and that GAA bound to anti-mTfR antibody scFv passed through the BBB and reached the tissues of the central nervous system.

### Example 39: Measurement of concentration of glycogen in tissue

The concentration of glycogen contained in the supernatant obtained by homogenizing the brain and anterior tibial muscle was measured generally by the following method. The measurement was performed using a Glycogen assay kit (Biovision) according to the attached protocol. To 96F Nontreated Black microwell (Thermo Scientific), 50 µL of each of a standard sample for calibration curve containing glycogen at a known concentration and a tissue homogenate supernatant was added. Then, 1 µL of a hydrolysis reaction reagent, Hydrolysis Enzyme Mix, was added to each well, mixed, and reacted at room temperature for 30 minutes. Then, 50 µL of Development Enzyme Mix containing OxiRed Probe was added to each well, mixed, and reacted at room temperature for 30 minutes in the dark. The fluorescence intensity from each well was measured using a SpectraMax iD3 (Molecular Devices) (excitation wavelength: 535 nm, detection wavelength: 587 nm). A calibration curve was prepared from the measured values of each standard sample for calibration curve, and the measured value of each analyte was interpolated into the calibration curve to calculate the concentration of glycogen in each analyte, and further the amount of glycogen contained per mg of tissue (mg/g tissue) was calculated.

As an index of efficacy, a reduction ratio (%) representing a glycogen reduction effect in each tissue was used. The reduction ratio (%) was defined as {([KO] - [WT]) - ([Test article] - [WT])} × 100/([KO] - [WT]). [WT] represents an average concentration of glycogen of the normal control group, [KO] represents an average concentration of glycogen of the pathological control group, and [Test article] represents an average concentration of glycogen of each test substance administration group.

The measurement results of the concentration (µg/g wet weight) of glycogen in the brain and the reduction ratio (%) are shown in Table 4. The concentration of glycogen in the brain was decreased in both the rAAV-mscFv-GS3-hGAA and rAAV-hGAA (Pro)-GS-mscFv2 administration groups as compared with the pathological control group (the reduction ratios (%) were 101% and 23.9%, respectively). On the other hand, in the rAAV-hGAA administration group expressing hGAA to which the anti-mTfR antibody scFv was not fused, the concentration of glycogen in the brain decreased, but the reduction ratio (%) remained at 5.47%.

**[Table 4]**

| (Table 4) Results of measuring concentration of glycogen in brain | | |
|---|---|---|
| | Concentration of glycogen in brain (µg/g wet weight) | Reduction ratio (%) |
| Normal control group | 0.05 | - |
| Pathological control group | 2.47 | 0 |
| rAAV-hGAA (Pro)-GS-mscFv2 administration group | 1.90 | 23.9 |
| rAAV-mscFv-GS3-hGAA administration group | 0.03 | 101 |
| rAAV-hGAA administration group | 2.34 | 5.47 |

The measurement results of the concentration (µg/g wet weight) of glycogen in the anterior tibial muscle and the reduction ratio (%) are shown in Table 5. The concentration of glycogen in the anterior tibial muscle was significantly decreased in all the administration groups as compared with the pathological control group.

**[Table 5]**

| (Table 5) Results of measuring concentration of glycogen in anterior tibial muscle | | |
|---|---|---|
| | Concentration of glycogen in anterior tibial muscle (µg/g wet weight) | Reduction ratio (%) |
| Normal control group | 0.08 | - |
| Pathological control group | 13.28 | 0 |
| rAAV-hGAA (Pro)-GS-mscFv2 administration group | 3.97 | 70.5 |
| rAAV-mscFv-GS3-hGAA administration group | 0.24 | 98.8 |
| rAAV-hGAA administration group | 0.59 | 96.2 |

These results show that mscFv-GS3-hGAA and hGAA-GS-mscFv2 expressed in vivo by intravenous injection of rAAV-mscFv-GS3-hGAA and rAAV-hGAA-GS-mscFv2 into mice passed through the BBB and reached the tissues of the central nervous system, and exhibited the enzymatic activity as hGAA in the tissues of the central nervous system, thereby degrading the concentration of glycogen accumulated in the central nervous system.

### Example 40: Measurement of concentration of hemoglobin in blood

The hemoglobin concentration was measured using the anticoagulated blood obtained in Example 37. The hemoglobin concentration was measured by the SLS hemoglobin method described in Example 19. The blood hemoglobin concentration is an item usually used as an index of anaemia. The results are illustrated in FIG. 17.

Four weeks after the administration, a decrease in blood hemoglobin concentration was confirmed only in the rAAV-mscFv-GS3-hGAA administration group. In the rAAV-hGAA (Pro)-GS-mscFv2 and rAAV-hGAA administration groups, no clear difference was observed as compared with the normal control group and the pathological control group. The above results are consistent with Conclusion 1 of Example 20 and Conclusion 2 of Example 29 that rAAV virions containing genes encoding fusion proteins of anti-TfR antibodies and desired physiologically active proteins whose anti-TfR-mediated binding activity (EC₅₀) to TfR is adjusted to 0.5 nM, for example, 3 to 100 nM can be provided as rAAV virions for safe treatment that do not cause anaemia while maintaining high expression levels of the fusion proteins in the blood and exerting the activity of the physiologically active proteins in the central nervous system.

In addition, the above results show that intravenous injection of rAAV-hGAA (Pro)-GS-mscFv2 can express hGAA (Pro)-GS-mscFv2 in vivo for at least one month, and that the expressed hGAA-GS (Pro)-mscFv2 can exert hGAA activity in the central nervous system and muscle tissue of the pathological model mouse, and can thus degrade glycogen accumulated. In addition, the results show that it is effective as a safe therapeutic agent for Pompe syndrome which does not cause anaemia while maintaining a high blood concentration and exhibiting efficacy by regulating the activity of the anti-TfR antibody.

### Industrial Applicability

The present invention can provide a transferrin receptor antibody and a physiologically active protein having a reduced possibility of causing an anemia symptom by appropriately adjusting the affinity for or binding activity to a transferrin receptor, when a fusion protein of an anti-transferrin receptor antibody and the physiologically active protein is used in gene therapy; and a viral vector having integrated therein a gene encoding such a fusion protein.

### Reference Signs List

1. Functional equivalent of first AAV-ITR
2. Mouse α-fetoprotein enhancer/mouse albumin promoter
3. Chicken β-actin/MVM chimeric intron
4. Gene encoding conjugate (mscFv-GS3-hI2S) in which hI2S binds to C-terminus of mouse anti-mouse transferrin receptor single chain antibody (mouse anti-mTfRscFv antibody) via linker sequence consisting of three repetitions of amino acid sequence of SEQ ID NO: 1
5. Bovine growth hormone poly A signal
6. Functional equivalent of second AAV-ITR
7. Ampicillin-resistance gene
8. Replication origin (ColE1 ori)
9. Gene encoding conjugate (mscFv2-GS3-hI2S) in which hI2S binds to C-terminus of mouse anti-mouse transferrin receptor single chain antibody (mouse anti-mTfRscFv antibody 2) in which amino acid mutations are introduced at two positions of CDR of heavy chain and two positions of CDR of light chain of mscFv, via linker sequence consisting of three repetitions of amino acid sequence of SEQ ID NO: 1
10. Gene encoding conjugate (hI2S-mscFv2) in which mouse anti-mTfRscFv antibody 2 binds to C-terminus of hI2S
11. Genes encoding hI2S
12. p5 promoter
13. Rep region of AAV2
14. Cap region of AAV8
15. p5 promoter functioning as enhancer

SEQ ID NO: 1: example 1 of amino acid sequence of linker
SEQ ID NO: 2: example 2 of amino acid sequence of linker
SEQ ID NO: 3: example 3 of amino acid sequence of linker
SEQ ID NO: 4: example 4 of amino acid sequence of linker
SEQ ID NO: 5: nucleotide sequence of first inverted terminal repeat (first ITR) of serotype 2 AAV, wild type
SEQ ID NO: 6: nucleotide sequence of first inverted terminal repeat (second ITR) of serotype 2 AAV, wild type
SEQ ID NO: 7: suitable example of nucleotide sequence of first inverted terminal repeat (first ITR), synthetic sequence
SEQ ID NO: 8: suitable example of nucleotide sequence of second inverted terminal repeat (second ITR), synthetic sequence
SEQ ID NO: 9: nucleotide sequence of mouse α-fetoprotein enhancer/mouse albumin promoter, synthetic sequence
SEQ ID NO: 10: nucleotide sequence of chicken β-actin/MVM chimeric intron, synthetic sequence
SEQ ID NO: 11: example of nucleotide sequence of internal ribosome binding site derived from 5' untranslated region of mouse encephalomyocarditis virus
SEQ ID NO: 12: amino acid sequence of human transferrin receptor
SEQ ID NO: 13: example of amino acid sequence of Fab region of heavy chain of anti-hTfR antibody
SEQ ID NO: 14: amino acid sequence of human I2S
SEQ ID NO: 15: example of amino acid sequence of light chain of anti-hTfR antibody
SEQ ID NO: 16: example of amino acid sequence of heavy chain of anti-hTfR antibody
SEQ ID NO: 17: nucleotide sequence of bovine growth hormone polyA signal
SEQ ID NO: 18: nucleotide sequence of DNA fragment synthesized for preparing pAAV-mMAP-mscFv-GS3-hI2S vector, synthetic sequence
SEQ ID NO: 19: amino acid sequence of mscFv-GS3-hI2S
SEQ ID NO: 20: nucleotide sequence encoding mscFv-GS3-hI2S, synthetic sequence
SEQ ID NO: 21: nucleotide sequence of DNA fragment synthesized for preparing pAAV-mMAP-mscFv2-GS3-hI2S vector, synthetic sequence
SEQ ID NO: 22: amino acid sequence of mscFv2-GS3-hI2S
SEQ ID NO: 23: nucleotide sequence encoding mscFv2-GS3-hI2S, synthetic sequence
SEQ ID NO: 24: nucleotide sequence of DNA fragment synthesized for preparing pAAV-mMAP-hI2S-mscFv2 vector, synthetic sequence
SEQ ID NO: 25: amino acid sequence of hI2S-mscFv2
SEQ ID NO: 26: nucleotide sequence encoding hI2S-mscFv2, synthetic sequence
SEQ ID NO: 27: nucleotide sequence of DNA fragment synthesized for preparing pAAV-mMAP-hI2S vector, synthetic sequence
SEQ ID NO: 28: nucleotide sequence encoding hI2S
SEQ ID NO: 29: nucleotide sequence of DNA fragment synthesized for preparing pR2 (mod) C6 vector, synthetic sequence
SEQ ID NO: 30: nucleotide sequence of DNA fragment synthesized for preparing pR2 (mod) C8 vector, synthetic sequence
SEQ ID NO: 31: nucleotide sequence of Cap region of AAV8
SEQ ID NO: 32: nucleotide sequence containing Rep region of AAV2, synthetic sequence
SEQ ID NO: 33: nucleotide sequence of primer SI-1, synthetic sequence
SEQ ID NO: 34: nucleotide sequence of primer SI-2, synthetic sequence
SEQ ID NO: 35: nucleotide sequence of probe SI-1, synthetic sequence
SEQ ID NO: 36: nucleotide sequence of primer SI-3, synthetic sequence
SEQ ID NO: 37: nucleotide sequence of primer SI-4, synthetic sequence
SEQ ID NO: 38: nucleotide sequence of probe SI-2, synthetic sequence
SEQ ID NO: 39: amino acid sequence of mouse transferrin receptor
SEQ ID NO: 40: amino acid sequence 1 of light chain CDR1 of anti-hTfR antibody
SEQ ID NO: 41: amino acid sequence 2 of light chain CDR1 of anti-hTfR antibody
SEQ ID NO: 42: amino acid sequence 1 of light chain CDR2 of anti-hTfR antibody
SEQ ID NO: 43: amino acid sequence 2 of light chain CDR2 of anti-hTfR antibody
SEQ ID NO: 44: amino acid sequence 1 of light chain CDR3 of anti-hTfR antibody
SEQ ID NO: 45: amino acid sequence 1 of heavy chain CDR1 of anti-hTfR antibody
SEQ ID NO: 46: amino acid sequence 2 of heavy chain CDR1 of anti-hTfR antibody
SEQ ID NO: 47: amino acid sequence 1 of heavy chain CDR2 of anti-hTfR antibody
SEQ ID NO: 48: amino acid sequence 2 of heavy chain CDR2 of anti-hTfR antibody
SEQ ID NO: 49: amino acid sequence 1 of heavy chain CDR3 of anti-hTfR antibody
SEQ ID NO: 50: amino acid sequence 2 of heavy chain CDR3 of anti-hTfR antibody
SEQ ID NO: 51: amino acid sequence of conjugate of Fab region of heavy chain of anti-hTfR antibody and human I2S
SEQ ID NO: 52: suitable example of nucleotide sequence of first long terminal repeat (first LTR), synthetic sequence
SEQ ID NO: 53: suitable example of nucleotide sequence of second long terminal repeat (second LTR), synthetic sequence
SEQ ID NO: 54: nucleotide sequence of leader of Sendai virus genome
SEQ ID NO: 55: nucleotide sequence of trailer of Sendai virus genome
SEQ ID NO: 56: amino acid sequence of mscFv3-GS3-hI2S
SEQ ID NO: 57: nucleotide sequence of DNA fragment synthesized for preparing pAAV-mMAP-mscFv3-GS3-hI2S vector, synthetic sequence
SEQ ID NO: 58: amino acid sequence of hGAA
SEQ ID NO: 59: nucleotide sequence of DNA fragment synthesized for preparing pAAV-mMAP-mscFv-GS3-hGAA vector, synthetic sequence
SEQ ID NO: 60: amino acid sequence of mscFv-GS3-hGAA
SEQ ID NO: 61: amino acid sequence of hGAA (Pro)
SEQ ID NO: 62: nucleotide sequence of DNA fragment synthesized for preparing pAAV-mMAP-hGAA (Pro)-GS-mscFv2 vector, synthetic sequence
SEQ ID NO: 63: amino acid sequence of hGAA (Pro)-GS-mscFv2
SEQ ID NO: 64: nucleotide sequence of DNA fragment synthesized for preparing pAAV-mMAP-hGAA vector, synthetic sequence

## Claims

1. A nucleic acid molecule encoding a fusion protein of an anti-transferrin receptor antibody and a physiologically active protein, wherein the fusion protein has a binding activity (EC₅₀) to a transferrin receptor of from 0.5 nM to 1 µM, and wherein the nucleic acid molecule comprising any nucleotide sequence selected from the group consisting of (1) to (6) below:
(1) a nucleotide sequence comprising a first inverted terminal repeat (ITR) or a functional equivalent thereof, downstream thereof a nucleotide sequence encoding the fusion protein of the anti-transferrin receptor antibody and the physiologically active protein, and further downstream thereof a nucleotide sequence comprising a second inverted terminal repeat (ITR) or a functional equivalent thereof;
(2) a nucleotide sequence comprising a first inverted terminal repeat (ITR) or a functional equivalent thereof, downstream thereof a nucleotide sequence comprising a gene expression regulatory site, further downstream thereof a nucleotide sequence encoding the fusion protein of the anti-transferrin receptor antibody and the physiologically active protein, and further downstream thereof a nucleotide sequence comprising a second inverted terminal repeat (ITR) or a functional equivalent thereof;
(3) a nucleotide sequence comprising a first long terminal repeat (LTR) or a functional equivalent thereof, downstream thereof a nucleotide sequence encoding the fusion protein of the anti-transferrin receptor antibody and the physiologically active protein, and further downstream thereof a nucleotide sequence comprising a second long terminal repeat (LTR) or a functional equivalent thereof;
(4) a nucleotide sequence comprising a first long terminal repeat (LTR) or a functional equivalent thereof, downstream thereof a nucleotide sequence comprising a gene expression regulatory site, further downstream thereof a nucleotide sequence encoding the fusion protein of the anti-transferrin receptor antibody and the physiologically active protein, and further downstream thereof a nucleotide sequence comprising a second long terminal repeat (LTR) or a functional equivalent thereof;
(5) a nucleotide sequence comprising a leader or a functional equivalent thereof, downstream thereof a nucleotide sequence encoding the fusion protein of the anti-transferrin receptor antibody and the physiologically active protein, and further downstream thereof a nucleotide sequence comprising a trailer or a functional equivalent thereof; and
(6) a nucleotide sequence comprising a leader or a functional equivalent thereof, downstream thereof a nucleotide sequence comprising a gene expression regulatory site, further downstream thereof a nucleotide sequence encoding the fusion protein of the anti-transferrin receptor antibody and the physiologically active protein, and further downstream thereof a nucleotide sequence comprising a trailer or a functional equivalent thereof further.

2. The nucleic acid molecule according to claim 1, wherein the nucleic acid molecule is selected from the group consisting of (1) to (4) below:
(1) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a heavy chain of the anti-transferrin receptor antibody, and a nucleotide sequence encoding a light chain of the anti-transferrin receptor antibody;
(2) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a heavy chain of the anti-transferrin receptor antibody, and a nucleotide sequence encoding a light chain of the anti-transferrin receptor antibody;
(3) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a light chain of the anti-transferrin receptor antibody, and a nucleotide sequence encoding a heavy chain of the anti-transferrin receptor antibody; and
(4) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a light chain of the anti-transferrin receptor antibody, and a nucleotide sequence encoding a heavy chain of the anti-transferrin receptor antibody.

3. The nucleic acid molecule according to claim 1, wherein the nucleic acid molecule is selected from the group consisting of (1) to (4) below:
(1) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a heavy chain of the anti-transferrin receptor antibody via a linker, and a nucleotide sequence encoding a light chain of the anti-transferrin receptor antibody;
(2) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a heavy chain of the anti-transferrin receptor antibody via a linker, and a nucleotide sequence encoding a light chain of the anti-transferrin receptor antibody;
(3) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a light chain of the anti-transferrin receptor antibody via a linker, and a nucleotide sequence encoding a heavy chain of the anti-transferrin receptor antibody; and
(4) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a light chain of the anti-transferrin receptor antibody via a linker, and a nucleotide sequence encoding a heavy chain of the anti-transferrin receptor antibody.

4. The nucleic acid molecule according to claim 3, wherein the linker is a peptide comprising from 1 to 50 amino acid residues.

5. The nucleic acid molecule according to claim 4, wherein the linker is a peptide comprising an amino acid sequence selected from the group consisting of one glycine, one serine, an amino acid sequence Gly-Ser, an amino acid sequence Gly-Gly-Ser, an amino acid sequence of SEQ ID NO: 1, an amino acid sequence of SEQ ID NO: 2, an amino acid sequence of SEQ ID NO: 3, and an amino acid sequence comprising successively from 2 to 10 of these amino acid sequences.

6. The nucleic acid molecule according to claim 1, wherein the nucleic acid molecule is selected from the group consisting of (1) to (4) below:
(1) a nucleic acid molecule wherein the nucleotide sequence encoding the fusion protein of the anti-transferrin receptor antibody and the physiologically active protein comprises a nucleotide sequence encoding a conjugate in which a heavy chain of the anti-transferrin receptor antibody binds to a C-terminus of a light chain of the anti-transferrin receptor antibody via a second linker, and further the physiologically active protein binds to a C-terminus thereof directly or via a linker;
(2) a nucleic acid molecule wherein the nucleotide sequence encoding the fusion protein of the anti-transferrin receptor antibody and the physiologically active protein comprises a nucleotide sequence encoding a conjugate in which a light chain of the anti-transferrin receptor antibody binds to a C-terminus of a heavy chain of the anti-transferrin receptor antibody via a second linker, and further the physiologically active protein binds to a C-terminus thereof directly or via a linker;
(3) a nucleic acid molecule wherein the nucleotide sequence encoding the fusion protein of the anti-transferrin receptor antibody and the physiologically active protein comprises a nucleotide sequence encoding a conjugate in which a heavy chain of the anti-transferrin receptor antibody binds to a C-terminus of the physiologically active protein directly or via a linker, and further a light chain of the anti-transferrin receptor antibody binds to a C-terminus thereof via a second linker; and
(4) a nucleic acid molecule wherein the nucleotide sequence encoding the fusion protein of the anti-transferrin receptor antibody and the physiologically active protein comprises a nucleotide sequence encoding a conjugate in which a light chain of the anti-transferrin receptor antibody binds to a C-terminus of the physiologically active protein directly or via a linker, and further a heavy chain of the anti-transferrin receptor antibody binds to a C-terminus thereof via a second linker.

7. The nucleic acid molecule according to claim 6, wherein the linker is a peptide comprising from 1 to 50 amino acid residues.

8. The nucleic acid molecule according to claim 7, wherein the linker is a peptide comprising an amino acid sequence selected from the group consisting of one glycine, one serine, an amino acid sequence Gly-Ser, an amino acid sequence Gly-Gly-Ser, an amino acid sequence of SEQ ID NO: 1, an amino acid sequence of SEQ ID NO: 2, an amino acid sequence of SEQ ID NO: 3, and an amino acid sequence comprising successively from 2 to 10 of these amino acid sequences.

9. The nucleic acid molecule according to claim 8, wherein the second linker comprises from 8 to 50 amino acid residues.

10. The nucleic acid molecule according to claim 9, wherein the second linker is selected from the group consisting of an amino acid sequence Gly-Ser, an amino acid sequence Gly-Gly-Ser, an amino acid sequence Gly-Gly-Gly, amino acid sequences of SEQ ID NO: 1, SEQ ID NO: 2, and SEQ ID NO: 3, an amino acid sequence comprising successively 3 of the amino acid sequences of SEQ ID NO: 1, and an amino acid sequence comprising successively from 2 to 10 of these amino acid sequences.

11. The nucleic acid molecule according to claim 2, wherein the nucleic acid molecule is selected from the group consisting of (1) to (4) below:
(1) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a heavy chain of the anti-transferrin receptor antibody, further downstream thereof a nucleotide sequence encoding an internal ribosome binding site, and further downstream thereof a nucleotide sequence encoding a light chain of the anti-transferrin receptor antibody;
(2) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a heavy chain of the anti-transferrin receptor antibody, further downstream thereof a nucleotide sequence encoding an internal ribosome binding site, and further downstream thereof a nucleotide sequence encoding a light chain of the anti-transferrin receptor antibody;
(3) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a light chain of the anti-transferrin receptor antibody, further downstream thereof a nucleotide sequence encoding an internal ribosome binding site, and further downstream thereof a nucleotide sequence encoding a heavy chain of the anti-transferrin receptor antibody; and
(4) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a light chain of the anti-transferrin receptor antibody, further downstream thereof a nucleotide sequence encoding an internal ribosome binding site, and further downstream thereof a nucleotide sequence encoding a heavy chain of the anti-transferrin receptor antibody.

12. The nucleic acid molecule according to any one of claims 3 to 5, wherein the nucleic acid molecule is selected from the group consisting of (1) to (4) below:
(1) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a heavy chain of the anti-transferrin receptor antibody via a linker, further downstream thereof a nucleotide sequence encoding an internal ribosome binding site, and further downstream thereof a nucleotide sequence encoding a light chain of the anti-transferrin receptor antibody;
(2) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a heavy chain of the anti-transferrin receptor antibody via a linker, further downstream thereof a nucleotide sequence encoding an internal ribosome binding site, and further downstream thereof a nucleotide sequence encoding a light chain of the anti-transferrin receptor antibody;
(3) a nucleic acid molecule wherein the nucleotide sequence encoding the fusion protein of the anti-transferrin receptor antibody and the physiologically active protein comprises a nucleotide sequence encoding a conjugate in which the physiologically active protein binds to a C-terminus of a light chain of the anti-transferrin receptor antibody via a linker, further downstream thereof a nucleotide sequence encoding an internal ribosome binding site, and further downstream thereof a nucleotide sequence encoding a heavy chain of the anti-transferrin receptor antibody; and
(4) a nucleic acid molecule wherein the nucleotide sequence encoding the fusion protein of the anti-transferrin receptor antibody and the physiologically active protein comprises a nucleotide sequence encoding a conjugate in which the physiologically active protein binds to an N-terminus of a light chain of the anti-transferrin receptor antibody via a linker, further downstream thereof a nucleotide sequence encoding an internal ribosome binding site, and further downstream thereof a nucleotide sequence encoding a heavy chain of the anti-transferrin receptor antibody.

13. The nucleic acid molecule according to claim 11 or 12, wherein the internal ribosome binding site is derived from a 5' untranslated region of a virus or gene selected from the group consisting of virus belonging to the family *Picornaviridae*, foot-and-mouth disease virus, hepatitis A virus, hepatitis C virus, coronavirus, bovine enterovirus, Theiler's murine encephalomyelitis virus, Coxsackie type B virus, human immunoglobulin heavy chain binding protein gene, Drosophila antennapedia gene, and Drosophila ultrabithorax gene.

14. The nucleic acid molecule according to claim 11 or 12, wherein the internal ribosome binding site is derived from the 5' untranslated region of the virus belonging to the family *Picornaviridae.*

15. The nucleic acid molecule according to any one of claims 1 to 14, wherein the gene expression regulatory site is selected from the group consisting of a cytomegalovirus-derived promoter, an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, a mouse albumin promoter, a human albumin promoter, a human α-1 antitrypsin promoter, and a mouse α-fetoprotein enhancer/mouse albumin promoter.

16. The nucleic acid molecule according to any one of claims 1 to 15, wherein the anti-transferrin receptor antibody is an antigen binding fragment.

17. The nucleic acid molecule according to any one of claims 1 to 15, wherein the anti-transferrin receptor antibody is a Fab.

18. The nucleic acid molecule according to any one of claims 1 to 17, wherein the physiologically active protein is selected from the group consisting of a growth hormone, a lysosomal enzyme, a somatomedin, an insulin, a glucagon, a cytokine, a lymphokine, a blood coagulation factor, an anti-transferrin receptor antibody, a fusion protein of an anti-transferrin receptor antibody with another protein, granulocyte-macrophage colony-stimulating factor (GM-CSF), granulocyte-colony stimulating factor (G-CSF), macrophage-colony stimulating factor (M-CSF), erythropoietin, darbepoetin, tissue-plasminogen activator (t-PA), thrombomodulin, follicle-stimulating hormone (FSH), gonadotropin-releasing hormone (GnRH), gonadotropin, nerve growth factor (NGF), ciliary neurotrophic factor (CNTF), glial-cell derived neurotrophic factor (GDNF), neurotrophin-3, neurotrophin-4/5, neurotrophin-6, neuregulin-1, activin, basic fibroblast growth factor (bFGF), fibroblast growth factor 2 (FGF2), epidermal growth factor (EGF), vascular endothelial growth factor (VEGF), interferon α, interferon β, interferon γ, interleukin-6, PD-1, a PD-1 ligand, tumor necrosis factor-α receptor (TNF-α receptor), an enzyme having beta-amyloid-degrading activity, etanercept, pegvisomant, metreleptin, abatacept, asfotase, a GLP-1 receptor agonist, and an antibody medicament.

19. The nucleic acid molecule according to any one of claims 1 to 17, wherein the physiologically active protein is selected from the group consisting of α-L-iduronidase, iduronate-2-sulfatase, acid α-glucosidase, glucocerebrosidase, β-galactosidase, a GM2 activating protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoA α-glucosaminide N-acetyltransferase, N-acetylglucosamine-6-sulfatase, acid ceramidase, amylo-1,6-glucosidase, sialidase, palmitoylprotein thioesterase-1, tripeptidylpeptidase-1, hyaluronidase-1, CLN1 and CLN2.

20. The nucleic acid molecule according to any one of claims 1 to 19, wherein the first inverted terminal repeat and the second inverted terminal repeat are derived from an adeno-associated virus, derived from an adenovirus, or are mutants thereof.

21. The nucleic acid molecule according to any one of claims 1 to 19, wherein the first inverted terminal repeat comprises a nucleotide sequence represented by SEQ ID NO: 5 and the second inverted terminal repeat comprises a nucleotide sequence represented by SEQ ID NO: 6.

22. The nucleic acid molecule according to any one of claims 1 to 19, wherein
the first inverted terminal repeat is selected from the group consisting of (1) to (3) below:
(1) an inverted terminal repeat comprising a nucleotide sequence exhibiting 80% or more identity with the nucleotide sequence represented by SEQ ID NO: 5,
(2) an inverted terminal repeat comprising a nucleotide sequence showing 90% or more identity with the nucleotide sequence represented by SEQ ID NO: 5, and
(3) an inverted terminal repeat comprising the nucleotide sequence represented by SEQ ID NO: 5 modified by substitution, deletion, or addition of from 1 to 20 nucleotides; and
the second inverted terminal repeat is selected from the group consisting of (4) to (6) below:
(4) an inverted terminal repeat comprising a nucleotide sequence exhibiting 80% or more identity with the nucleotide sequence represented by SEQ ID NO: 6,
(5) an inverted terminal repeat comprising a nucleotide sequence exhibiting 90% or more identity with the nucleotide sequence represented by SEQ ID NO: 6, and
(6) an inverted terminal repeat comprising the nucleotide sequence represented by SEQ ID NO: 6 modified by substitution, deletion, or addition of from 1 to 20 nucleotides.

23. The nucleic acid molecule according to any one of claims 1 to 19, wherein the functional equivalent of the first inverted terminal repeat comprises a nucleotide sequence represented by SEQ ID NO: 7 and the functional equivalent of the second inverted terminal repeat comprises a nucleotide sequence represented by SEQ ID NO: 8.

24. The nucleic acid molecule according to any one of claims 1 to 19, wherein
the functional equivalent of the first inverted terminal repeat is selected from the group consisting of (1) to (3) below:
(1) a functional equivalent comprising a nucleotide sequence exhibiting 80% or more identity with the nucleotide sequence represented by SEQ ID NO: 7,
(2) a functional equivalent comprising a nucleotide sequence showing 90% or more identity with the nucleotide sequence represented by SEQ ID NO: 7, and
(3) a functional equivalent comprising the nucleotide sequence represented by SEQ ID NO: 7 modified by substitution, deletion, or addition of from 1 to 20 nucleotides; and
the functional equivalent of the second inverted terminal repeat is selected from the group consisting of (4) to (6) below:
(4) a functional equivalent comprising a nucleotide sequence exhibiting 80% or more identity with the nucleotide sequence represented by SEQ ID NO: 8,
(5) a functional equivalent comprising a nucleotide sequence exhibiting 90% or more identity with the nucleotide sequence represented by SEQ ID NO: 8, and
(6) a functional equivalent comprising the nucleotide sequence represented by SEQ ID NO: 8 modified by substitution, deletion, or addition of from 1 to 20 nucleotides.

25. The nucleic acid molecule according to any one of claims 1 to 19, wherein the first long terminal repeat and the second long terminal repeat are derived from a lentivirus or a retrovirus, or are mutants thereof.

26. The nucleic acid molecule according to any one of claims 1 to 19, wherein the first long terminal repeat comprises a nucleotide sequence represented by SEQ ID NO: 52 and the second long terminal repeat comprises a nucleotide sequence represented by SEQ ID NO: 53.

27. The nucleic acid molecule according to any one of claims 1 to 19, wherein
the first long terminal repeat is selected from the group consisting of (1) to (3) below:
(1) a long terminal repeat comprising a nucleotide sequence exhibiting 80% or more identity with the nucleotide sequence represented by SEQ ID NO: 52,
(2) a long terminal repeat comprising a nucleotide sequence showing 90% or more identity with the nucleotide sequence represented by SEQ ID NO: 52, and
(3) a long terminal repeat comprising the nucleotide sequence represented by SEQ ID NO: 52 modified by substitution, deletion, or addition of from 1 to 20 nucleotides; and
the second long terminal repeat is selected from the group consisting of (4) to (6) below:
(4) a long terminal repeat comprising a nucleotide sequence exhibiting 80% or more identity with the nucleotide sequence represented by SEQ ID NO: 53,
(5) a long terminal repeat comprising a nucleotide sequence exhibiting 90% or more identity with the nucleotide sequence represented by SEQ ID NO: 53, and
(6) a long terminal repeat comprising the nucleotide sequence represented by SEQ ID NO: 53 modified by substitution, deletion, or addition of from 1 to 20 nucleotides.

28. The nucleic acid molecule according to any one of claims 1 to 19, wherein the leader and the trailer are derived from a Sendai virus, or are mutants thereof.

29. The nucleic acid molecule according to any one of claims 1 to 19, wherein the leader comprises a nucleotide sequence represented by SEQ ID NO: 54 and the trailer comprises a nucleotide sequence represented by SEQ ID NO: 55.

30. The nucleic acid molecule according to any one of claims 1 to 19, wherein
the leader is selected from the group consisting of (1) to (3) below:
(1) a leader comprising a nucleotide sequence exhibiting 80% or more identity with the nucleotide sequence represented by SEQ ID NO: 54,
(2) a leader comprising a nucleotide sequence showing 90% or more identity with the nucleotide sequence represented by SEQ ID NO: 54, and
(3) a leader comprising a nucleotide sequence represented by SEQ ID NO: 54 modified by substitution, deletion, or addition of from 1 to 20 nucleotides; and
the trailer is selected from the group consisting of (1) to (3) below:
(4) a trailer comprising a nucleotide sequence exhibiting 80% or more identity with the nucleotide sequence represented by SEQ ID NO: 55,
(5) a trailer comprising a nucleotide sequence exhibiting 90% or more identity with the nucleotide sequence represented by SEQ ID NO: 55, and
(6) a trailer comprising a nucleotide sequence represented by SEQ ID NO: 55 modified by substitution, deletion, or addition of from 1 to 20 nucleotides.

31. The nucleic acid molecule according to any one of claims 1 to 30, wherein the binding activity (EC₅₀) is from 3 to 500 nM, from 3 to 100 nM, from 4 nM to 1 µM, from 4 to 500 nM, from 4 to 100 nM, from 5 nM to 1 µM, from 5 to 500 nM, or from 5 to 100 nM.

32. A cell, a tissue or an animal having introduced therein the nucleic acid molecule according to any one of claims 1 to 31.

33. A stem cell having introduced therein the nucleic acid molecule according to any one of claims 1 to 31.

34. The stem cell according to claim 33, wherein the stem cell is selected from the group consisting of a mesenchymal stem cell, a dental pulp-derived stem cell, a hematopoietic stem cell, an embryonic stem cell, an endothelial stem cell, a mammary stem cell, an intestinal stem cell, a hepatic stem cell, a pancreatic stem cell, a neural stem cell, and an iPS cell.

35. A plasmid comprising the nucleic acid molecule according to any one of claims 1 to 31.

36. A viral virion comprising the nucleic acid molecule according to any one of claims 1 to 31.

37. A pharmaceutical composition comprising the viral virion according to claim 36; and a carrier.
